# EUROPEAN PATENT SPECIFICATION

(11) **EP 3 253 441 B1**
(45) Date of publication and mention of the grant of the patent: **19.08.2020**
(21) Application number: 16747389.1
(22) Date of filing: 05.02.2016
(51) Int. Cl.: A61N 1/372, A61N 1/378, A61N 1/36, A61N 1/05, A61B 5/00, A61N 2/00

(54) **MEDICAL APPARATUS INCLUDING AN IMPLANTABLE SYSTEM AND AN EXTERNAL SYSTEM**
MEDIZINISCHE VORRICHTUNG EINEM IMPLANTIERBAREN SYSTEM UND EINEM EXTERNEN SYSTEM
APPAREIL MÉDICAL COMPRENANT UN SYSTÈME IMPLANTABLE ET UN SYSTÈME EXTERNE

(30) Priority: 06.02.2015 US 201562112858 P
(43) Date of publication of application: 13.12.2017
(73) Proprietor: Nalu Medical, Inc., Carlsbad, CA 92008 (US)
(72) Inventor: PIVONKA, Daniel, Palo Alto, CA 94306 (US); YAKOVLEV, Anatoly, Santa Clara, CA 95054 (US); HARTLEY, Lee, Fason, Carlsbad, CA 92009 (US); FLAHERTY, R., Maxwell, Auburndale, FL 33823 (US); FLAHERTY, J., Christopher, Auburndale, FL 33823 (US)
(74) Representative: Clark, Jane Anne
(86) International application number: PCT/US2016/016888
(87) International publication number: WO 2016/127130

(56) References cited:
- WO-A1-2014/153219
- WO-A1-2014/153228
- US-A1- 2008 300 660
- US-A1- 2009 281 597
- US-A1- 2010 161 002
- US-A1- 2012 004 708
- US-A1- 2012 004 708
- US-A1- 2012 283 800
- US-A1- 2014 275 847
- US-A1- 2014 288 393

## Description

### Field of the Invention

The present invention relates generally to medical apparatus for a patient, and in particular, apparatus that include portions implanted in a patient and portions external to the patient.

### BACKGROUND OF THE INVENTION

Implantable devices that treat a patient and/or record patient data are known. For example, implants that deliver energy such as electrical energy, or deliver agents such as pharmaceutical agents are commercially available. Implantable electrical stimulators can be used to pace or defibrillate the heart, as well as modulate nerve tissue (e.g. to treat pain). Most implants are relatively large devices with batteries and long conduits, such as implantable leads configured to deliver electrical energy or implantable tubes (i.e. catheters) to deliver an agent. These implants require a fairly invasive implantation procedure, and periodic battery replacement, which requires additional surgery. The large sizes of these devices and their high costs have prevented their use in a variety of applications.

Nerve stimulation treatments have shown increasing promise recently, showing potential in the treatment of many chronic diseases including drug-resistant hypertension, motility disorders in the intestinal system, metabolic disorders arising from diabetes and obesity, and both chronic and acute pain conditions among others. Many of these implantable device configurations have not been developed effectively because of the lack of miniaturization and power efficiency, in addition to other limitations.

There is a need for apparatus, systems and devices that provide one or more implantable devices and are designed for simplicity of implantation and use, as well as enhanced flexibility and capability in treating patients and/or recording patient data.

WO 2014/153219 describes a method for treating urological disorders in a patient, the method including: placing an introducer into a patient's body through an incision site on the patient's body, the patient suffering from an urological disorder; placing an implantable wireless device into an inner lumen of the introducer, the implantable wireless device suitable to fit into the inner lumen and configured to receive electromagnetic energy non-inductively through the inner lumen of the introducer, positioning the implantable wireless device adjacent to or near one or more excitable tissue in the patient, the one or more excitable tissue regulating a nerve activity associated with the urological disorder; and causing neural modulation of the one or more excitable tissue through one or more electrodes on the implantable wireless device.

WO 2014/153228 describes an implantable wirelessly powered device that includes: one or more electrodes configured to apply one or more electrical pulses to an excitable tissue; and a first antenna configured to: receive, from a second antenna and through electrical radiative coupling, an input signal containing electrical energy, the second antenna being physically separate from the implantable device; and one or more circuits electrically connected to the first antenna, the circuits configured to: create the one or more electrical pulses suitable for stimulation of excitable tissue using the electrical energy contained in the input signal; and supply the one or more electrical pulses to the one or more electrodes, wherein the implantable device is shaped and arranged for delivery into a subject's body through an introducer or a needle of 18 gauge or smaller.

### SUMMARY

The invention is defined in claim 1. Further aspects and preferred embodiments are defined in the appended claims. Aspects, embodiments and examples of the present disclosure which do not fall under the scope of the appended claims do not form part of the invention and are merely provided for illustrative purposes. Furthermore, the methods presented in the present description are provided for illustrative purposes only and do not form part of the present invention. Described herein are apparatus, systems and devices for treating patient and/or recording patient data. According to one aspect of the present inventive concepts, a medical apparatus for a patient comprises an external system configured to transmit one or more transmission signals, each transmission signal comprising at least power or data. The medical apparatus further comprises an implantable system configured to receive the one or more transmission signals from the external system and the external system comprises a first external device comprising at least one external antenna configured to transmit a first transmission signal to the implantable system, the first transmission signal comprising at least power or data. The external system further comprises an external transmitter configured to drive the at least one external antenna, an external power supply configured to provide power to at least the external transmitter, and an external controller configured to control the external transmitter. The implantable system comprises a first implantable device comprising at least one implantable antenna configured to receive the first transmission signal from the first external device, an implantable receiver configured to receive the first transmission signal from the at least one implantable antenna, at least one implantable functional element configured to interface with the patient, an implantable controller configured to control the at least one implantable functional element, an implantable energy storage assembly configured to provide power to an element selected from the group consisting of: the at least one implantable functional element; the implantable controller; the implantable receiver; and combinations thereof; and an implantable housing surrounding at least the implantable controller and the implantable receiver. The external transmitter can operate in a frequency range between 0.1GHz and 3.0GHz. The external system can asynchronously transfer data to the implantable system. The implantable controller can monitor power transfer in real time and adjust the one or more transmissions from the external system to improve efficiency.

In some embodiments, the external system one or more transmission signals comprise both power and data. The at least one external antenna first transmission signal can comprise both power and data. The implantable energy storage assembly can be further configured to receive power from the first transmission signal.

In some embodiments, the medical apparatus comprises a patient treatment apparatus.

In some embodiments, the medical apparatus comprises a patient information recording apparatus. The medical apparatus can be configured to perform a patient diagnosis based on recorded patient information.

In some embodiments, the apparatus is configured to treat pain. The apparatus can be configured to treat back pain. The apparatus can be configured to treat a pain type selected from the group consisting of: back pain, joint pain; neuropathic pain; tennis elbow; muscle pain; shoulder pain; chronic, intractable pain of the back and/or lower limbs including unilateral or bilateral pain; neuropathic groin pain; perineal pain; phantom limb pain; complex regional pain syndrome; failed back surgery syndrome, cluster headaches; migraines; inflammatory pain; arthritis; abdominal pain; pelvic pain; and combinations thereof.

In some embodiments, the apparatus is configured to treat a patient disease or disorder selected from the group consisting of: chronic pain; acute pain; migraine; cluster headaches; urge incontinence; fecal incontinence; bowel disorders; tremor; obsessive compulsive disorder; depression; epilepsy; inflammation; tinnitus; high blood pressure; heart failure; carpal tunnel syndrome; sleep apnea; obstructive sleep apnea; dystonia; interstitial cystitis; gastroparesis; obesity; mobility issues; arrhythmia; rheumatoid arthritis; dementia; Alzheimer's disease; eating disorder; addiction; traumatic brain injury; chronic angina; congestive heart failure; muscle atrophy; inadequate bone growth; post-laminectomy pain; liver disease; Crohn's disease; irritable bowel syndrome; erectile dysfunction; kidney disease; and combinations thereof.

In some embodiments, the apparatus is configured to treat heart failure of the patient. The first implantable device can be configured to stimulate the spinal cord of the patient. The first implantable device can be configured to stimulate tissue selected from the group consisting of: spinal canal; nerves in the spinal canal; nerves in the epidural space; peripheral nerves; posterior spinal nerve root; dorsal root; dorsal root ganglion; pre-ganglionic tissue on posterior spinal nerve root; post-ganglionic tissue on posterior nerve root; dorsal ramus; grey ramus communicans; white ramus communicans; ventral ramus; and combinations thereof. The first implantable device can be configured to stimulate tissue to enhance a cardiac treatment. The first implantable device can be configured to stimulate multiple spinal locations to enhance the cardiac treatment. The apparatus can be further configured to record at least one of a heart parameter or a spine parameter, and the first implantable device can be configured to deliver stimulation energy based on the recorded parameter.

In some embodiments, the apparatus is configured to at least one of pace or defibrillate the heart of the patient.

In some embodiments, the apparatus is configured to record a patient parameter. The patient parameter can comprise a parameter selected from the group consisting of: blood glucose; blood pressure; EKG; heart rate; cardiac output; oxygen level; pH level; pH of blood; pH of a bodily fluids; temperature; inflammation level; bacteria level; type of bacteria present; gas level; blood gas level; neural activity; neural spikes; neural spike shape; action potential; local field potential (LFP); EEG; muscular activity; gastric volume; peristalsis rate; impedance; tissue impedance; electrode-tissue interface impedance; physical activity level; pain level; body position; body motion; organ motion; respiration rate; respiration level; perspiration rate; sleep level; sleep cycle; digestion state; digestion level; urine production; urine flow; bowel movement; tremor; ion concentration; chemical concentration; hormone level; viscosity of a bodily fluid; patient hydration level; and combinations thereof. The first implantable device can comprise an implantable sensor configured to measure the patient parameter. The first external device can comprise an external or implantable sensor configured to measure the patient parameter.

In some embodiments, the apparatus is configured to prevent a foreign electromagnetic field from causing the stimulation of patient tissue.

In some embodiments, the apparatus is configured to deliver an agent into the patient. The apparatus can be configured to deliver an agent into a patient location selected from the group consisting of: blood vessel; vein; subcutaneous tissue; an organ; and combinations thereof.

In some embodiments, the apparatus is configured to monitor a patient parameter and deliver an agent based on the monitored patient parameter. The monitored patient parameter can comprise blood glucose and the delivered agent can comprise insulin. The first external device can comprise a sensor configured to monitor the patient parameter. The first implantable device can comprise a sensor configured to monitor the patient parameter. The first external device can comprise an agent delivery element configured to deliver the agent. The first implantable device can comprise an agent delivery element configured to deliver the agent.

In some embodiments, apparatus is configured to perform a function selected from the group consisting of: sense a blood parameter such as white blood cell count and delivering a chemotherapeutic or other agent based on the blood parameter; sensing a hormone level and delivering a hormone or a hormone affecting agent; and combinations thereof.

In some embodiments, the medical apparatus is configured to cause stochastic resonance. The apparatus can be configured to add white noise to perform a function selected from the group consisting of: enhance sensitivity of nerves to be stimulated; boost a weak signal to be recorded; and combinations thereof.

In some embodiments, the first implantable device at least one implantable functional element is configured to stimulate tissue. The first implantable device at least one implantable functional element can be configured to stimulate tissue by delivering energy to tissue. The at least one implantable functional element can be configured to deliver energy selected from the group consisting of: electrical energy; light energy; laser light energy; sound energy; subsonic sound energy; ultrasonic sound energy; electromagnetic energy; magnetic energy; mechanical energy; heat energy; cryogenic energy; and combinations thereof.

In some embodiments, the first implantable device at least one implantable functional element is configured to record electrical activity of tissue.

In some embodiments, the first implantable device at least one implantable functional element is configured to interface with spinal cord tissue.

In some embodiments, the first implantable device at least one implantable functional element is configured to interface with nerve tissue.

In some embodiments, the first implantable device at least one implantable functional element is configured to interface with tissue selected from the group consisting of: spinal cord tissue; spinal canal tissue; epidural space tissue; nerve tissue; and combinations thereof.

In some embodiments, the first implantable device at least one implantable functional element is configured to interface with tissue selected from the group consisting of: one or more nerves; one or more locations along, in and/or proximate to the spinal cord; peripheral nerves of the spinal cord including locations around the back; the tibial nerve (and/or sensory fibers that lead to the tibial nerve); the occipital nerve; the sphenopalatine ganglion; the sacral and/or pudendal nerve; brain tissue, such as the thalamus; baroreceptors in a blood vessel wall, such as in the carotid artery; one or more muscles; the medial nerve; the hypoglossal nerve and/or one or more muscles of the tongue; cardiac tissue; the anal sphincter; the dorsal root ganglion; motor nerves; muscle tissue; spine; vagus nerve; renal nerve; organ; heart; liver; kidney; artery; vein; bone; and combinations thereof.

In some embodiments, the transmission signal comprises data comprising configuration data configured to modify stimulation by the implantable system. The configuration data can comprise an energy delivery parameter selected from the group consisting of: initiation of energy delivery; cessation of energy delivery; amount of energy to be delivered; rate of energy delivery; amplitude of energy delivery; power of energy delivery; frequency of energy delivery; waveform shape of energy delivery; duration of energy delivery; time of energy delivery initiation; and combinations thereof. The configuration data can comprise an agent delivery parameter selected from the group consisting of: initiation of agent delivery; cessation of agent delivery; amount of agent to be delivered; volume of agent to be delivered; rate of agent delivery; duration of agent delivery; time of agent delivery initiation; and combinations thereof.

In some embodiments, the transmission signal comprises data comprising configuration data configured to modify sensing by the implantable system. The configuration data can comprise data selected from the group consisting of: initiation of sensor recording; cessation of sensor recording; frequency of sensor recording; resolution of sensor recording; thresholds of sensor recording; sampling frequency of sensor recording; dynamic range of sensor recording; initiation of calibration of sensor recording; and combinations thereof.

In some embodiments, the medical apparatus further comprises a sensor configured to produce a sensor signal, and the first implantable device is configured to perform closed-loop energy delivery based on the sensor signal. The sensor can comprise multiple sensors. The first implantable device at least one implantable functional element can comprise the sensor. The sensor can be configured to assess an apparatus parameter selected from the group consisting of: power transfer; link gain; power use; implantable energy storage charge and/or discharge rate; antenna mismatch; load impedance; voltage and/or current amplitudes; instantaneous power usage; average power usage; voltage supply ripple and/or variation; battery life; bit error rate; signal integrity; and combinations thereof. The first implantable device at least one implantable functional element can comprise one or more sensors selected from the group consisting of: electrode; sensor configured to record electrical activity of tissue; blood glucose sensor; gas sensor; blood gas sensor; ion concentration sensor; oxygen sensor; pressure sensor; blood pressure sensor; heart rate sensor; cardiac output sensor; inflammation sensor; neural activity sensor; neural spike sensor; muscular activity sensor; gastric volume sensor; peristalsis rate sensor; pH sensor; strain gauge; accelerometer; gyroscope; GPS; respiration sensor; respiration rate sensor; flow sensor; viscosity sensor; temperature sensor; magnetic sensor; optical sensor; MEMs sensor; chemical sensor; hormone sensor; impedance sensor; tissue impedance sensor; electrode-tissue interface impedance sensor; body position sensor; body motion sensor; organ motion sensor; physical activity level sensor; perspiration sensor; patient hydration sensor; breath monitoring sensor; sleep monitoring sensor; food intake monitoring sensor; digestion monitoring sensor; urine movement sensor; bowel movement sensor; tremor sensor; pain level sensor; and combinations thereof. The first implantable device at least one implantable functional element can comprise one or more sensors configured to record a patient parameter selected from the group consisting of: blood glucose; blood pressure; EKG; heart rate; cardiac output; oxygen level; pH level; pH of blood; pH of a bodily fluids; tissue temperature; inflammation level; bacteria level; type of bacteria present; gas level; blood gas level; neural activity; neural spikes; neural spike shape; action potential; local field potential (LFP); EEG; muscular activity; gastric volume; peristalsis rate; impedance; tissue impedance; electrode-tissue interface impedance; physical activity level; pain level; body position; body motion; organ motion; respiration rate; respiration level; perspiration rate; sleep level; sleep cycle; digestion state; digestion level; urine production; urine flow; bowel movement; tremor; ion concentration; chemical concentration; hormone level; viscosity of a bodily fluid; patient hydration level; and combinations thereof.

In some embodiments, the first implantable device further comprises a flexible conduit connecting the at least one implantable antenna and the implantable housing. The flexible conduit can comprise one or more coaxial cables. The flexible conduit can comprise a length between 0.5cm and 60cm, such as a length between 0.5cm and 10cm or between 0.5cm and 5cm.

In some embodiments, the at least one implantable antenna comprises a first implantable antenna and a second implantable antenna, each configured to receive one or more of the transmission signals from the external system. The first implantable antenna and the second implantable antenna can be connected to the implantable receiver in series. The first implantable antenna and the second implantable antenna can be connected to the implantable receiver in parallel. The first implantable antenna and the second implantable antenna can be separately connected to the implantable receiver. The implantable receiver can comprise a first implantable receiver connected to the first implantable antenna and a second implantable receiver connected to the second implantable antenna. The first implantable antenna and the second implantable antenna can be arranged in an X-pattern. The first implantable device implantable receiver can be configured to receive one or more of the transmission signals from the second implantable antenna. The first implantable antenna and the second implantable antenna can each be positioned within the first implantable device implantable housing. The second implantable antenna can be positioned relatively orthogonal to the first implantable antenna. The first implantable antenna can be positioned in a first plane, the second implantable antenna can be positioned in a second plane, and the first plane can be oriented between 30° and 90° relative to the second plane. The first implantable antenna can be positioned in a first plane, the second implantable antenna can be positioned in a second plane, and the first plane can be oriented between 40° and 90° relative to the second plane.

In some embodiments, the medical apparatus further comprises a substrate, and the at least one implantable antenna is positioned on the substrate. The substrate can comprise a printed circuit board. The substrate can comprise a foldable substrate. The substrate can comprise a flexible substrate. The first implantable device at least one implantable antenna can comprise a first implantable antenna and a second implantable antenna, and the first implantable antenna can be positioned on the substrate in a first plane, and the second implantable antenna can be positioned on the substrate in a second plane, and the first plane and the second plane can comprise different planes. The first plane can be oriented relative to the second plane by an angle selected from the group consisting of: between 5° and 90°; between 40° and 90°; approx. 30°; approx. 60°; and approx. 90°. The first implantable device at least one implantable antenna can further comprise at least a third implantable antenna positioned on the substrate in a third plane, and the third plane can be different than the first plane.

In some embodiments, the first implantable device at least one implantable antenna comprises a first implantable antenna configured to one or more of the external system transmission signals, a second implantable antenna configured to receive one or more of the external system transmission signals, and a third implantable antenna configured to receive one or more of the external system transmission signals. The implantable receiver can be configured to receive a transmission signal from the first implantable antenna, a transmission signal from the second implantable antenna, and a transmission signal from the third implantable antenna. The first implantable antenna, the second implantable antenna, and the third implantable antenna can each be positioned within the first implantable device implantable housing. The first implantable antenna can comprise an electrical dipole antenna. The second implantable antenna and the third implantable antenna can each be positioned orthogonal to the electrical dipole antenna. The second implantable antenna can comprise a loop antenna, and the third implantable antenna can comprise a loop antenna. The second implantable antenna can be positioned relatively orthogonal to the third implantable antenna. The first implantable antenna can be positioned outside the first implantable device implantable housing and the second and third implantable antennas are positioned within the first implantable device implantable housing. The first implantable antenna, the second implantable antenna and the third implantable antenna can each comprise a loop antenna positioned in different planes. The first implantable antenna, the second implantable antenna and the third implantable antenna can each comprise a loop antenna positioned orthogonal to each other. The first implantable antenna, the second implantable antenna and the third implantable antenna can each be positioned on a substrate. The substrate can comprise a printed circuit board. The substrate can comprise a foldable substrate. The substrate can comprise a flexible substrate. The substrate can comprise a first portion positioned in a first plane, a second portion positioned in a second plane, and a third portion positioned in a third plane, and the first implantable antenna can be positioned on the substrate first portion, the second implantable antenna can be positioned on the substrate second portion, and the third implantable antenna can be positioned on the substrate third portion. The first, second and third planes can be oriented between 40° and 90° relative to each other. The first, second and third planes can be oriented approximately 45° relative to each other. The first, second and third planes can be oriented approximately 60° relative to each other.

In some embodiments, the first implantable device at least one implantable antenna comprises an antenna selected from the group consisting of: loop antenna; multiple-turn loop antenna; planar loop antenna; coil antenna; dipole antenna; electric dipole antenna; magnetic dipole antenna; patch antenna; loaded dipole antenna; concentric loop antenna; loop antenna with ferrite core; and combinations thereof.

In some embodiments, the first implantable device at least one implantable antenna comprises a loop antenna. The first implantable device at least one implantable antenna can comprise an elongated loop antenna.

In some embodiments, the first implantable device at least one implantable antenna comprises a multiple-turn loop antenna.

In some embodiments, the first implantable device at least one implantable antenna comprises a minor axis with a length between 1.0mm and 8.0mm. The first implantable device at least one implantable antenna can comprise a minor axis with a length between 2.0mm and 5.0mm.

In some embodiments, the first implantable device at least one implantable antenna comprises a major axis with a length between 3.0mm and 15.0mm. The first implantable device at least one implantable antenna can comprise a major axis with a length between 4.0mm and 8.0mm. The first implantable device at least one implantable antenna can comprise a major axis with a length between 3.0mm and 15.0mm.

In some embodiments, the first implantable device at least one implantable antenna comprises an unfoldable antenna.

In some embodiments, the first implantable device at least one implantable antenna is positioned within the implantable housing.

In some embodiments, the first implantable device at least one implantable antenna is positioned outside of the implantable housing.

In some embodiments, the first implantable device at least one implantable antenna comprises a first implantable antenna positioned inside of the implantable housing and one implantable antenna positioned outside of the implantable housing.

In some embodiments, the first implantable device at least one implantable antenna is configured to be positioned below the skin surface at a distance between 0.5cm and 7.0cm. The first implantable device at least one implantable antenna can be configured to be positioned below the skin surface at a distance between 1.0cm and 3.0cm.

In some embodiments, the first transmission signal comprises a wavelength λ, and the first implantable device at least one implantable antenna is configured to be positioned in the patient at a distance of between 0.1 λ and 10.0 λ from the first external device at least one external antenna. The first implantable device at least one implantable antenna can be configured to be positioned in the patient at a distance of between 0.2 λ and 2.0 λ from the at least one external antenna. The first external device at least one external antenna can be configured to transmit a transmission signal with a frequency between 0.1GHz and 3.0GHz. The first external device at least one external antenna can be configured to transmit a transmission signal with a frequency between 0.4GHz and 1.50GHz. The first external device at least one external antenna can be configured to transmit a transmission signal with a frequency between approximately 0.902GHz and 0.928GHz.

In some embodiments, the first implantable device at least one implantable antenna is further configured to transmit a data signal to the external system. The first implantable device at least one implantable antenna can be configured to transmit the data signal to the at least one external antenna. The data signal transmitted by the first implantable device at least one implantable antenna can comprise at least one of patient information or implantable system information.

In some embodiments, the implantable system comprises a second implantable device comprising a second implantable receiver and at least one implantable antenna configured to receive a transmission signal from the external system, and the second implantable device second implantable receiver is configured to receive the transmission signal from the second implantable device at least one implantable antenna. The second implantable device can comprise a second implantable housing surrounding the second implantable receiver and the second implantable device at least one implantable antenna. The first implantable device and the second implantable device can each comprise a unique ID, and the external system can communicate with the first implantable device and the second implantable device independently.

In some embodiments, the implantable system further comprises a second implantable device comprising a second implantable receiver and at least one implantable antenna configured to receive one or more transmission signals from the external system. The implantable system can further comprise a third implantable device comprising a third implantable receiver and at least one implantable antenna configured to receive one or more transmission signals from the external system, and the second implantable device second receiver can be configured to receive a transmission signal from the second implantable device at least one implantable antenna, and the third implantable device third receiver can be configured to receive a transmission signal from the third implantable device at least one implantable antenna. The second implantable device can comprise a second implantable housing surrounding the second implantable receiver and second implantable device at least one implantable antenna, and the third implantable device can comprise a third implantable housing surrounding the third implantable device at least one implantable antenna.

In some embodiments, the implantable receiver is configured to recover data without synchronizing to a received transmission signal. The transmitted signal can comprise a power signal, and a clock and/or data can be recovered without synchronizing to the power signal. The transmitted signal can comprise a clock and/or data signal, and a clock and/or data can be recovered without synchronizing to the transmitted clock and/or data signal. The recovered signal can comprise a clock and/or data and a clock and/or data can be recovered from the transmission signal without synchronizing to the recovered clock and/or data.

In some embodiments, the implantable receiver comprises a power signal rectifier. The power signal rectifier can be configured to provide power to at least one of the implantable energy storage assembly or the controller. The implantable receiver can further comprise a DC-DC voltage converter. The power signal rectifier can comprise at least one self-driven synchronous rectifier. The at least one self-driven synchronous rectifier is connected in a charge-pump configuration. The power signal rectifier can further comprise a DC-DC converter configured to boost voltage supplied to the implantable energy storage assembly. The power signal rectifier can comprise at least one diode-capacitor ladder stage. The power signal rectifier can comprise as least one rectification element configured to at least one of: minimize forward conduction losses or minimize reverse conduction losses.

In some embodiments, the implantable receiver comprises an implantable data receiver. The implantable data receiver can comprise a demodulator as described herebelow, such as a demodulator comprising an envelope detector. The implantable data receiver can comprise an envelope averaging circuit.

In some embodiments, the implantable receiver is further configured to drive the first implantable device at least one implantable antenna to provide a data signal to the external system.

In some embodiments, the implantable receiver comprises a matching network. The matching network can be configured to detune to prevent oversaturation. The medical apparatus can further comprise a second implantable device comprising a second implantable receiver comprising a matching network, and the first implantable device implantable receiver's matching network can be configured to detune based on power received by the second implantable device second implantable receiver.

In some embodiments, the implantable receiver is configured to alter load impedance to backscatter energy.

In some embodiments, the implantable receiver is configured to manipulate a signal at a tissue interface to transmit a data signal through body conduction.

In some embodiments, the implantable receiver comprises an implantable power converter. The implantable power converter can comprise a component selected from the group consisting of: buck-boost converter; DC-DC converter; boost converter; switched capacitor; charge pump; and combinations thereof. The implantable power converter can be configured to interface with the implantable energy storage assembly. The implantable power converter can be configured to receive a signal from the implantable controller, and adjust one or more of: voltage; current; charge rate; discharge rate; switching frequency; and combinations thereof.

In some embodiments, the implantable energy storage assembly comprises a first implantable energy storage assembly configured to provide power to the implantable controller and/or the implantable receiver, and a second implantable energy storage assembly configured to provide power to the at least one implantable functional element.

In some embodiments, the implantable energy storage assembly is further configured to provide power to the first implantable device at least one implantable antenna.

In some embodiments, the implantable energy storage assembly comprises at least one capacitor. The at least one capacitor can comprise a capacitance between 0.01µF and 10F. The at least one capacitor can comprise a capacitance between 0.1µF and 1.0µF. The at least one capacitor can comprise a breakdown voltage of at least 1.0V. The at least one capacitor can comprise a breakdown voltage of at least 1.5V. The at least one capacitor can comprise a breakdown voltage of at least 4.0V. The at least one capacitor can comprise a breakdown voltage of at least 10V. The at least one capacitor can comprise a breakdown voltage of at least 15V. The at least one capacitor can comprise multiple capacitors. The implantable energy storage assembly can comprise at least one battery. The battery can comprise a rechargeable battery.

In some embodiments, the transmission signal comprises a power signal, and the implantable controller is configured to transmit a stimulation signal to the at least one implantable functional element, and the stimulation signal is independent of the power signal. The stimulation signal and the power signal can differ in one or more of: amplitude; changes in amplitude; average amplitude; frequency; changes in frequency; average frequency; phase; changes in phase; average phase; waveform shape; pulse shape; duty cycle; polarity; and combinations thereof. The stimulation signal parameters can be independent of the received transmission signal parameters. The stimulation signal parameters can comprise one or more parameters selected from the group consisting of: amplitude; frequency; duty cycle; polarity; pulse shape; and combinations thereof, and the received transmission signal parameters comprise one or more parameters selected from the group consisting of: amplitude, frequency; duty cycle; phase; envelope; and combinations thereof.

In some embodiments, the implantable controller receives commands from the implantable receiver. The commands can adjust one or more of: stimulation parameter; data rate of receiver; data rate of data transmitted by the first implantable device at least one implantable antenna; implantable functional element configuration; state of a controller; antenna impedance; clock frequency; sensor configuration; electrode configuration; power management parameters; implantable energy storage assembly parameters; agent delivery parameter; sensor configuration parameter; and combinations thereof.

In some embodiments, the implantable controller controls the energy sent to the at least one implantable functional element. The implantable controller can adjusts an energy parameter selected from the group consisting of: amplitude; frequency; pulse width; voltage; current; pulse shape; duty cycle; polarity; drive impedance; implantable energy storage assembly capacity; and combinations thereof.

In some embodiments, the implantable controller controls charge balance. The implantable controller can actively control charge balance. Numerous configurations for controlling charge balance are described herebelow.

In some embodiments, the implantable controller further comprises a matching network configured to match the impedance of the first implantable device at least one implantable antenna with the impedance of the implantable receiver. The matching network can comprise an adjustable matching network.

In some embodiments, the implantable controller is configured to produce a stimulation signal for the at least one implantable functional element. The stimulation signal can comprise a waveform selected from the group consisting of: square wave; sine wave; triangle wave; ramp; waveform with exponential increase; waveform with exponential decrease; pulse shape which minimizes power consumption; Gaussian pulse shape; pulse train; root-raised cosine; bipolar pulses; and combinations thereof. The stimulation signal can comprise a combination of two or more waveforms selected from the group consisting of: square wave; sine wave; triangle wave; ramp; waveform with exponential increase; waveform with exponential decrease; pulse shape which minimizes power consumption; Gaussian pulse shape; pulse train; root-raised cosine; bipolar pulses; and combinations thereof. The stimulation signal can comprise a stimulation signal with a frequency component between 1.0Hz and 50kHz. The stimulation signal can comprise a stimulation signal with a frequency component between 10Hz and 500Hz, between 40Hz and 160Hz and/or between 5kHz and 15kHz. The frequency component can comprise one or more waveforms or pulses that are repeated at frequencies between 1.0Hz and 50kHz, such as between 10Hz and 500Hz, between 40Hz and 160Hz and/or between 5kHz and 15kHz. The stimulation signal can comprise a stimulation signal with a duty cycle between 0.1% and 25%. The stimulation signal can comprise a frequency modulated stimulation waveform. The frequency modulated stimulation signal waveform can comprise a frequency component between 1kHz and 50kHz. The stimulation signal can comprise a mix of low frequency signals and high frequency signals. The low frequency signals can comprise one or more signals with a frequency between 1Hz and 1000Hz, and the high frequency signals can comprise one or more signals with a frequency between 1kHz and 50kHz. The stimulation signal can comprise a train of high frequency signals and bursts of low frequency signals. The stimulation signal can comprise a train of low frequency signals and bursts of high frequency signals. The stimulation signal can comprise one or more high frequency signals modulated with one or more low frequency signals. The stimulation signal can comprise a first signal comprising at least one high frequency waveform and a second signal comprising at least one low frequency waveform, and the first signal and second signal can be independently controllable. The stimulation signal can comprise a signal that cycles among different waveform shapes at specified time intervals. The stimulation signal can comprise at least one of a pseudo random binary sequence non-return-to-zero waveform or a pseudo random binary sequence return-to-zero waveform.

In some embodiments, the implantable controller comprises a clamping circuit configured to at least one of fast-charge or fast-discharge of at least a portion of the first implantable device, such as at least the implantable energy storage assembly. The clamping circuit can be configured to limit at least one of rise time or fall time to be less than or equal to 10% of the pulse width of an applied stimulation pulse. The clamping circuit can be configured to limit at least one of rise time or fall time to be less than or equal to 1µsecond.

In some embodiments, implantable controller comprises an adjustable loading impedance configured to stabilize the load seen on the at least one implantable antenna. The adjustable loading impedance can be controlled by the charge rate of the implantable energy storage assembly.

In some embodiments, the implantable controller comprises an integrated circuit. The integrated circuit can comprise one or more components selected from the group consisting of: matching network; rectifier; DC-DC converter; regulator; bandgap reference; overvoltage protection; overcurrent protection; active charge balance circuit; analog to digital converter (ADC); digital to analog converter (DAC); current driver; voltage driver; digital controller; clock generator; data receiver; data demodulator; data modulator; data transmitter; electrode drivers; sensing interface analog front end; power management circuit; implantable energy storage assembly interface; memory register; timing circuit; and combinations thereof.

In some embodiments, the at least one implantable functional element is configured to interface with tissue of the patient.

In some embodiments, the at least one implantable functional element is configured to interface with a patient location.

In some embodiments, the at least one implantable functional element comprises multiple implantable functional elements. The multiple implantable functional elements can comprise multiple stimulation elements. The multiple implantable functional elements can comprise multiple sensors. The multiple implantable functional elements can comprise at least one stimulation element and at least one sensor. At least one single implantable functional element can be configured as a sensor and a stimulation element. The at least one implantable functional element can comprise at least one stimulation element. The at least one stimulation element can comprise one or more elements selected from the group consisting of: an electrode; an energy delivery element; an electrical energy delivery element; a light energy delivery element; a laser light energy delivery element; a sound energy delivery element; a subsonic sound energy delivery element; an ultrasonic sound delivery element; an electromagnetic field generating element; a magnetic field generating element; a mechanical transducer; a tissue manipulating element; a heat generating element; a cooling element; an agent delivery element; a pharmaceutical drug delivery element; and combinations thereof.

In some embodiments, the at least one implantable functional element comprises one or more implantable functional elements configured to record a patient parameter and treat the patient. The at least one implantable functional element can comprise one or more electrodes. The at least one implantable functional element can be configured to treat the patient by performing a function selected from the group consisting of: delivering energy; delivering an agent; and combinations thereof. The at least one implantable functional element can comprise one or more electrodes configured to deliver electrical energy and sense electrical activity. The medical apparatus can be configured to deliver energy as part of stochastic resonance.

In some embodiments, the first implantable device at least one implantable functional element comprises at least one electrode. The at least one electrode can comprise one or more electrodes selected from the group consisting of: microelectrode; cuff electrode; array of electrodes; linear array of electrodes; circular array of electrodes; paddle-shaped array of electrodes; bifurcated electrodes; and combinations thereof. The first implantable device can further comprise an implantable lead. The first implantable device at least one implantable functional element can be positioned on the implantable lead. The implantable lead can comprise a lumen and a stylet insertable into the lumen. The first implantable device at least one stimulation element can be positioned on the implantable housing. The at least one implantable functional element can comprise an agent delivery element. The agent delivery element can comprise an element selected from the group consisting of: catheter; needle; iontophoretic element; porous membrane; and combinations thereof.

In some embodiments, the first implantable device further comprises an implantable lead. The implantable lead can comprise the at least one implantable functional element. The implantable lead can comprise a lumen and a stylet insertable into the lumen. The medical apparatus can further comprise a second implantable device comprising a second at least one implantable functional element and a second implantable lead, and the second implantable lead can comprise the second at least one implantable functional element. The implantable lead can comprise a diameter between 1mm and 4mm. The implantable lead can comprise a diameter between 1mm and 2mm. The implantable lead can comprise a length between 3cm and 60cm. The implantable lead can comprise a length between 6cm and 30cm. The at least one implantable functional element can comprise between 2 and 64 implantable functional elements positioned on the implantable lead. The at least one of the implantable functional elements can comprise an electrode. The at least one implantable functional element can comprise between 4 and 32 implantable functional elements positioned on the implantable lead. The implantable lead can comprise an implantable paddle lead. The implantable lead can comprise a catheter constructed and arranged to deliver an agent to the patient.

In some embodiments, the at least one implantable functional element is positioned on the implantable housing.

In some embodiments, the implantable housing further surrounds the first implantable device at least one implantable antenna.

In some embodiments, the implantable housing further surrounds the implantable energy storage assembly.

In some embodiments, the implantable housing further surrounds at least a portion of the at least one implantable functional element.

In some embodiments, the first implantable device further comprises a printed circuit boat, and the implantable housing further surrounds the printed circuit board. The printed circuit board can comprise multiple discrete printed circuit boards, each surrounded by the implantable housing. The multiple discrete printed circuit boards can each be on a different plane. The printed circuit board can comprise a folded printed circuit board. The first implantable device at least one implantable antenna can be positioned on the printed circuit board. The first implantable device at least one implantable antenna can comprise multiple implantable antennas positioned on the printed circuit board. The printed circuit board can comprise a flexible printed circuit board.

In some embodiments, the implantable housing comprises material transmissive to RF signals.

In some embodiments, the implantable housing comprises one or more materials selected from the group consisting of: glass; ceramic; stainless steel; titanium; polyurethane; an organic compound; liquid crystal polymer (LCP); gold; platinum; tungsten; and combinations thereof.

In some embodiments, the implantable housing comprises two discrete components sealed to each other.

In some embodiments, the implantable housing comprises three discrete components sealed to each other.

In some embodiments, the implantable housing comprises one or more feedthroughs. The implantable device can further comprise one or more conduits, and the feedthroughs collectively surround the one or more conduits. The one or more conduits can comprise one or more conduits selected from the group consisting of: lead; wire; optical fiber; fluid delivery tube; mechanical linkage; and combinations thereof.

In some embodiments, the implantable housing comprises a major axis with a length less than or equal to 20mm. The implantable housing major axis can comprise a length less than or equal to 15mm. The implantable housing major axis can comprise a length less than or equal to 12mm. The implantable housing major axis can comprise a length less than or equal to 10mm.

In some embodiments, the implantable housing comprises a minor axis with a length less than or equal to 8mm. The implantable housing minor axis can comprise a length less than or equal to 6mm, or less than or equal to 5mm.

In some embodiments, the implantable housing comprises a wall thickness between 0.2mm and 1.0mm. The implantable housing can comprise a wall thickness between 0.2mm and 0.5mm. The implantable housing can comprise a wall thickness of approximately 0.3mm.

In some embodiments, the implantable housing comprises a displacement volume less than or equal to 2000mm3. The implantable housing can comprise a displacement volume less than or equal to 600mm3.

In some embodiments, the implantable housing comprises an anchoring element. The anchoring element can comprise an element selected from the group consisting of: silicone sleeve; suture tab; suture eyelet; bone anchor, wire loops; porous mesh; penetrable wing; penetrable tab; bone screw eyelet; tine; pincers; suture slits; and combinations thereof.

In some embodiments, the implantable housing comprises a geometric shape similar to a shape selected from the group consisting of: disc; pill; cylinder; sphere; rectangular prism; trapezoidal prism; a portion of a toroid; and combinations thereof.

In some embodiments, the implantable housing comprises a thermal shielding layer over at least a portion of the implantable housing.

In some embodiments, the implantable housing comprises an electromagnetic shielding layer over at least a portion of the implantable housing.

In some embodiments, the first implantable device further comprises an implantable transmitter configured to transmit data to the external system. The implantable transmitter can be configured to drive the first implantable device at least one implantable antenna. The implantable transmitter can be configured to transmit data using one or more of: load modulation; a signal carrier; and/or body conduction. The implantable transmitter can be configured to have an adjustable parameter, the adjustable parameter selected from the group consisting of: data rate; pulse width; duration of carrier signal; amplitude of carrier signal; frequency of carrier signal; configurable load; and combinations thereof.

In some embodiments, the first implantable device further comprises an implantable sensor. The implantable sensor can comprise multiple sensors. The at least one implantable functional element can comprise the implantable sensor. The at least one implantable functional element can comprise a stimulation element and a sensing element. The at least one implantable functional element can comprise an electrode configured to deliver stimulation energy and record data representing electrical activity of tissue. The implantable sensor can comprise a sensor configured to record data representing a patient physiologic parameter. The implantable sensor can comprise a sensor selected from the group consisting of: electrode; sensor configured to record electrical activity of tissue; blood glucose sensor such as an optical blood glucose sensor; pressure sensor; blood pressure sensor; heart rate sensor; inflammation sensor; neural activity sensor; muscular activity sensor; pH sensor; strain gauge; accelerometer; gyroscope; GPS; respiration sensor; respiration rate sensor; temperature sensor; magnetic sensor; optical sensor; MEMs sensor; chemical sensor; hormone sensor; impedance sensor; tissue impedance sensor; body position sensor; body motion sensor; physical activity level sensor; perspiration sensor; patient hydration sensor; breath monitoring sensor; sleep monitoring sensor; food intake monitoring sensor; urine movement sensor; bowel movement sensor; tremor sensor; pain level sensor; and combinations thereof. The implantable sensor can comprise a sensor configured to record a patient parameter selected from the group consisting of: blood glucose; blood pressure; EKG; heart rate; cardiac output; oxygen level; pH level; pH of blood; pH of a bodily fluids; tissue temperature; inflammation level; bacteria level; type of bacteria present; gas level; blood gas level; neural activity; neural spikes; neural spike shape; action potential; local field potential (LFP); EEG; muscular activity; gastric volume; peristalsis rate; impedance; tissue impedance; electrode-tissue interface impedance; physical activity level; pain level; body position; body motion; organ motion; respiration rate; respiration level; perspiration rate; sleep level; sleep cycle; digestion state; digestion level; urine production; urine flow; bowel movement; tremor; ion concentration; chemical concentration; hormone level; viscosity of a bodily fluid; patient hydration level; and combinations thereof.

In some embodiments, the first implantable device further comprises an implantable diagnostic assembly. The implantable diagnostic assembly can be configured to assess a patient physiologic condition. The implantable diagnostic assembly can be configured to assess a condition of the first implantable device. The implantable diagnostic assembly can be configured to assess a first implantable device condition selected from the group consisting of: power level; temperature; signal integrity; antenna match; electrode impedance; charge rate; discharge rates; voltage level; current level; and combinations thereof. The implantable diagnostic assembly can be configured to assess communication between the first implantable device and the first external device. The implantable diagnostic assembly can comprise a sensor. The sensor can comprise an electrode configured to record a result of stimulation. The sensor can be configured to record one or more of: neural activity and/or muscular activity. The implantable diagnostic assembly can be configured to record impedance. The implantable diagnostic assembly can be configured to perform a frequency sweep. The implantable diagnostic assembly can be configured to perform an impulse response. The implantable diagnostic assembly can be configured to compare voltage and current of a stimulation signal.

In some embodiments, the first implantable device further comprises a trialing interface.

In some embodiments, implantable system further comprises a second implantable device comprising: at least one implantable antenna configured to receive a transmission signal from the external system; a second implantable receiver configured to receive the transmission signal from the second implantable device at least one implantable antenna; a second at least one implantable functional element configured to interface with tissue of the patient; a second implantable energy storage assembly configured to provide power to the at least one implantable functional element; and a second implantable housing surrounding the implantable receiver. The second implantable device can further comprise a second implantable controller configured to control the second at least one implantable functional element. The implantable system can be configured as a multi-point ready system. The first implantable device and the second implantable device can operate simultaneously. The first implantable device and the second implantable device can operate sequentially. The first implantable device and the second implantable device can operate asynchronously. The external system can further comprise a second external device, and the second external device can be configured to send one or more transmission signals to at least one of the first implantable device or the second implantable device. The external system can further comprise a clock, and the first external device and the second external device can be synchronized to the external system clock. The first implantable device and the second implantable device can each comprise a unique ID, and the external system can communicate with the first implantable device and the second implantable device independently. The external system can communicate with the first implantable device and the second implantable device using time-domain multiple access communication. The external system transmission signals can comprise power signals, and the first implantable device and the second implantable device can each be configured to deliver stimulation energy to tissue, and the stimulation energy can be delivered independent of the power signals. The first external device can be configured to transmit power to the second implantable device. The first external device can be further configured to transmit data to the second implantable device. The first implantable device can be configured to transmit data to the second implantable device. The external system can further comprise a second external device comprising: at least one external antenna configured to transmit a second transmission signal comprising at least one of power or data; a second external transmitter configured to drive the at least one external antenna; a second external power supply configured to provide power to at least the external transmitter; and a second external controller configured to control the external transmitter. The first external device can be configured to be positioned proximate the first implantable device and the second external device can be configured to be positioned proximate the second implantable device.

In some embodiments, external system further comprises a sensor configured to record data. The first implantable device can be configured to deliver stimulation energy based on the data recorded by the external system sensor. The external system sensor comprises a sensor selected from the group consisting of: electrode; sensor configured to record electrical activity of tissue; blood glucose sensor; blood gas sensor; oxygen sensor; pressure sensor; blood pressure sensor; pH sensor; strain gauge; accelerometer; gyroscope; GPS; respiration sensor; flow sensor; viscosity sensor; temperature sensor; optical sensor; MEMs sensor; chemical sensor; hormone sensor; impedance sensor; and combinations thereof. The external system sensor can be configured to assess a patient parameter selected from the group consisting of: blood glucose; blood pressure; EKG; heart rate; cardiac output; oxygen level; pH level; pH of blood; pH of a bodily fluids; tissue temperature; inflammation level; bacteria level; type of bacteria present; gas level; blood gas level; neural activity; neural spikes; neural spike shape; action potential; local field potential (LFP); EEG; muscular activity; gastric volume; peristalsis rate; impedance; tissue impedance; electrode-tissue interface impedance; physical activity level; pain level; body position; body motion; organ motion; respiration rate; respiration level; perspiration rate; sleep level; sleep cycle; digestion state; digestion level; urine production; urine flow; bowel movement; tremor; ion concentration; chemical concentration; hormone level; viscosity of a bodily fluid; patient hydration level; and combinations thereof. The external system sensor can comprise one or more sensors selected from the group consisting of: an energy sensor; a voltage sensor; a current sensor; a temperature sensor; an antenna matching and/or mismatching assessment sensor; power transfer sensor; link gain sensor; power use sensor; energy level sensor; energy charge rate sensor; energy discharge rate sensor; impedance sensor; load impedance sensor; instantaneous power usage sensor; average power usage sensor; bit error rate sensor; signal integrity sensor; and combinations thereof. The external system sensor can be configured to provide data such that the medical apparatus can assess one or more of: power transfer; link gain; power use; energy within an external power supply; performance of an external power supply; expected life of an external power supply; discharge rate of an external power supply; ripple or other variations of an external power supply; matching of an implantable antenna and an external antenna; communication error rate between the first implantable device and the first external device; integrity of transmission between the first implantable device and the first external device; and combinations thereof.

In some embodiments, the external system further comprises a transducer. The transducer can comprise a transducer selected from the group consisting of: light; light emitting diode; wireless transmitter; Bluetooth device; mechanical transducer; piezoelectric transducer; pressure transducer; temperature transducer; humidity transducer; vibrational transducer; audio transducer; speaker; and combinations thereof.

In some embodiments, the first external device further comprises an external housing surrounding at least the external transmitter and a flexible conduit connecting the at least one external antenna and the external housing. The flexible conduit can comprise one or more coaxial cables. The flexible conduit can comprise a length between 2 inches and 60 inches. The flexible conduit can comprise between 1 and 16 wires.

In some embodiments, the at least one external antenna comprises a polarizable antenna. The at least one external antenna can be configured to have adjustable polarization.

In some embodiments, the at least one external antenna comprises an array of external antennas. The array of external antennas can be configured to support at least one of beam shaping or focusing. The array of external antennas can be configured to adjust at least one of the amplitude or phase of the one or more transmission signals. The array of external antennas can be configured to increase a radiation footprint.

In some embodiments, the first external device at least one external antenna comprises a multi-turn spiral loop antenna configured to desensitize coupling sensitivity.

In some embodiments, the first external device at least one external antenna comprises an antenna with multiple concentric loops with varied dimensions and configured to desensitize coupling sensitivity. The multiple concentric loops can be connected in parallel and driven from the same feed point. The multiple concentric loops can be driven from the same feed point and connected using one or more components selected from the group consisting of: capacitors; inductors; varactors; and combinations thereof. The multiple concentric loops can be driven from the multiple feed points.

In some embodiments, the at least one external antenna comprises an array of selectable conductors configured to adjust at least one of a radiation pattern or an electromagnetic field of a resultant external antenna.

In some embodiments, the at least one external antenna comprises a surface and shield material positioned on the surface, and the shield material can be positioned on a side facing away from the patient's skin. The shield material can comprise at least one of: radio-absorptive shield material or radio-reflective shield material.

In some embodiments, the at least one external antenna comprises a surface and a spacer positioned on the surface, and the spacer can be configured to be positioned proximate the patient's skin. The spacer can comprise one or more materials selected to match impedance of the at least one external antenna to impedance of tissue of the patient. The spacer can comprise a thermal insulator. The spacer can comprise a thickness between 0.1cm and 3.0cm. The spacer can comprise a thickness between 0.2cm and 1.5cm. The spacer can comprise a compressible material. The spacer can comprise an inflatable spacer. The spacer can comprise multiple individually inflatable compartments.

In some embodiments, the at least one external antenna comprises a multi-feed point antenna. The multi-feed point antenna can be configured to support at least one of beam shaping or focusing. The multi-feed point antenna can be configured to allow adjustment of at least one of amplitude or phase of the one or more transmission signals. The multi-feed point antenna can be configured to increase a radiation footprint.

In some embodiments, the at least one external antenna comprises an antenna selected from the group consisting of: patch antenna; slot antenna; array of antennas; concentric loop antenna; antenna loaded with reactive elements; dipole antenna; polarizable antenna; selectable conductors that form an antenna; and combinations thereof.

In some embodiments, the at least one external antenna comprises a major axis with a length between 1cm and 10cm. The at least one external antenna can comprise a major axis with a length between 2cm and 5cm.

In some embodiments, the at least one external antenna is further configured to receive a signal. The at least one external antenna can be further configured to receive a signal from the at least one implantable antenna.

In some embodiments, the first external device comprises a flexible printed circuit board, and the at least one external antenna is positioned on the flexible printed circuit board.

In some embodiments, the implantable system comprises multiple implantable devices each comprising at least one implantable antenna, and the first external device at least one external antenna transmits at least power or data to the multiple implantable devices. The first implantable device at least one implantable antenna can comprise the multiple implantable antennas. The first external device at least one external antenna can comprise a single antenna. The first external device at least one external antenna can comprise multiple antennas.

In some embodiments, the implantable system comprises multiple implantable antennas, the first external device at least one external antenna defines a radiation footprint, and one or more of the multiple implantable antennas that receive at least one of power or data from the first external device at least one external antenna are positioned within a projection of the radiation footprint. The projection cross sectional area can change with depth into the patient. The first external device at least one external antenna can comprise a single external antenna. The first external device at least one external antenna can comprise multiple external antennas. The multiple implantable antennas receiving at least one of power or data can be surrounded by the implantable housing of the first implantable device. The implantable system can further comprise a second implantable device, and the one or more of the multiple implantable antennas receiving at least one of power or data comprise at least one implantable antenna of the first implantable device and at least one implantable antenna of the second implantable device.

In some embodiments, the at least one external antenna comprises multiple external antennas. The multiple external antennas can comprise a first external antenna with a first construction and a second external antenna with a second construction, and the first construction can be similar to the second construction. The multiple external antennas can comprise a first external antenna with a first construction and a second external antenna with a second construction, and the first construction can be dissimilar to the second construction. The first external device at least one external antenna can comprise a first external antenna and a second external antenna. The external system can further comprise a second external device comprising at least one external antenna configured to transmit both power and data to the implantable system. The first implantable device at least one implantable antenna can be configured to receive power from the multiple external antennas. The first implantable device at least one implantable antenna can be configured to receive power from the multiple external antennas simultaneously. The first implantable device at least one implantable antenna can be configured to receive power from the multiple external antennas sequentially. The external system can be configured to change from transmitting power to the first implantable device by a first external antenna, to transmitting power to the first implantable device by a second external antenna, when the first external antenna moves. The external system can be configured to change from transmitting power to the first implantable device by a first external antenna, to transmitting power to the first implantable device by a second external antenna, when the second external antenna is activated. The external system can be configured to change from transmitting power to the first implantable device by a first external antenna to transmitting power to the first implantable device by a second external antenna, to achieve improved power transfer. The external system can be configured to change from transmitting power to the first implantable device by a first external antenna to transmitting power to the first implantable device by a second external antenna, when the power received from the first external antenna decreases. The external system can be configured to change from transmitting power to the first implantable device by a first external antenna to transmitting power to the first implantable device by a second external antenna, when the power received from the first external antenna decreases below a threshold. The first implantable device at least one implantable antenna can receive data from a first external antenna only. The implantable system can comprise multiple implantable antennas, and the multiple external antennas can define a radiation footprint, and one or more of the multiple implantable antennas that receive at least one of power or data from the multiple external antennas can be positioned within a projection of the radiation footprint. The projection cross sectional area can change with depth into the patient. The multiple implantable antennas receiving at least one of power or data can be surrounded by the implantable housing of the first implantable device. The implantable system can further comprise a second implantable device, and the one or more of the multiple implantable antennas receiving at least one of power or data can comprise at least one implantable antenna of the first implantable device and at least one implantable antenna of the second implantable device.

In some embodiments, the external system comprises multiple external antennas and the implantable system comprises multiple implantable antennas, and two or more of the multiple external antennas transmit at least one of power or data to two or more of the multiple implantable antennas. A single external antenna can be configured to transmit power to multiple implantable antennas. A single implantable antenna can be configured to receive power from multiple external antennas. The multiple external antennas can comprise an external antenna array. Each external antenna of the external antenna array can be configured to be selectively activated to improve coupling with the implantable system. The external antenna array can be configured to compensate for movement of the external antenna array relative to the implantable system.

In some embodiments, the external transmitter comprises a transmitter that operates in a frequency range between 0.1GHz and 3.0GHz. The external transmitter can comprise a transmitter that operates in a frequency range between 0.4GHz and 1.5GHz. The external transmitter can comprise a transmitter that operates in a frequency range between approximately 0.902GHz and 0.928GHz.

In some embodiments, the external transmitter comprises a transmitter configured to produce the transmission signal at a power level between 0.1W and 4.0W. The external transmitter can comprise a transmitter configured to produce the transmission signal at a power level between 0.1W and 2.0W. The external transmitter can comprise a transmitter configured to produce the transmission signal at a power level between 0.2W and 1.0W.

In some embodiments, the external transmitter comprises a transmitter configured to perform data modulation comprising amplitude shift keying with pulse-width modulation. The external transmitter can be configured to perform multi-level amplitude shift keying. The external transmitter can comprise a configurable data transmission parameter selected from the group consisting of: modulation depth; data rate; pulse width; duty cycle; pulse shape; pulse timing; packet length; error correction; and combinations thereof. The external transmitter can be configured to provide low-depth modulation between 5% and 75% depth. The external transmitter can be configured to provide low-depth modulation between 10% and 50% depth.

In some embodiments, the external system transmits data to the implantable system using time-domain multiple access communication.

In some embodiments, external transmitter comprises a transmitter configured to transmit one or more data signals with a bandwidth between 0.1MHz and 100MHz. The external transmitter can comprise a transmitter configured to transmit one or more communication signals with a bandwidth between 1MHz and 26MHz.

In some embodiments, the external transmitter comprises a transmitter configured to transmit power using duty cycling.

In some embodiments, the external transmitter is configured to adjust the level of transmitted power. The external transmitter can be configured to set the duty cycling based on a stimulation level produced by the implantable system. The external transmitter can be configured to adjust the level of transmitted power to prevent oversaturation. The implantable system can be configured to transmit data, and the external transmitter can be configured to adjust the level of transmitted power to reduce interference with the implantable system data transmissions. The implantable system can further comprise a second implantable receiver, and the external transmitter can be configured to transmit a first power transmission to the first implantable receiver and a second, different power transmission to the second implantable receiver. The external transmitter is configured to adjust the duty cycling based on at least one of charge information or discharge information received from the implantable system.

In some embodiments, the external power supply comprises a battery. The battery can comprise a rechargeable battery. The external power supply can further comprise a second battery configured to serially replace the first battery.

In some embodiments, the external power supply can be configured to provide a voltage of at least 3V.

In some embodiments, the external power supply comprises a capacity between 1Watt-hour and 75Watt-hours. The external power supply can comprise a capacity of approximately 5Watt-hours.

In some embodiments, the external power supply comprises an AC power source.

In some embodiments, the external controller comprises a user interface. The user interface can be configured to allow an operator to adjust a parameter selected from the group consisting of: stimulation parameter; sensing parameter; therapy parameter; data recording parameter; power transfer; data rate; activity of the external transmitter; activity of the at least one external antenna; a parameter of an external functional element; a parameter of the at least one implantable functional element; and combinations thereof. The user interface can be configured to provide apparatus status information.

In some embodiments, the external controller is configured to confirm at least one of adequate data transmission or adequate power transmission to the implantable system. The external controller can be configured to detect power transmission to the implantable system below a threshold. The external controller can be configured to detect power transmission to the implantable system trending in an undesired direction. The external controller can be configured to detect at least one of improper data transfer to the implantable system or inadequate data transfer to the implantable system.

In some embodiments, the external controller comprises a matching network configured to match the impedance of the first external device at least one external antenna to the external transmitter. The matching network can comprise an adjustable matching network. The external controller can further comprise a directional coupler, and the directional coupler can be configured to measure a reflection coefficient. The external transmitter can comprise an output, and the external controller can be configured to monitor a standing wave pattern at the output of the external transmitter.

In some embodiments, external controller comprises a temperature sensor. The temperature sensor can be configured to monitor the temperature of one or more of: an external antenna; an external housing; the external power supply; and the external controller. The external controller can be configured to adjust one or more of: matching network; stimulation level; and transmission power level, based on temperature data recorded by the temperature sensor.

In some embodiments, the external controller comprises a lookup table of stimulation signal waveform patterns.

In some embodiments, the external controller comprises a set of adjustable stimulation signal parameter configured to be varied to produce a customized waveform, the adjustable stimulation parameters selected from the group consisting of: frequency; amplitude; duty cycle; duration of pulse and/or amplitude level; duration of stimulation waveform; repetition of stimulation waveform; pulse shape; and combinations thereof.

In some embodiments, first external device comprises an adhesive element configured to adhesively attach the external transmitter to the patient.

In some embodiments, the first external device further comprises an external housing. The external housing can surround at least the external transmitter. The external housing can surround at least the external controller. The external housing can surround at least the external power supply. The external housing can surround at least the first external device at least one external antenna. The external housing can comprise a watch housing.

In some embodiments, the first external device further comprises an external functional element comprising a component selected from the group consisting of: a sensor; a transducer; an electrode; energy delivery element; agent delivery element; and combinations thereof.

In some embodiments, the external system further comprises an external functional element comprising one or more sensors. The first external device can further comprise an external housing, and the one or more sensors can be positioned on the external housing. The one or more sensors can comprise a body conduction sensor. The one or more sensors can be configured to produce a signal representative of stimulation energy delivered by the implantable system. The one or more sensors can be configured to record patient information. The one or more sensors can comprise one or more sensors selected from the group consisting of: electrode; sensor configured to record electrical activity of tissue; blood glucose sensor such as an optical blood glucose sensor; pressure sensor; blood pressure sensor; heart rate sensor; inflammation sensor; neural activity sensor; muscular activity sensor; pH sensor; strain gauge; accelerometer; gyroscope; GPS; respiration sensor; respiration rate sensor; temperature sensor; magnetic sensor; optical sensor; MEMs sensor; chemical sensor; hormone sensor; impedance sensor; tissue impedance sensor; body position sensor; body motion sensor; physical activity level sensor; perspiration sensor; patient hydration sensor; breath monitoring sensor; sleep monitoring sensor; food intake monitoring sensor; urine movement sensor; bowel movement sensor; tremor sensor; pain level sensor; and combinations thereof. The one or more sensors can comprise one or more sensors configured to record patient information selected from the group consisting of: blood glucose; blood pressure; EKG; heart rate; cardiac output; oxygen level; pH level; pH of blood; pH of a bodily fluids; tissue temperature; inflammation level; bacteria level; type of bacteria present; gas level; blood gas level; neural activity; neural spikes; neural spike shape; action potential; local field potential; EEG; muscular activity; gastric volume; peristalsis rate; impedance; tissue impedance; electrode-tissue interface impedance; physical activity level; pain level; body position; body motion; organ motion; respiration rate; respiration level; perspiration rate; sleep level; sleep cycle; digestion state; digestion level; urine production; urine flow; bowel movement; tremor; ion concentration; chemical concentration; hormone level; viscosity of a bodily fluid; patient hydration level; and combinations thereof. The one or more sensors can be configured to record external system information. The one or more sensors can comprise one or more sensors selected from the group consisting of: an energy sensor; a voltage sensor; a current sensor; a temperature sensor; an antenna matching and/or mismatching assessment sensor; power transfer sensor; link gain sensor; power use sensor; energy level sensor; energy charge rate sensor; energy discharge rate sensor; impedance sensor; load impedance sensor; instantaneous power usage sensor; average power usage sensor; bit error rate sensor; signal integrity sensor; and combinations thereof. The one or more sensors can comprise one or more sensors configured to record external system information selected from the group consisting of: power transfer; link gain; power use; energy within the external power supply; performance of the external power supply; expected life of the external power supply; discharge rate of the external power supply; ripple or other variations of the external power supply; matching of the at least one external antenna and the at least one implantable antenna; communication error rate between the first implantable device and the first external device; integrity of transmission between the first implantable device and the first external device; and combinations thereof.

In some embodiments, the external system further comprises an external functional element comprising a sensor configured to record a patient parameter selected from the group consisting of: blood parameter; white blood cell count; hormone level; blood pressure; neural activity; and combinations thereof. The one or more sensors can comprise at least one body conduction sensor, such as a skin conduction sensor.

In some embodiments, the external system further comprises an external functional element comprising an external agent delivery element.

In some embodiments, the external system further comprises an external functional element comprising an external transmitter. The external transmitter can comprise a Bluetooth transmitter.

In some embodiments, external system further comprises an external functional element comprising a temperature sensor. The temperature sensor can be configured to measure temperature of the patient. The temperature sensor can be configured to measure temperature of the external system.

In some embodiments, the external system further comprises an external functional element comprising a transducer selected from the group consisting of: transducer selected from the group consisting of: light; light emitting diode; wireless transmitter; Bluetooth device; mechanical transducer; piezoelectric transducer; pressure transducer; temperature transducer; humidity transducer; vibrational transducer; audio transducer; speaker; and combinations thereof.

In some embodiments, the external system further comprises an external functional element comprising at least one electrode. The at least one electrode can be configured to perform a function selected from the group consisting of: record electrical activity; deliver electrical energy; and combinations thereof.

In some embodiments, the external system further comprises an external receiver configured to receive data from the implantable system. The external system external receiver can be configured to receive the data from the first implantable device at least one implantable antenna. The data received by the external system external receiver can comprise at least one of patient information or information of the implantable system. The at least one of patient information or information of the implantable system can comprise patient information selected from the group consisting of: blood glucose; blood pressure; EKG; heart rate; cardiac output; oxygen level; pH level; pH of blood; pH of a bodily fluids; tissue temperature; inflammation level; bacteria level; type of bacteria present; gas level; blood gas level; neural activity; neural spikes; neural spike shape; action potential; local field potential (LFP); EEG; muscular activity; gastric volume; peristalsis rate; impedance; tissue impedance; electrode-tissue interface impedance; physical activity level; pain level; body position; body motion; organ motion; respiration rate; respiration level; perspiration rate; sleep level; sleep cycle; digestion state; digestion level; urine production; urine flow; bowel movement; tremor; ion concentration; chemical concentration; hormone level; viscosity of a bodily fluid; patient hydration level; and combinations thereof. The at least one of patient information or information of the implantable system can comprise implantable system information selected from the group consisting of: power transfer; link gain; power use; energy within the implantable energy storage assembly; performance of the implantable energy storage assembly; expected life of the implantable energy storage assembly; discharge rate of the implantable energy storage assembly; ripple or other variations of the implantable energy storage assembly; matching of the at least one implantable antenna and the at least one external antenna; communication error rate between the first implantable device and the first external device; integrity of transmission between the first implantable device and the first external device; and combinations thereof.

In some embodiments, the external system further comprises a second external transmitter configured to transmit data. The second external transmitter can be configured to transmit data to one or more of: cell phone; computer; tablet; computer network; the Internet; LAN; data storage device; data analysis device; and combinations thereof. The second external transmitter can comprise a wireless transmitter. The second external transmitter can comprise a Bluetooth transmitter. The second external transmitter comprises a cellular transmitter.

In some embodiments, the medical apparatus further comprises a diagnostic assembly. The external system can comprise at least a portion of the diagnostic assembly. The implantable system can comprise at least a portion of the diagnostic assembly. The diagnostic assembly can be configured to analyze patient information. The patient information can comprise information selected from the group consisting of: blood glucose; blood pressure; EKG; heart rate; cardiac output; oxygen level; pH level; pH of blood; pH of a bodily fluids; tissue temperature; inflammation level; bacteria level; type of bacteria present; gas level; blood gas level; neural activity; neural spikes; neural spike shape; action potential; local field potential (LFP); EEG; muscular activity; gastric volume; peristalsis rate; impedance; tissue impedance; electrode-tissue interface impedance; physical activity level; pain level; body position; body motion; organ motion; respiration rate; respiration level; perspiration rate; sleep level; sleep cycle; digestion state; digestion level; urine production; urine flow; bowel movement; tremor; ion concentration; chemical concentration; hormone level; viscosity of a bodily fluid; patient hydration level; and combinations thereof. The diagnostic assembly can be configured to analyze implantable system information. The implantable system information can comprise information selected from the group consisting of: power transfer; link gain; power use; energy within the implantable energy storage assembly; performance of the implantable energy storage assembly; expected life of the implantable energy storage assembly; discharge rate of the implantable energy storage assembly; ripple or other variations of the implantable energy storage assembly; matching of the at least one implantable antenna and the at least one external antenna; communication error rate between the first implantable device and the first external device; integrity of transmission between the first implantable device and the first external device; and combinations thereof. The diagnostic assembly can be configured to analyze communication between the first implantable device and the first external device. The diagnostic assembly can be configured to analyze one or more of: neural activity; muscular activity; and combinations thereof. The diagnostic assembly can be configured to analyze impedance. The first implantable device can be configured to perform a function to create impedance data, and the function can be selected from the group consisting of: perform a frequency sweep; perform an impulse response; compare voltage and current of a stimulation waveform; and combinations thereof. The diagnostic assembly can be configured to perform a communication link analysis. The communication link analysis can comprise measuring a bit error rate. The communication link analysis can comprise trending communication link performance. The diagnostic assembly can comprise a user alert assembly. The user alert assembly can comprise a user alert element selected from the group consisting of: light; audio transducer; and combinations thereof.

In some embodiments, the external system further comprises an attachment assembly constructed and arranged to attach the first external device at least one external antenna proximate the patient's skin. The attachment assembly can comprise a component selected from the group consisting of: belt; belt with pockets; adhesive; strap; strap with pockets; shoulder strap; shoulder band; shirt; shirt with pockets; clothing; clothing with pockets; epidural electronics packaging; clip; bracelet; wrist band; wrist watch; necklace; and combinations thereof. The attachment assembly can comprise a belt configured to surround the first external device at least one external antenna. The first external device at least one external antenna can comprise an external antenna positioned on a flexible printed circuit board. The attachment assembly can comprise a belt with at least one pocket. The at least one pocket can be sized to surround one or more of: first external device at least one external antenna; the external controller; and the external power supply. The at least one pocket can comprise multiple pockets constructed and arranged to allow repositioning of one or more of: the first external device at least one external antenna; the external controller; the external transmitter; and the external power supply.

In some embodiments, the medical apparatus further comprises a reservoir constructed and arranged to store at least one agent. The reservoir can comprise a multichamber reservoir. The reservoir can be positioned in the first implantable device. The reservoir can be positioned in the first external device. The reservoir can comprise a refillable reservoir. The reservoir can comprise a fluid pumping mechanism, such as a syringe pump, a peristaltic pump or other pumping mechanism. The reservoir can comprise a volume of between 0.1ml and 50ml. The reservoir can comprise a volume between 0.1ml and 3.0ml. The reservoir can comprise a volume between 0.1ml and 1.5ml.

In some embodiments, the medical apparatus further comprises an agent. The apparatus can be configured to deliver the agent to the patient. The medical apparatus can further comprise a reservoir, and the reservoir can be constructed and arranged to store the agent. The agent can comprise an agent selected from the group consisting of: an analgesic agent; morphine; fentanyl; lidocaine; pain treating agent; a chemotherapeutic agent; a chemotherapeutic agent delivered systemically; a chemotherapeutic agent delivered proximate an organ such as the liver or brain; an antibiotic; a hormone; a heart medications; nitroglycerin; a beta blocker; a blood pressure lowering medication; a carbohydrate; glucose; dextrose; insulin; a diabetic medication; a neurological medication; an epilepsy medication; and combinations thereof. The apparatus can be constructed and arranged to deliver the agent to a patient location selected from the group consisting of: a vessel; a blood vessel; a vein; an artery; heart; brain; liver; spine; epidural space; intrathecal space; subcutaneous tissue; bone; and combinations thereof. The medical apparatus can further comprise at least one sensor configured to record a patient parameter, and the medical apparatus can be configured to deliver the agent based on the recorded patient parameter. The first implantable device at least one implantable functional element can comprise the at least one sensor. Alternatively or additionally, the first external device can comprise the at least one sensor.

According to another aspect of the present inventive concepts, a stimulation apparatus for stimulating tissue of a patient is provided, comprising: an external system configured to transmit power; and an implantable system configured to receive the power from the external system, and to deliver stimulation energy to tissue; and the stimulation energy delivered can be independent of the received power.

In some embodiments, the stimulation energy delivered is independent of one or more of: the position of one or more components of the external system; the changing position of one or more components of the external system; the frequency of the power received from the external system; the amplitude of the power received from the external system; changes in amplitude of the power received from the external system; duty cycle of the power received from the external system; envelope of the power received from the external system; and combinations thereof.

In some embodiments, the frequency of the received power is independent of the frequency of the stimulation energy delivered. The apparatus can be configured such that changes in the frequency of the received power do not affect the frequency of the delivered energy.

In some embodiments, the implantable system is configured to perform frequency multiplication to create the stimulation energy.

In some embodiments, the implantable system is configured to rectify the received power and produce stimulation energy at a different frequency than the frequency of the received power. The stimulation frequency can be created via an oscillator.

### BRIEF DESCRIPTION OF THE DRAWINGS

The foregoing and other objects, features and advantages of embodiments of the present inventive concepts will be apparent from the more particular description of preferred embodiments, as illustrated in the accompanying drawings in which like reference characters refer to the same or like elements. The drawings are not necessarily to scale, emphasis instead being placed upon illustrating the principles of the preferred embodiments.
**Fig. 1** is a schematic anatomical view of a medical apparatus comprising an external system and an implantable system, consistent with the present inventive concepts.
**Fig. 2** is a schematic anatomical view of a medical apparatus comprising multiple external devices and multiple implantable devices, consistent with the present inventive concepts.
**Fig. 3** is a perspective view of an implantable device comprising an implantable lead and tethered antenna, consistent with the present inventive concepts.
**Fig. 4** is a perspective view of an implantable device comprising an implantable lead and an antenna positioned on a substrate, consistent with the present inventive concepts.
**Fig. 5** is a perspective view of an external device comprising a tethered antenna, consistent with the present inventive concepts.
**Fig. 6A** is a top view of a substrate comprising components and multiple antennas, shown in an unfolded state, consistent with the present inventive concepts.
**Fig. 6B** is a perspective view of the substrate of Fig. 6A, shown in a partially folded state, consistent with the present inventive concepts.
**Fig. 6C** is an end sectional view of an implantable device comprising an implantable housing surrounding the folded substrate of Figs. 6A-B, consistent with the present inventive concepts.
**Fig. 7A** is an end view of a substrate including various components and in an unfolded condition, consistent with the present inventive concepts.
**Fig. 7B** is an end sectional view of an implantable device comprising an implantable housing surrounding the substrate of Fig. 7A after the substrate has been folded to fit within the implantable housing, consistent with the present inventive concepts.
**Fig. 8** is an anatomical view of a medical apparatus comprising an external antenna positioned relative to multiple implantable devices that have been implanted in a patient, consistent with the present inventive concepts.
**Fig. 9** is an anatomical view of a medical apparatus comprising an external antenna comprising multiple concentric loops and positioned relative to multiple implantable devices that have been implanted in a patient, consistent with the present inventive concepts.
**Fig. 10** is an anatomical view of a medical apparatus comprising an external antenna comprising an array of antennas and positioned relative to multiple implantable devices that have been implanted in a patient, consistent with the present inventive concepts.
**Fig. 11** is a side view of an implantable device comprising an implantable lead with a stylet, consistent with the present inventive concepts.
**Fig. 12A** is an exploded view of an implantable device comprising an implantable housing surrounding multiple antennas and various electrical components, consistent with the present inventive concepts.
**Fig. 12B** is a perspective view of the implantable device of Fig. 12A shown in an assembled form, consistent with the present inventive concepts.
**Fig. 12C** is a perspective view of the implantable device of Fig. 12B, further including an implantable lead with implantable functional elements, consistent with the present inventive concepts.
**Fig. 13** is a perspective view of an apparatus comprising an implantable device and a limb-attached external device positioned proximate the implantable device, consistent with the present inventive concepts.
**Fig. 13A** is an end sectional view of the apparatus of Fig. 13, also shown positioned about a limb of the patient, consistent with the present inventive concepts.
**Fig. 13B** is a side sectional view of the implantable device of Figs. 13 and 13A, consistent with the present inventive concepts.

### DETAILED DESCRIPTION OF THE DRAWINGS

The terminology used herein is for the purpose of describing particular embodiments and is not intended to be limiting of the inventive concepts. Furthermore, embodiments of the present inventive concepts may include several novel features, no single one of which is solely responsible for its desirable attributes or which is essential to practicing an inventive concept described herein. As used herein, the singular forms "a," "an" and "the" are intended to include the plural forms as well, unless the context clearly indicates otherwise.

It will be further understood that the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") when used herein, specify the presence of stated features, integers, steps, operations, elements, and/or components, but do not preclude the presence or addition of one or more other features, integers, steps, operations, elements, components, and/or groups thereof.

It will be understood that, although the terms first, second, third etc. may be used herein to describe various limitations, elements, components, regions, layers and/or sections, these limitations, elements, components, regions, layers and/or sections should not be limited by these terms. These terms are only used to distinguish one limitation, element, component, region, layer or section from another limitation, element, component, region, layer or section. Thus, a first limitation, element, component, region, layer or section discussed below could be termed a second limitation, element, component, region, layer or section without departing from the teachings of the present application.

It will be further understood that when an element is referred to as being "on", "attached", "connected" or "coupled" to another element, it can be directly on or above, or connected or coupled to, the other element, or one or more intervening elements can be present. In contrast, when an element is referred to as being "directly on", "directly attached", "directly connected" or "directly coupled" to another element, there are no intervening elements present. Other words used to describe the relationship between elements should be interpreted in a like fashion (e.g., "between" versus "directly between," "adjacent" versus "directly adjacent," etc.).

It will be further understood that when a first element is referred to as being "in", "on" and/or "within" a second element, the first element can be positioned: within an internal space of the second element, within a portion of the second element (e.g. within a wall of the second element); positioned on an external and/or internal surface of the second element; and combinations of one or more of these.

Spatially relative terms, such as "beneath," "below," "lower," "above," "upper" and the like may be used to describe an element and/or feature's relationship to another element(s) and/or feature(s) as, for example, illustrated in the figures. It will be understood that the spatially relative terms are intended to encompass different orientations of the device in use and/or operation in addition to the orientation depicted in the figures. For example, if the device in a figure is turned over, elements described as "below" and/or "beneath" other elements or features would then be oriented "above" the other elements or features. The device can be otherwise oriented (e.g., rotated 90 degrees or at other orientations) and the spatially relative descriptors used herein interpreted accordingly.

The term "and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

As described herein, "room pressure" shall mean pressure of the environment surrounding the systems and devices of the present inventive concepts. Positive pressure includes pressure above room pressure or simply a pressure that is greater than another pressure, such as a positive differential pressure across a fluid pathway component such as a valve. Negative pressure includes pressure below room pressure or a pressure that is less than another pressure, such as a negative differential pressure across a fluid component pathway such as a valve. Negative pressure can include a vacuum but does not imply a pressure below a vacuum. As used herein, the term "vacuum" can be used to refer to a full or partial vacuum, or any negative pressure as described hereabove.

The term "diameter" where used herein to describe a non-circular geometry is to be taken as the diameter of a hypothetical circle approximating the geometry being described. For example, when describing a cross section, such as the cross section of a component, the term "diameter" shall be taken to represent the diameter of a hypothetical circle with the same cross sectional area as the cross section of the component being described.

The terms "major axis" and "minor axis" of a component where used herein are the length and diameter, respectively, of the smallest volume hypothetical cylinder which can completely surround the component.

The term "transducer" where used herein is to be taken to include any component or combination of components that receives energy or any input, and produces an output. For example, a transducer can include an electrode that receives electrical energy, and distributes the electrical energy to tissue (e.g. based on the size of the electrode). In some configurations, a transducer converts an electrical signal into any output, such light (e.g. a transducer comprising a light emitting diode or light bulb), sound (e.g. a transducer comprising a piezo crystal configured to deliver ultrasound energy), pressure, heat energy, cryogenic energy, chemical energy; mechanical energy (e.g. a transducer comprising a motor or a solenoid), magnetic energy, and/or a different electrical signal (e.g. a Bluetooth or other wireless communication element). Alternatively or additionally, a transducer can convert a physical quantity (e.g. variations in a physical quantity) into an electrical signal. A transducer can include any component that delivers energy and/or an agent to tissue, such as a transducer configured to deliver one or more of: electrical energy to tissue (e.g. a transducer comprising one or more electrodes); light energy to tissue (e.g. a transducer comprising a laser, light emitting diode and/or optical component such as a lens or prism); mechanical energy to tissue (e.g. a transducer comprising a tissue manipulating element); sound energy to tissue (e.g. a transducer comprising a piezo crystal); chemical energy; electromagnetic energy; magnetic energy; and combinations of one or more of these.

The term "transmission signal" where used herein is to be taken to include any signal transmitted between two components, such as via a wired or wireless communication pathway. For example, a transmission signal can comprise a power and/or data signal wirelessly transmitted between a component external to the patient and one or more components implanted in the patient. A transmission signal can include a signal transmitted using body conduction. Alternatively or additionally, a transmission signal can comprise reflected energy, such as energy reflected from any power and/or data signal.

The term "data signal" where used herein is to be taken to include a transmission signal including at least data. For example, a data signal can comprise a transmission signal including data and sent from a component external to the patient and one or more components implanted in the patient. Alternatively, a data signal can comprise a transmission signal including data sent from an implanted component to one or more components external to the patient. A data signal can comprise a radiofrequency signal including data (e.g. a radiofrequency signal including both power and data) and/or a data signal sent using body conduction.

The term "implantable" where used herein is to be taken to define a component which is constructed and arranged to be fully or partially implanted in a patient's body and/or a component that has been fully or partially implanted in a patient. The term "external" where used herein is to be taken to define a component which is constructed and arranged to be positioned outside of the patient's body.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination. For example, it will be appreciated that all features set out in any of the claims (whether independent or dependent) can be combined in any given way.

The present inventive concepts include a medical apparatus for treating a patient, and/or recording patient information. The patient can comprise a human or other mammalian patient. The medical apparatus can comprise a stimulation apparatus. The medical apparatus comprises an implantable system and an external system. The implantable system can comprise one or more similar and/or dissimilar implantable devices. Each implantable device can comprise one or more implantable antennas configured to receive power and/or data. Each implantable device can comprise an implantable receiver configured to receive the power and/or data from the one or more implantable antennas. Each implantable device can comprise one or more implantable functional elements. An implantable functional element can be configured to interface with the patient (e.g. interface with tissue or the patient or any patient location). Alternatively or additionally, an implantable functional element can interface with a portion of an implantable device. In some embodiments, the one or more implantable functional elements can comprise one or more transducers, electrodes, and/or other elements configured to deliver energy to tissue. Alternatively or additionally, the one or more implantable functional elements can comprise one or more sensors, such as a sensor configured to record a physiologic parameter of the patient. In some embodiments, one or more implantable functional elements are configured to record device information and/or patient information (e.g. patient physiologic or patient environment information).

Each implantable device can comprise an implantable controller configured to control (e.g. modulate power to, send a signal to and/or receive a signal from) the one or more implantable functional elements. Each implantable device can comprise an implantable energy storage assembly configured to provide power to the implantable controller, the implantable receiver and/or the one or more implantable functional elements. In some embodiments, an implantable energy storage assembly is further configured to provide power to an implantable antenna (e.g. when the implantable device is further configured to transmit data to one or more external devices). Each implantable device can comprise an implantable housing surrounding at least the implantable controller and the implantable receiver. In some embodiments, one or more implantable antennas are positioned within the implantable housing. Alternatively or additionally, one or more implantable antennas and/or implantable functional elements can be tethered (e.g. electrically tethered) to the implantable housing. In some embodiments, one or more implantable functional elements are positioned on an implantable lead, such as a flexible lead mechanically fixed or attachable to the implantable housing and operably connected (e.g. electrically, fluidly, optically and/or mechanically) to one or more components internal to the implantable housing. The implantable lead can be inserted (e.g. tunneled) through tissue of the patient, such that its one or more functional elements are positioned proximate tissue to be treated and/or positioned at an area in which data is to be recorded.

The external system of the medical apparatus of the present inventive concepts can comprise one or more similar and/or dissimilar external devices. Each external device can comprise one or more external antennas configured to transmit power and/or data to one or more implanted components of the implantable system. Each external device can comprise an external transmitter configured to drive the one or more external antennas. Each external device can comprise an external power supply configured to provide power to at least the external transmitter. Each external device can comprise an external controller configured to control the external transmitter. Each external device can comprise an external housing that surrounds at least the external transmitter. In some embodiments, the external housing surrounds the one or more external antennas, the external power supply and/or the external controller.

The external controller can comprise a discrete controller separate from the one or more external devices, and/or a controller integrated into one or more external devices. The external controller can comprise a user interface, such as a user interface configured to set and/or modify one or more treatment and/or data recording settings of the medical apparatus of the present inventive concepts. In some embodiments, the external controller can be configured to collect and/or diagnose recorded patient information, such as to provide the information and/or diagnosis to a clinician of the patient, to a patient family member and/or to the patient themselves. The collected information and/or diagnosis can be used to adjust treatment or other operating parameters of the medical apparatus.

In some embodiments, a medical apparatus comprises a stimulation apparatus for stimulating tissue of a patient. The stimulation apparatus comprises an external system configured to transmit power, and an implanted system configured to receive the power from the external system and to deliver stimulation energy to tissue. The stimulation signal (also referred to as "stimulation energy") delivered by the implanted system can be independent of the power received from the external system, such as to be independent of one or more of: the position of one or more components of the external system; the changing position of one or more components of the external system; the frequency of the power received from the external system; the amplitude of the power received from the external system; changes in amplitude of the power received from the external system; duty cycle of the power received from the external system; envelope of the power received from the external system; and combinations of one or more of these.

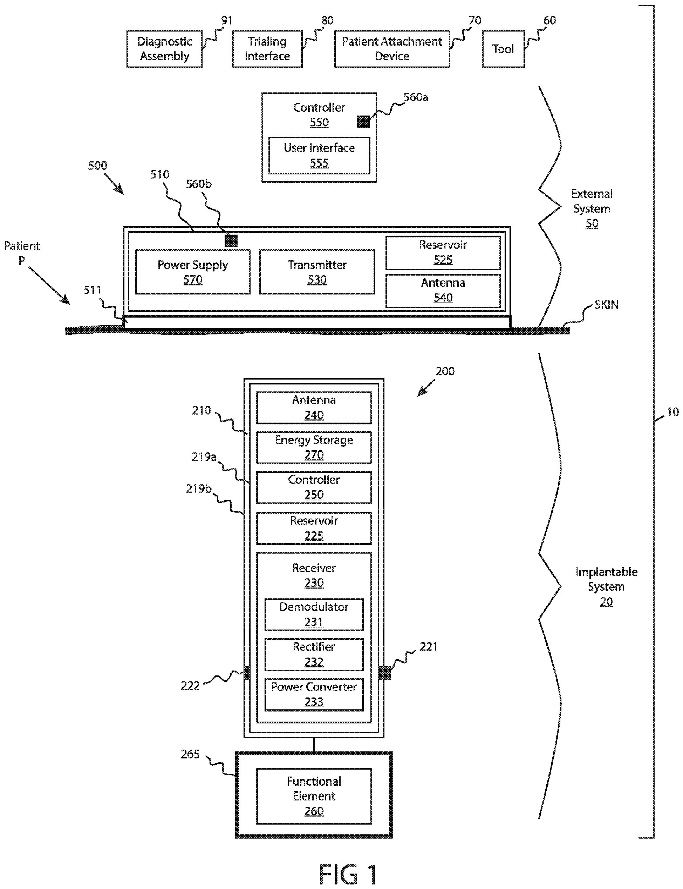

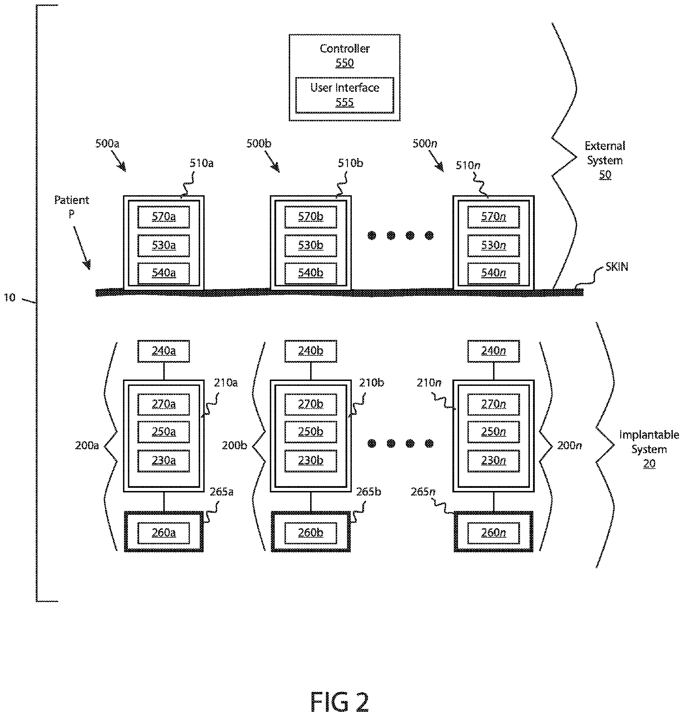

**Referring now to** **Fig. 1****,** a schematic anatomical view of a medical apparatus for treating and/or diagnosing a patient is illustrated, consistent with the present inventive concepts. Apparatus 10 comprises implantable system 20 and external system 50. Implantable system 20 comprises implantable device 200 shown implanted beneath the skin of patient P. In some embodiments, implantable system 20 comprises multiple implantable devices 200 (singly or collectively implantable device 200), such as is described herebelow in reference to Fig. 2. External system 50 comprises external device 500 which includes housing 510. In some embodiments, external system 50 comprises multiple external devices 500 (singly or collectively external device 500), also as is described herebelow in reference to Fig. 2. External system 50 can further comprise controller 550, which can comprise a user interface, such as user interface 555. Controller 550 is configured to control one or more external devices 500.

Apparatus 10 can comprise a patient treatment apparatus, such as a stimulation apparatus configured to stimulate tissue, such as by having one or more implantable devices 200 deliver energy and/or an agent (e.g. a pharmaceutical compound or other agent) to one or more tissue locations, while receiving power and/or data from one or more external devices 500. Alternatively or additionally, apparatus 10 can comprise a patient diagnostic apparatus, such as by having one or more implantable devices 200 record a patient parameter (e.g. a patient physiologic parameter) from one or more tissue locations, while receiving power and/or data from one or more external devices 500. In some embodiments, during its use, one or more implantable devices 200 at least receives power from one or more external devices 500. Alternatively or additionally, apparatus 10 can comprise a patient information recording apparatus, such as by having one or more implantable devices 200 and/or one or more external devices 500 record patient information (e.g. patient physiologic information and/or patient environment information). In some embodiments, one or more implantable devices 200 and/or one or more external devices 500 further collect information (e.g. status information or configuration settings) of one or more of the components of apparatus 10.

In some embodiments, apparatus 10 is configured as a stimulation apparatus in which external system 50 transmits a power signal to implantable system 20, and implantable system 20 delivers stimulation energy to tissue with a stimulation signal, with the power signal and the stimulation signal having one or more different characteristics. The power signal can further be modulated with data (e.g. configuration or other data to be sent to one or more implantable devices 200). In these embodiments, the characteristics of the stimulation signal delivered (e.g. amplitude, frequency, duty cycle and/or pulse width), can be independent (e.g. partially or completely independent) of the characteristics of the power signal (e.g. amplitude, frequency, phase, envelope, duty cycle and/or modulation). For example, the frequency and modulation of the power signal can change without affecting the stimulation signal, or the stimulation signal can be changed (e.g. via controller 550), without requiring the power signal to change. In some embodiments, implantable system 20 can be configured to rectify the power signal, and produce a stimulation waveform with entirely difference characteristics (e.g. amplitude, frequency and/or duty cycle) from the rectified power signal. Implantable system 20 can comprise an oscillator and/or controller configured to produce the stimulation signal. In some embodiments, implantable system 20 can be configured to perform frequency multiplication, in which multiple signals are multiplexed, mixed, added, and/or combined in other ways to produce a broadband stimulation signal.

In some embodiments, apparatus 10 is configured such that external system 50 transmits data (e.g. data and power) to implantable system 20, and implantable system 20 recovers (e.g. decodes, demodulates or otherwise recovers) the transmitted data without synchronizing to the carrier and/or data symbol rate of the transmitted signal from external system 50. In some embodiments, the transmitted signal comprises a power signal, and a clock and/or data is recovered without synchronizing to the power signal. In some embodiments, the transmitted signal comprises a clock and/or data signal, and a clock and/or data is recovered without synchronizing to the transmitted clock and/or data signal. In some embodiments, the recovered signal comprises a clock and/or data and a clock and/or data is recovered from the transmission signal without synchronizing to the recovered clock and/or data. Avoiding synchronization reduces power consumption of each implantable device 200, such as by obviating the need for (and avoiding the power consumed by) a frequency locked loop (FLL), phase locked loop (PLL); high frequency clock; and/or crystal oscillator needed to perform the synchronization. Avoiding these components can also be correlated to reduced package size of each implantable device 200 (e.g. avoidance of a relatively large sized crystal oscillator). Asynchronous data transfer between external system 50 and implantable system 20 is also advantageous as it relates to: increased communication data rate; power transfer efficiency; operation with more than one implantable device 200; and combinations of one or more of these. In some embodiments, one or more components of apparatus 10 are of similar construction and arrangement as similar components described in United States Patent Application Number 13/591,188 (US 2013/0053767), titled "Method of Making and Using an Apparatus for a Locomotive Micro-Implant using Active Electromagnetic Propulsion", filed August 21, 2012. In some embodiments, external system 50 and implantable system 20 provide asynchronous data transfer or are otherwise configured as described in United States Patent Application Number 13/734,772 (US 2013/0215979), titled "Method and Apparatus for Efficient Communication with Implantable Devices", filed January 4, 2013.

Apparatus 10 can be configured to treat a patient disease or disorder and/or it can be configured to record patient information. Apparatus 10 can be configured to treat pain, such as back pain. In some embodiments, apparatus 10 is configured to treat a type of pain selected from the group consisting of: back pain; joint pain; neuropathic pain; tennis elbow; muscle pain; shoulder pain; chronic, intractable pain of the back and/or lower limbs including unilateral or bilateral pain; neuropathic groin pain; perineal pain; phantom limb pain; complex regional pain syndrome; failed back surgery syndrome; cluster headaches; migraines; inflammatory pain; arthritis; abdominal pain; pelvic pain; and combinations of one or more of these. In some embodiments, apparatus 10 is configured to treat a patient disease or disorder selected from the group consisting of: chronic pain; acute pain; migraine; cluster headaches; urge incontinence; fecal incontinence; bowel disorders; tremor; obsessive compulsive disorder; depression; epilepsy; inflammation; tinnitus; high blood pressure; heart failure; carpal tunnel syndrome; sleep apnea; obstructive sleep apnea; dystonia; interstitial cystitis; gastroparesis; obesity; mobility issues; arrhythmia; rheumatoid arthritis; dementia; Alzheimer's disease; eating disorder; addiction; traumatic brain injury; chronic angina; congestive heart failure; muscle atrophy; inadequate bone growth; post-laminectomy pain; liver disease; Crohn's disease; irritable bowel syndrome; erectile dysfunction; kidney disease; and combinations of one or more of these.

Apparatus 10 can be configured to treat heart disease, such as heart failure of a patient. In these embodiments, stimulation of the spinal cord can be performed. In canine and porcine animals with failing hearts, spinal cord stimulation has been shown to reverse left ventricular dilation and improve cardiac function, while suppressing the prevalence of cardiac arrhythmias. In canines, coronary artery occlusion has been associated with increased intracardiac nerve firing, and stimulation at spinal segment T1 has been shown to suppress that nerve firing. Stimulation via apparatus 10 at one or more spinal cord locations can be used to suppress undesired cardiac nerve firing in humans and other mammalian patients. In some embodiments, stimulation via apparatus 10 at multiple spinal cord locations is used to enhance a cardiac treatment. For example, one or more functional elements 260 of one or more implantable devices 200 can be implanted at one or more spinal cord locations. Power and/or data can be transmitted to the one or more implantable devices 200 via one or more external devices 500 of external system 50. One or more stimulation signals can be delivered to spinal cord tissue, such as to treat heart failure or other cardiac disease or disorder. In some embodiments, one or more functional elements 260 are configured to deliver energy (e.g. electrical energy) to tissue to treat heart failure, such as tissue selected from the group consisting of: spinal canal; nerves in the spinal canal; nerves in the epidural space; peripheral nerves; posterior spinal nerve root; dorsal root; dorsal root ganglion; pre-ganglionic tissue on posterior spinal nerve root; post-ganglionic tissue on posterior nerve root; dorsal ramus; grey ramus communicans; white ramus communicans; ventral ramus; and combinations of one or more of these. In some embodiments, one or more functional elements of apparatus 10 (e.g. one or more functional elements 260 of implantable system 20) are used to record a patient parameter, such as a patient heart or spine parameter, and the information recorded is used to adjust the delivered stimulation signals. The at least one heart parameter can comprise a parameter selected from the group consisting of: EKG; blood oxygen; blood pressure; heart rate; ejection fraction; wedge pressure; cardiac output; and combinations thereof.

Apparatus 10 can be configured to pace and/or defibrillate the heart of a patient. One or more functional elements 260 can be positioned proximate cardiac tissue and deliver a stimulation signal as described herein (e.g. based on power and/or data received by implantable system 20 from external system 50). The stimulation signal can be used to pace, defibrillate and/or otherwise stimulate the heart. Alternatively or additionally, apparatus 10 can be configured to record cardiac activity (e.g. by recording EKG, blood oxygen, blood pressure, heart rate, ejection fraction, wedge pressure, cardiac output and/or other properties or functions of the cardiovascular system), such as to determine an onset of cardiac activity dysfunction or other undesired cardiac state. In some embodiments, apparatus 10 is configured to both record cardiac information and deliver a stimulation signal to cardiac tissue. For example, apparatus 10 can be configured such that external system 50 transmits power and/or data to implantable system 20. Implantable system 20 monitors cardiac activity, and upon detection of an undesired cardiovascular state, implantable system 20 delivers a pacing and/or defibrillation signal to the tissue that is adjacent to one or more functional elements 260 configured to deliver a cardiac stimulation signal.

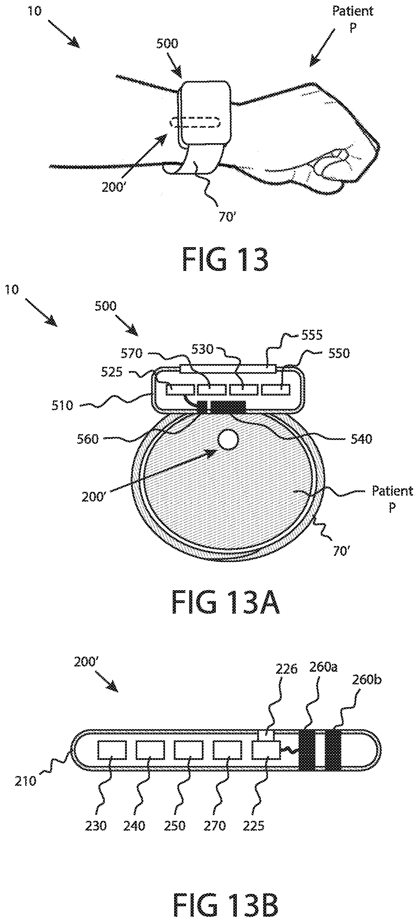

Apparatus 10 can be configured to perform a diagnostic procedure including measuring one or more patient parameters (e.g. patient physiologic or other patient parameters), such as are described in detail herebelow. In some embodiments, apparatus 10 is configured to measure a physiologic parameter that can be sensed from one or more sensor-based functional elements 260 positioned in subcutaneous tissue. In these embodiments, external system 50 can comprise an external device 500 configured for placement proximate an implantable device 200 implanted in a position to record data from subcutaneous tissue (e.g. blood glucose data). The external device 500 can comprise a wrist band or wrist watch configuration such as when the implantable device 200 is positioned in subcutaneous tissue proximate the patient's wrist, such as is described herebelow in reference to Fig. 13. Power and/or data can be sent to the implantable device 200 from the external device 500, and data (e.g. blood glucose data) can be sent to external device 500 (or another component of external system 50) by implantable device 200, such as using a communication configuration described in detail herebelow. In some embodiments, external device 500 comprises a functional element 560 configured to deliver an agent (e.g. insulin or glucose delivered by a needle-based functional element 560), based on the information received from implantable device 200. Alternatively or additionally, implantable device 200 comprises a functional element 260 configured to deliver an agent (e.g. insulin or glucose delivered by a needle-based functional element 260), based on the information recorded by implantable device 200. Various closed loop sensing and agent delivery combinations and configurations should be considered within the scope of the present inventive concepts, including but not limited to: sensing a blood parameter such as white blood cell count and delivering a chemotherapeutic or other agent based on the blood parameter; sensing a hormone level and delivering a hormone or a hormone affecting agent; sensing blood pressure and delivering stimulation energy and/or a blood pressure affecting agent; sensing neural activity and delivering stimulation energy and/or a neural affecting agent or other agent based on the neural activity, such as for treating epilepsy; and combinations of one or more of these.

External system 50 can be configured to transmit power and/or data (e.g. implantable system 20 configuration data) to one or more implantable devices 200 of implantable system 20. Configuration data provided by external system 50 (e.g. via one or more antennas 540 of one or more external devices 500) can include a stimulation parameter such as an energy delivery stimulation parameter selected from the group consisting of: initiation of energy delivery; cessation of energy delivery; amount of energy to be delivered; rate of energy delivery; amplitude of energy delivery; power of energy delivery; frequency of energy delivery; waveform shape of energy delivery; duration of energy delivery; time of energy delivery initiation; and combinations of one or more of these. The configuration data can include a stimulation parameter such as an agent (e.g. a pharmaceutical agent) delivery stimulation parameter selected from the group consisting of: initiation of agent delivery; cessation of agent delivery; amount of agent to be delivered; volume of agent to be delivered; rate of agent delivery; duration of agent delivery; time of agent delivery initiation; and combinations of one or more of these. The configuration data can include a sensing parameter, such as a sensing parameter selected from the group consisting of: initiation of sensor recording; cessation of sensor recording; frequency of sensor recording; resolution of sensor recording; thresholds of sensor recording; sampling frequency of sensor recording; dynamic range of sensor recording; initiation of calibration of sensor recording; and combinations of one or more of these.

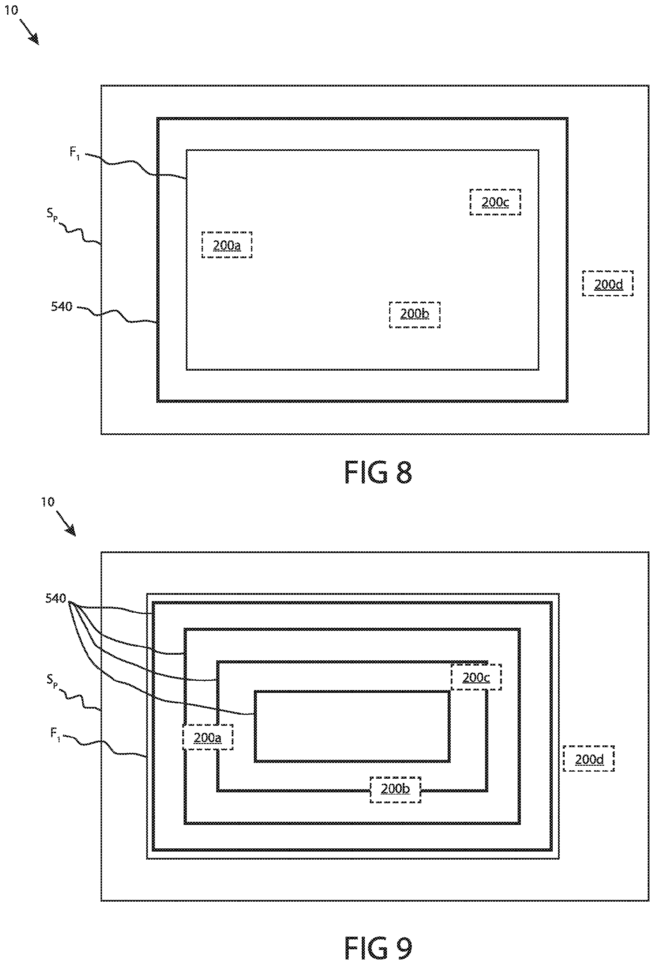

External system 50 can comprise one or more external devices 500. External system 50 can comprise one or more antennas 540, such as when a single external device 500 comprises one or more antennas 540 or when multiple external devices 500 each comprise one or more antennas 540. The one or more antennas 540 can transmit power and/or data to one or more antennas 240 of implantable system 20, such as when a single implantable device 200 comprises one or more antennas 240 or when multiple implantable devices 200 each comprise one or more antennas 240. In some embodiments, one or more antennas 540 define a radiation footprint (e.g. a footprint defining a volume, such as a volume of tissue, in which electromagnetic transmissions radiated by antennas 540 can be properly received by antennas 240), as defined herebelow in reference to Figs. 8, 9 or 10. The implanted position of the one or more antennas 240 relative to the positioning of the one or more antennas 540 can be influenced by the radiation footprint, also as described herebelow.

External system 50 transmits power and/or data with a transmission signal comprising at least one wavelength, λ. External system 50 and/or implantable system 20 can be configured such that the distance between an external antenna 540 transmitting the power and/or data and one or more implantable antennas 240 receiving the power and/or data transmission signal is equal to between 0.1λ and 10.0λ, such as between 0.2λ and 2.0λ. In some embodiments, one or more transmission signals are delivered at a frequency range between 0.1GHz and 10.6GHz, such as between 0.1GHz and 3.0GHz, between 0.4GHz and 1.5GHz, or between 0.902GHz and 0.928GHz, or in a frequency range proximate to 866MHz, or approximately between 863MHz and 870MHz.

In addition to transmitting power and/or data to implantable system 20, external system 50 can be further configured to provide information (e.g. apparatus 10 performance information and/or patient information) to one or more other devices, such as tool 60 shown in Fig. 1 and described in detail herebelow.

One or more external devices 500 (singly or collectively external device 500) can be configured to transmit power and/or data (e.g. implantable system 20 configuration data) to one or more implantable devices 200. In some embodiments, one or more external devices 500 are configured to transmit both power and data (e.g. simultaneously and/or sequentially) to one or more implantable devices 200. In some embodiments, one or more external devices 500 are further configured to receive data from one or more implantable devices 200 (e.g. via data transmitted by one or more antennas 240 of one or more implantable devices 200). Each external device 500 can comprise housing 510, power supply 570, a transmitter 530, and/or one or more antennas 540, each described in detail herebelow. Each external device 500 can further comprise one or more functional elements 560, such as a functional element comprising a sensor, electrode, energy delivery element, and/or any transducer, also described in detail herebelow. In some embodiments, a functional element 560 comprises one or more sensors configured to monitor performance of external device 500 (e.g. to monitor voltage of power supply 570, quality of transmission of power and/or data to implantable system 20, temperature of a portion of an external device 500, and the like).

One or more housings 510 (singly or collectively housing 510) of each external device 500 can comprise one or more rigid and/or flexible materials which surround various components of external device 500 such as antenna 540, transmitter 530 and/or power supply 570 shown in Fig. 1. In some embodiments, a housing 510 further surrounds a controller 550 and/or a power supply 570. In some embodiments, housing 510 comprises both a rigid material and a flexible material. In some embodiments, housing 510 comprises a material selected from the group consisting of: plastic; injection-molded plastic; an elastomer; metal; and combinations of one or more of these. In some embodiments, housing 510 comprises a shielded portion (e.g. shielded to prevent transmission of electromagnetic waves), and an unshielded portion, such as an unshielded portion surrounding antenna 540.

Housing 510 can comprise an adhesive element, such as an adhesive element configured to temporarily attach an external device 500 to the patient's skin. Housing 510 can be constructed and arranged to engage (e.g. fit in the pocket of) a patient attachment device, such as patient attachment device 70 described herebelow.

One or more antennas 540 (singly or collectively antenna 540) can each comprise one or more external antennas. Antenna 540 can comprise one or more polarizable antennas, such as one or more antennas with adjustable polarization. Antenna 540 can comprise an array of antennas, such as an array of antennas configured to: support beam shaping and/or focusing; allow adjustment of the amplitude and/or phase of the transmission signal; increase the radiation footprint; and combinations of one or more of these. An array of antennas 540 can be configured to be selectively activated, such as to improve coupling with one or more implanted antennas 240, such as to adjust for movement of the array of the antennas 540 relative to the implanted antennas 240. Antenna 540 can comprise an array of selectable conductors configured to adjust a radiation pattern and/or an electromagnetic field of a resultant antenna. Antenna 540 can comprise a surface and shield material positioned on the surface, such as when the shield material is positioned on the side facing away from the patient's skin. The shield material can comprise radio-absorptive shield material and/or radio-reflective shield material.

In some embodiments, a spacer 511 is positioned between antenna 540 and the patient's skin, such as a spacer comprising a thickened portion of housing 510 or a discrete spacer 511 placed on a side of housing 510 (as shown) or on a side of antenna 540. Spacer 511 can comprise one or more materials that match the impedance of antenna 540 to the impedance of the patient's tissue. Spacer 511 can comprise a thickness of between 0.1cm to 3cm, such as a thickness between 0.2cm and 1.5cm. Spacer 511 can comprise materials which isolate heat (e.g. a spacer 511 comprising thermally insulating material). Spacer 511 can comprise a soft or otherwise compressible material (e.g. foam) for patient comfort. Spacer 511 can be inflatable, such as to control the separation distance of an external antenna 540 from the patient's skin. An inflatable spacer 511 can be compartmentalized into several sections with independently controlled air pressure or volume to adjust the separation distance of an external antenna 540 and the patient's skin and/or its angle (e.g. tilt) with respect to the tissue surface.

In some embodiments, antenna 540 comprises a multi-feed point antenna, such as a multi-feed point antenna configured to: support beam shaping and/or focusing; allow adjustment of amplitude and/or phase of a transmission signal; increase the radiation footprint; or combinations of one or more of these.

In some embodiments, antenna 540 comprises one or more antennas selected from the group consisting of: patch antenna; slot antenna; array of antennas; concentric loop antenna; antenna loaded with reactive elements; dipole antenna; polarizable antenna; selectable conductors that form an antenna; and combinations of one or more of these.

Antenna 540 can comprise a major axis between 1cm and 10cm, such as a major axis between 2cm and 5cm. Antenna 540 can be further configured to receive a signal, such as when an antenna 240 is configured to transmit data to an external device 500. Antenna 540 can be positioned on (e.g. fabricated onto) a substrate, such as a flexible printed circuit board or other printed circuit board (e.g. a single or multiple layer printed circuit board comprising electrical traces connecting components).

A single external antenna 540 can be configured to transmit power and/or data to multiple implantable devices 200 (e.g. each containing one or more antennas 240). In some embodiments, a single external device 500, comprising one or more antennas 540 can be configured to transmit power and/or data to multiple implantable devices 200.

One or more antennas 540 can comprise a multi-turn spiral loop antenna, such as a multi-turn spiral loop antenna configured to desensitize coupling sensitivity and/or boost input voltage. In some embodiments, one or more antennas 540 comprise multiple concentric loops with varied dimensions, such as concentric loops configured to desensitize coupling sensitivity. In these embodiments, the multiple concentric loops can be: connected in parallel and driven from the same feed point; driven from the same feed point and connected using one or more of a capacitor, inductor, varactor, and combinations of one or more of these; and/or driven from multiple feed points.

In some embodiments, one or more external devices 500 comprise a first antenna 540 and a second antenna 540. In these embodiments, the first antenna 540 can be similar or dissimilar to the second antenna 540. In some embodiments, a first antenna 540 and a dissimilar second antenna 540 are positioned within a single external device 500 (e.g. within housing 510). In other embodiments, a first antenna 540 is positioned in a first external device 500, and a dissimilar second antenna 540 is positioned in a second external device 500. The similarity or dissimilarity of the antennas can be configured to enhance one or more design and/or performance parameters selected from the group consisting of: implantable device 200 operation depth; polarization; power efficiency; radiation footprint F₁ (described herein); directional gain; beam shaping and/or focusing; sensitivity to implantable device 200 placement; patient comfort; patient usability; data transfer; and combinations of one or more of these. In some embodiments, the first antenna 540 can be optimized for a different design parameter than the second antenna 540, and each antenna 540 can be activated independently or simultaneously to realize both benefits. In some embodiments, the first antenna 540 can be similar to the second antenna 540 and placed in an array to increase the radiation footprint F₁ (described herein) or placed in different external locations to operate with multiple implants 200 implanted at different sites.

In some embodiments, a first external antenna 540 and a second external antenna 540 transmit power and/or data to a single implantable antenna 240. In some embodiments, a first antenna 540 and a second antenna 540 can transmit power and/or data to the one or more antennas 240 simultaneously or sequentially. In sequential power and/or data transfers, a first external device 500 comprising a first one or more antennas 540 can be replaced (e.g. swapped) with a second external device 500 comprising a second one or more antennas 540. Alternatively or additionally, sequential power and/or data transfer can be initiated by one or more of the following conditions: when the first external antenna 540 moves (e.g. moves relative to the implanted antenna 240); when a second external device 500 comprising the second antenna 540 is turned on or otherwise activated; when the second antenna 540 provides improved power and/or data transfer to the antenna 240 than is provided by the first antenna 540; and/or when power received from the first antenna 540 decreases (e.g. decreases below a threshold). In some embodiments, an antenna 240 receives power from a first antenna 540 and a second antenna 540, but only receives data from the first antenna 540.

One or more transmitters 530 (singly or collectively external transmitter 530) can each comprise one or more external transmitters that drive one or more antennas 540 (e.g. one or more antennas 540 positioned in a single external device 500 or multiple external devices 500). Transmitter 530 is operably attached to antenna 540 and is configured to provide one or more drive signals to antenna 540, such as one or more power signals and/or data signals transmitted to one or more implantable devices 200 of implantable system 20. In some embodiments, transmitter 530 comprises a transmitter that operates in a frequency range between 0.1GHz and 3.0GHz, such as a transmitter that operates in a frequency range between 0.4GHz and 1.5GHz, or between approximately 0.902GHz and 0.928GHz. Transmitter 530 can comprise a transmitter that produces a transmission signal with a power level between 0.1W and 4.0W, such as a transmission signal with a power level between 0.1W and 2.0W or between 0.2W and 1.0W.

As described hereabove, one or more external devices 500 can be configured to transmit data (e.g. configuration data) to one or more implantable devices 200, such as via a data transmission produced by transmitter 530 and sent to one or more antennas 540. In some embodiments, a transmitter 530 is configured to perform data modulation comprising amplitude shift keying with pulse-width modulation. In these embodiments, the transmitter can be configured to perform multi-level amplitude shift keying. The amplitude shift-keying can be configured to provide low-depth modulation between 5-75% depth, such as between 10-50% depth. In some embodiments, one or more external devices 500 transmit data to one or more implantable devices 200 using time division multiple access (TDMA). In some embodiments, one or implantable devices 200 are independently addressable through unique identification (ID) codes. Alternatively or additionally, transmitter 530 can be configured to transmit one or more data signals with a bandwidth between 0.1MHz and 100MHz, or between 1MHz and 26MHz.

As described hereabove, one or more external devices 500 can be configured to transmit power to one or more implantable devices 200, such as via a power transmission produced by transmitter 530 and set to one or more antennas 540. One or more transmitters 530 can deliver power to one or more implantable devices 200 simultaneously or sequentially. In some embodiments, one or more transmitters 530 are configured to adjust the level of power transmitted to one or more implantable devices 200, such as by adjusting one or more duty cycling parameters. In these embodiments, power transmitted can be adjusted to: set a power transfer based on a stimulation level produced by implantable system 20; prevent oversaturation; to reduce interference with implantable system 20 data transmissions (e.g. when one or more implantable devices 200 are further configured to transmit data to external system 50); set a power transfer based on charge information and/or discharge information related to an implantable device 200 (e.g. charge rate and/or discharge rate of an implantable energy storage assembly 270); and combinations of one or more of these. In some embodiments, implantable system 20 comprises a first receiver 230 (e.g. of a first implantable device 200) and a second receiver 230 (e.g. of a second implantable device 200). One or more transmitters 530 can be configured to transmit a first power transmission to the first receiver 230, and a second power transmission to the second receiver 230. The first power transmission and the second power transmission can be adjusted or otherwise be different, such as to prevent oversaturation.

In some embodiments, transmitter 530 (and/or another component of external system 50) is further configured as a receiver, such as to receive data from implantable system 20. For example, a transmitter 530 can be configured to receive data via one or more antennas 240 of one or more implantable devices 200. Data received can include patient information (e.g. patient physiologic information, patient environment information or other patient information) and/or information related to an implantable system 20 parameter (e.g. an implantable device 200 configuration parameter as described herein).

In some embodiments, transmitter 530 comprises a first transmitter to transmit power and/or data to one or more implantable devices 200, and a second transmitter to transmit data to a different device, as described herebelow. In these embodiments, a second transmitter of transmitter 530 can be configured to transmit data to tool 60 or another device such as a controller 550, a cell phone; computer; tablet; a computer network such as the internet or a LAN; and combinations of one or more of these. In some embodiments, the second transmitter of transmitter 530 comprises a wireless transmitter; a Bluetooth transmitter; a cellular transmitter; and combinations of one or more of these. In some embodiments, a functional element 560 comprises a transmitter such as a Bluetooth transmitter.

Each power supply 570 (singly or collectively power supply 570) can be operably attached to a transmitter 530, and one or more other electrical components of each external device 500. Power supply 570 can comprise a power supplying and/or energy storage element selected from the group consisting of: battery; rechargeable battery; AC power converter; capacitor; and combinations of one or more of these. In some embodiments, power supply 570 comprises two or more batteries, such as two or more rechargeable batteries, such as to allow the first battery to be replaced (e.g. serially replaced) by the second battery. In some embodiments, power supply 570 is configured to provide a voltage of at least 3V. In some embodiments, power supply 570 is configured to provide a capacity between 1Watt-hour and 75Watt-hours, such as a battery or capacitor with a capacity of approximately 5Watt-hours. In some embodiments, power supply 570 comprises an AC power source.

Each controller 550 (singly or collectively controller 550) comprises an external controller configured to control one or more components of apparatus 10. Controller 550 can comprise a user interface 555. Controller 550 can send and/or receive commands to and/or from one or more external devices 500 via a wireless or wired connection (wired connection not shown but such as one or more insulated conductive wires). In some embodiments, one or more external devices 500 comprise controller 550, such as when user interface 555 is integrated into housing 510 of external device 500. In some embodiments, apparatus 10 comprises multiple external controllers 550.

Controller 550 can be configured to adjust one or more parameters of apparatus 10, such as a stimulation parameter; a sensing parameter; a therapy parameter; a data recording parameter (e.g. a patient data recording parameter and/or an implantable device 200 data recording parameter); power transfer; data rate; activity of one or more external transmitters 530; activity of one or more external antennas 540; a functional element 260 parameter; a functional element 560 parameter; and combinations of one or more of these, such as is described hereabove. Controller 550 can be further configured to provide information, such as patient physiologic information recorded by one or more implantable devices 200, or apparatus 10 information, such as performance and/or configuration information (singly or collectively "status information") of one or more external devices 500 and/or implantable devices 200. In some embodiments, the controller 550 uses information recorded by one or more implantable devices 200, apparatus 10 information, and/or information from external devices 500 to adapt configuration parameters of one or more components of apparatus 10.

In some embodiments, controller 550 can be configured to confirm that an adequate power transmission and/or an adequate data transmission has occurred between one or more external devices 500 and one or more implantable devices 200. In these embodiments, controller 550 can comprise diagnostic assembly 91 described herebelow, or otherwise be configured to detect one or more of: power transmission to the implantable system 20 (e.g. to detect power transmission to implantable system 20 below a threshold); power transmission to the implantable system 20 trending in an undesired direction; improper and/or inadequate data transfer to the implantable system 20; and combinations of one or more of these. In some embodiments, the controller 550 monitors power transfer in real-time and adjusts power transmission accordingly to optimize or at least improve efficiency (e.g. the rectifier 232 efficiency) of one or more implantable devices 200.

In some embodiments, controller 550 and/or another component of apparatus 10 comprises a matching network configured to match the impedance of one or more antennas 540 to one or more transmitters 530. The matching network can comprise an adjustable matching network. The matching network can comprise a directional coupler configured to measure a reflection coefficient. A transmitter 530 can comprise an output, and a controller 550 can be configured to monitor a standing wave pattern at the output of the transmitter 530.

In some embodiments, controller 550 can comprise a temperature sensor, such as a functional element 560 described herein configured as a temperature sensor and positioned proximate a portion of controller 550, housing 510 and/or one or more antennas 540 (e.g. to measure the temperature of one or more portions of an external device 500). In these embodiments, the temperature data recorded by the functional element 560 is used to adjust one or more of: matching network; stimulation level (e.g. stimulation energy delivered by one or more implantable devices 200); power transmission level (e.g. level of power transmitted between one or more external devices 500 and one or more implantable devices 200); and combinations of one or more of these. In some embodiments, the temperature sensor-based functional element 560 is a part of a safety mechanism that deactivates controller 550 and/or another component of apparatus 10 if the recorded temperature exceeds a threshold. Alternatively or additionally, a temperature sensor-based functional element 560 can be configured to measure temperature of the patient, such as when placed on housing 510, such as to adjust energy or agent delivery performed by implantable device 200 based on the recorded patient temperature.

In some embodiments, controller 550 comprises a lookup table of stimulation signal waveform patterns, such as to allow a clinician, patient and/or other operator of apparatus 10 to select a predetermined stimulation pattern. In some embodiments, controller 550 comprises a set of adjustable stimulation signal parameters configured to be varied to allow an operator to construct customized waveforms, the adjustable stimulation signal parameters selected from the group consisting of: frequency; amplitude; duty cycle; duration of pulse and/or amplitude level; duration of stimulation waveform; repetition of stimulation waveform; pulse shape; and combinations of one or more of these. In some embodiments, the controller 550 is configured to allow an operator to create a customized waveform by specifying an amplitude at one or more discrete steps of a stimulation signal.

In some embodiments, controller 550 comprises a transmitter configured to transmit data to tool 60 or another device such as a cell phone; computer; tablet; a computer network such as the internet or a LAN; and combinations of one or more of these. In these embodiments, controller 550 can comprise a wireless transmitter; a Bluetooth transmitter; a cellular transmitter; and combinations of one or more of these.

User interface 555 of each controller 550 can comprise one or more user input components and/or user output components, such as a component selected from the group consisting of: keyboard; mouse; keypad; switch; membrane switch; touchscreen; display; audio transducer such as a speaker or buzzer; vibrational transducer; light such as an LED; and combinations of one or more of these.

In some embodiments, one or more components of external system 50 and/or other external component of apparatus 10, comprises one or more functional elements 560, such as functional elements 560a and/or 560b (singly or collectively functional element 560), shown positioned in controller 550 and in external device 500, respectively. Each functional element 560 can comprise a sensor, an electrode, an energy delivery element, an agent delivery element, and/or any transducer. In some embodiments, one or more functional elements 560 comprise a transducer selected from the group consisting of: light; light emitting diode; wireless transmitter; Bluetooth device; mechanical transducer; piezoelectric transducer; pressure transducer; temperature transducer; humidity transducer; vibrational transducer; audio transducer; speaker; and combinations of one or more of these. In some embodiments, functional element 560 comprises a needle, a catheter (e.g. a distal portion of a catheter), an iontophoretic element or a porous membrane, such as an agent delivery element configured to deliver one or more agents contained (e.g. one or more agents in a reservoir, such as reservoir 525 described herebelow) within an external device 500 and delivered into the patient (e.g. into subcutaneous tissue, into muscle tissue and/or into a blood vessel such as a vein). In some embodiments, the functional element 560 can comprise an electrode for sensing electrical activity and/or delivering electrical energy. In some embodiments, apparatus 10 is configured to cause stochastic resonance, and the addition of white noise can enhance the sensitivity of nerves to be stimulated and/or boosts weak signals to be recorded.

In some embodiments, one or more functional elements 560 comprise a sensor, such as a sensor configured to record data related to a patient parameter (e.g. a patient physiologic parameter), an external system 50 parameter and/or an implantable system 20 parameter. In some embodiments, operation of one or more implantable devices 200 (e.g. stimulation energy delivered by one or more implantable devices 200) is configured to be delivered based on the data recorded by one or more sensor-based functional elements 560, such as in a closed-loop energy delivery mode.

Functional element 560 can comprise one or more sensors selected from the group consisting of: electrode; sensor configured to record electrical activity of tissue; blood glucose sensor such as an optical blood glucose sensor; pressure sensor; blood pressure sensor; heart rate sensor; inflammation sensor; neural activity sensor; muscular activity sensor; pH sensor; strain gauge; accelerometer; gyroscope; GPS; respiration sensor; respiration rate sensor; temperature sensor; magnetic sensor; optical sensor; MEMs sensor; chemical sensor; hormone sensor; impedance sensor; tissue impedance sensor; body position sensor; body motion sensor; physical activity level sensor; perspiration sensor; patient hydration sensor; breath monitoring sensor; sleep monitoring sensor; food intake monitoring sensor; urine movement sensor; bowel movement sensor; tremor sensor; pain level sensor; and combinations of one or more of these.

Functional element 560 can comprise one or more sensors configured to record data regarding a patient parameter selected from the group consisting of: blood glucose; blood pressure; EKG; heart rate; cardiac output; oxygen level; pH level; pH of blood; pH of a bodily fluids; tissue temperature; inflammation level; bacteria level; type of bacteria present; gas level; blood gas level; neural activity; neural spikes; neural spike shape; action potential; local field potential (LFP); EEG; muscular activity; gastric volume; peristalsis rate; impedance; tissue impedance; electrode-tissue interface impedance; physical activity level; pain level; body position; body motion; organ motion; respiration rate; respiration level; perspiration rate; sleep level; sleep cycle; digestion state; digestion level; urine production; urine flow; bowel movement; tremor; ion concentration; chemical concentration; hormone level; viscosity of a bodily fluid; patient hydration level; and combinations of one or more of these.

Functional element 560 can comprise one or more sensors configured to record data representing a parameter of external system 50 or any component of apparatus 10. Functional element 560 can comprise one or more sensors selected from the group consisting of: an energy sensor; a voltage sensor; a current sensor; a temperature sensor (e.g. a temperature of one or more components of external device 500 or controller 550); an antenna matching and/or mismatching assessment sensor; power transfer sensor; link gain sensor; power use sensor; energy level sensor; energy charge rate sensor; energy discharge rate sensor; impedance sensor; load impedance sensor; instantaneous power usage sensor; average power usage sensor; bit error rate sensor; signal integrity sensor; and combinations of one or more of these. Apparatus 10 can be configured to analyze (e.g. via controller 250) the data recorded by functional element 560 to assess one or more of: power transfer; link gain; power use; energy within power supply 570; performance of power supply 570; expected life of power supply 570; discharge rate of power supply 570; ripple or other variations of power supply 570; matching of antennas 240 and 540; communication error rate between implantable device 200 and external device 500; integrity of transmission between implantable device 200 and external device 500; and combinations of one or more of these.

In some embodiments, one or more functional elements 560 are positioned on a housing 510. A functional element 560 can comprise a body conduction sensor, such as a body conduction sensor configured to record and/or receive data via skin conduction. A functional element 560 can be configured to record data associated with stimulation delivered by one or more implantable devices 200 (e.g. record data associated with stimulation energy delivered by one or more functional elements 260), such as to provide closed loop or semi-closed loop stimulation. A functional element 560 can be configured to record temperature, such as when apparatus 10 is configured to deactivate or otherwise modify the performance of an external device 500 when the recorded temperature (e.g. patient temperature and/or external device 500 temperature) exceeds a threshold.

Implantable system 20 comprises one or more implantable devices 200, such as one or more implantable devices 200 provided sterile or configured to be sterilized for implantation into the patient. A first implantable device 200 can be of similar or dissimilar construction and arrangement to a second implantable device 200. Each implantable device 200 can be configured to treat a patient and/or record patient information, such as by delivering energy and/or an agent to tissue and/or by recording one or more physiologic parameters of tissue.

One or more portions of an implantable device 200 or other component of implantable system 20 can be configured to be visualized or contain a visualizable portion or other visualizable element, such as visualizable element 222 shown. Visualizable element 222 can comprise a material selected from the group consisting of: radiopaque material; ultrasonically reflective material; magnetic material; and combinations of one or more of these. In these embodiments, each implantable device 200 can be visualized (e.g. during and/or after implantation) via an imaging device such as an X-ray, fluoroscope, ultrasound imager and/or MRI.

In some embodiments, implantable system 20 comprises multiple implantable devices 200 and implantable system 20 comprises a "multi-point ready" system, in which the operation (e.g. energy delivery, agent deliver, data recording and/or other function) of the multiple implantable devices 200 is performed simultaneously, asynchronously, and/or sequentially. The implantable devices 200 can be part of a network with one or more external devices 500 in which the treating of a patient and/or the recording of patient information relies on operation of the implantable devices 200 at one or more implantation sites in a synchronized, asynchronized, and/or otherwise coordinated way. The synchronization or otherwise coordination can be controlled by a single or multiple external devices 500, which can further be synchronized to a single clock. Each implantable device 200 of implantable system 20 can receive a power signal and/or a data signal from one or more external devices 500. In some embodiments of the multi-point ready implantable system 20, each implantable device 200 comprises a unique ID, such that each implantable device 200 can be individually addressed (e.g. receive unique signals from external system 50). In some embodiments, external system 50 transmits high-bandwidth signals to implantable system 20, such that time-domain multiple access communication can be performed while operating in near real time. In some embodiments, implantable system 20 is configured as a multi-point ready system such that stimulation energy delivered by implantable system 20 is independent of power received by implantable system 20 from external system 50.

Each implantable device 200 is configured to receive power and/or data (e.g. implantable system 20 configuration data) from one or more external devices 500. In some embodiments, one or more implantable devices 200 are configured to receive both power and data (e.g. simultaneously and/or sequentially) from one or more external devices 500. In some embodiments, a single external device 500 sends power and/or data to multiple implantable devices 200. Alternatively or additionally, a single implantable device 200 can receive power and/or data from multiple external devices 500. In some embodiments, a first external device 500 is positioned on or near the patient's skin at a location proximate an implanted first implantable device 200, and a second external device 500 is positioned on or near the patient's skin (hereinafter "on" the patient's skin) at a location proximate an implanted second implantable device 200. In these embodiments, the first external device 500 transmits data and/or power to at least the first implantable device 200 and the second external device 500 transmits data and/or power to at least the second implantable device 200.

Each implantable device 200 can comprise one or more functional elements 260, configured to stimulate, deliver energy to, deliver an agent to, record information from and/or otherwise interface with the patient. Alternatively or additionally, the one or more functional elements 260 can be configured to record patient information. Each implantable device 200 can comprise housing 210, receiver 230, controller 250, energy storage assembly 270 and/or one or more antennas 240, each described in detail herebelow. Each functional element 260 can comprise a sensor and/or any transducer, as described in detail herebelow. One or more functional elements 260 can be positioned on a lead 265, such as is described herebelow in reference to Fig. 2. Each implantable device 200 can further comprise anchor element 221, as described in detail herebelow.

In some embodiments, one or more implantable devices 200 are further configured to transmit data to one or more external devices 500, such as via one or more antennas 240 transmitting a signal to one or more antennas 540, or otherwise. Data transmitted by an implantable device 200 can comprise patient information (e.g. patient physiologic information recorded by one or more functional elements 260 configured as a physiologic sensor), or implantable device 200 information (e.g. data recorded by one or more functional elements 260 configured as a sensor and positioned in implantable device 200, or other implantable device 200 configuration and/or performance data).

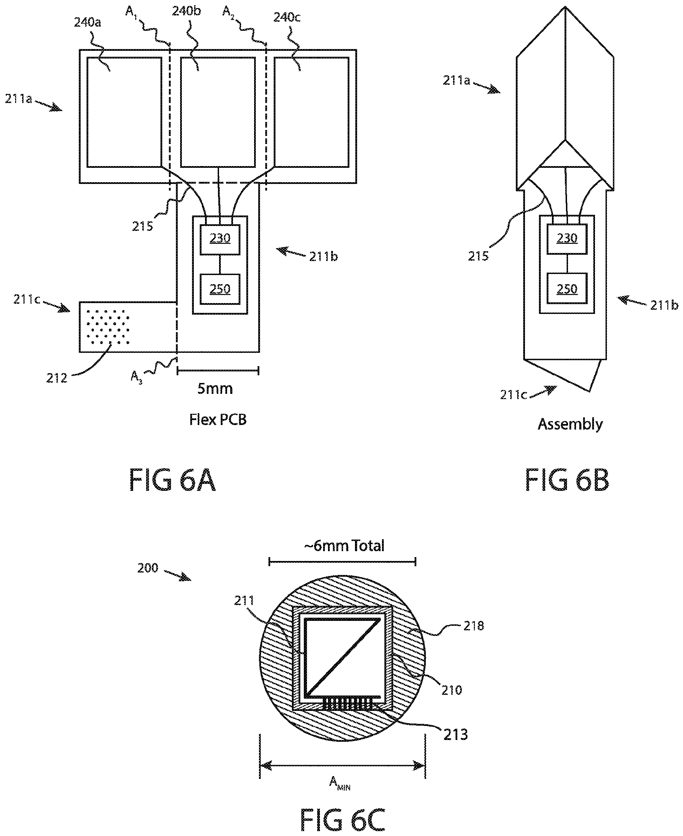

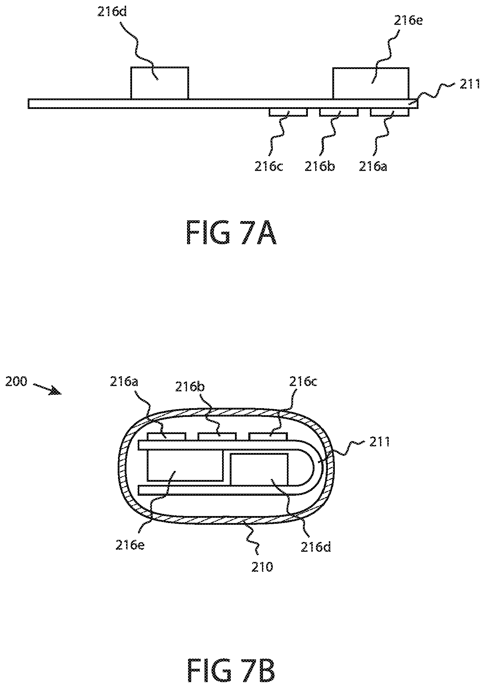

Housing 210 of each implantable device 200 can comprise one or more rigid and/or flexible materials which surround various components, such as antenna 240, energy storage assembly 270, controller 250 and/or receiver 230 as shown in Fig. 1. In some embodiments, one or more functional elements 260 are positioned in, on and/or within housing 210. In some embodiments, housing 210 surrounds a substrate, such as a flexible and/or foldable printed circuit board as described herebelow in reference to Figs. 6A-C or Figs. 7A-B, such as multiple discrete or continuous printed circuit boards positioned in different planes (e.g. a flexible or foldable printed circuit board).

Housing 210 can comprise one or more shapes or combination of shapes, such as one or more shapes selected from the group consisting of: disc; pill; cylinder; sphere; rectangular prism; trapezoidal prism; a portion of a toroid; and combinations of one or more of these.

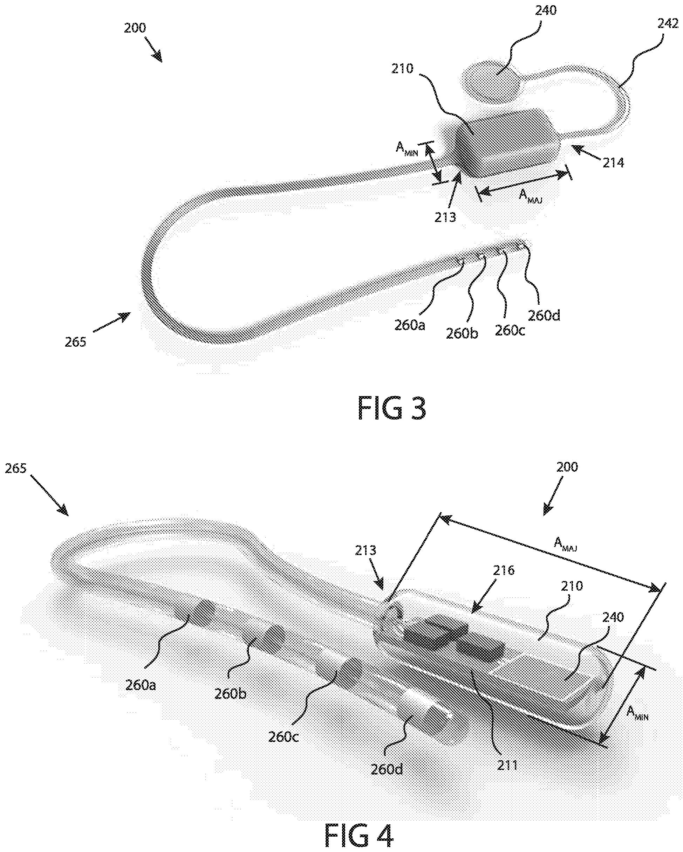

Housing 210 can comprise a major axis and a minor axis, defined hereabove and such as is described herebelow in reference to Figs. 3, 4 or 6C. In some embodiments, housing 210 comprises a major axis less than or equal to 20mm, such as a major axis less than or equal to 15mm, 12mm or 10mm. In some embodiments, housing 210 comprises a minor axis less than or equal to 8mm, such as a minor axis less than or equal to 6mm, or less than or equal to 5mm. Housing 210 can comprise a wall thickness between 0.2mm and 1.0mm, such as a wall thickness between 0.2mm and 0.5mm, such as a wall thickness of approximately 0.3mm. Housing 210 can comprise a displacement volume less than or equal to 2000mm³, such as less than or equal to 600mm³.

Housing 210 can comprise one or more portions that are transmissive to radiofrequency (RF) signals. In some embodiments, housing 210 comprises glass. In some embodiments, housing 210 comprises a material selected from the group consisting of: glass; ceramic; stainless steel; titanium; polyurethane; an organic compound; liquid crystal polymer (LCP); gold; platinum; tungsten; and combinations of one or more of these.

Housing 210 can comprise one or more passageways or other feedthroughs, such as for the passage of a lead, wire, optical fiber, fluid delivery tube, mechanical linkage and/or other conduit through a wall of housing 210, such as is described herebelow in reference to feedthroughs 213 of Fig. 6C.

In some embodiments, one or more inner or outer surfaces (or portions of surfaces) of housing 210 includes an insulating and/or shielding layer (e.g. a conductive electromagnetic shielding layer), such as inner coating 219a and/or outer coating 219b shown (singly or collectively coating 219). Coating 219 can comprise an electrically insulating and/or a thermally insulating layer or other coating. In some embodiments, one or more portions of housing 210 comprise an electrically shielding coating 219, while other portions are transmissive to electromagnetic signals such as radiofrequency signals.

In some embodiments, one or more implantable devices 200 comprises one or more anchor elements configured to secure one or more portions of implantable device 200 to tissue, such as anchor element 221 shown positioned on housing 210. Anchor element 221 can comprise one or more anchoring elements selected from the group consisting of: silicone sleeve; suture tab; suture eyelet; bone anchor, wire loops; porous mesh; penetrable wing; penetrable tab; bone screw eyelet; tine; pincers; suture slits; and combinations of one or more of these.

One or more antennas 240 (singly or collectively antenna 240) can be configured to receive power and/or data, and receiver 230 can receive the power and/or data from the one or more antennas 240. Each antenna 240 can comprise one or more implantable antennas, such as one or more antennas positioned within housing 210 (as described herebelow in reference to Fig. 6C), and/or one or more antennas electrically attached to a tether (as described herebelow in reference to Fig. 3). In some embodiments, one or more implantable devices 200 comprise at least two antennas 240, or at least three antennas 240. Antenna 240 can be configured to receive power and/or data from one or more external devices 500, such that an attached receiver 230 receives the power and/or data. In some embodiments, implantable system 20 comprises at least two implantable devices 200, each of which comprise one or more (e.g. two or three) antennas 240 which are positioned within a housing 210 (e.g. as described herebelow in reference to Fig. 4) and/or electrically tethered to a housing 210 (e.g. as described herebelow in reference to Fig. 3). In some embodiments, an implantable device 200 comprises a first antenna 240 positioned in a first plane and a second antenna 240 positioned in a second plane. The first plane and second plane can be relatively orthogonal planes, or planes oriented between 30° and 90° relative to each other, such as between 40° and 90°, approximately 30°, approximately 45° and/or approximately 60° relative to each other. In some embodiments, an implantable device 200 comprises a first antenna 240 positioned in a first plane, a second antenna 240 positioned in a second plane, and a third antenna 240 positioned in a third plane, such as is described herebelow in reference to Figs. 6B-C.

In some embodiments, implantable device 200 comprises one or more antennas 240 positioned on a substrate, such as a printed circuit board (PCB), a flexible printed circuit board and/or a foldable substrate (e.g. a substrate comprising rigid portions and hinged portions), such as is described herebelow in reference to substrate 211 and antennas 240 of Figs. 6A-C. In some embodiments, the substrate can be folded or otherwise pivoted to position the various antennas 240 on differently oriented planes, such as multiple planes oriented between 5° and 90° relative to each other, such as two antennas 240 positioned on two planes oriented between 30° and 90° or between 40° and 90° relative to each other, or three antennas 240 positioned on three planes oriented between 5° and 60° relative to each other. Two or more antennas 240 can be positioned on two or more different planes that are approximately 45° relative to each other, or approximately 60° or approximately 90° relative to each other.

Implantable device 200 can comprise three antennas 240. In some embodiments, a first antenna 240 can comprise an electrical dipole antenna, and the second and third antennas 240 can be positioned in different planes than the first antenna 240. In some embodiments, the three antennas 240 each comprise a loop antenna, such as when each loop antenna is positioned on a different plane. In some embodiments, a first antenna 240 comprises an electrical dipole antenna, and a second antenna 240 and a third antenna 240 each comprise a loop antenna. In these embodiments, the second antenna 240 and the third antenna 240 can be positioned relatively orthogonal to each other (e.g. positioned on two relatively orthogonal planes). In some embodiments, a first antenna (e.g. an electrical dipole antenna) is positioned outside of housing 210, while a second antenna (e.g. a loop antenna) and a third antenna (e.g. a loop antenna) are each positioned on, in and/or within housing 210. In some embodiments, implantable device 200 can comprise one or more antennas 240 in which any combination of antenna types (as described herein) are used in combination.

One or more antennas 240 can comprise an antenna selected from the group consisting of: loop antenna; multiple-turn loop antenna; planar loop antenna; coil antenna; dipole antenna; electric dipole antenna; magnetic dipole antenna; patch antenna; loaded dipole antenna; concentric loop antenna; loop antenna with ferrite core; and combinations of one or more of these. One or more antennas 240 can comprise a loop antenna, such as an elongated loop antenna or a multiple-turn loop antenna.

One or more antennas 240 can comprise a multi-turn spiral loop antenna, such as a multi-turn spiral loop antenna configured to desensitize coupling sensitivity and/or boost input voltage. In some embodiments, one or more antennas 240 comprise multiple concentric loops with varied dimensions, such as concentric loops configured to desensitize coupling sensitivity. In these embodiments, the multiple concentric loops can be: connected in parallel and driven from the same feed point; driven from the same feed point and connected using one or more of a capacitor, inductor, varactor, and combinations of one or more of these; and/or driven from multiple feed points.

One or more antennas 240 can comprise a minor axis and a major axis. In some embodiments, one or more antennas 240 comprise a minor axis between 1mm and 8mm, such as between 2mm and 5mm. In some embodiments, one or more antennas 240 comprise a major axis between 3mm and 15mm, such as between 4mm and 8mm. In some embodiments, one or more antennas 240 comprise a major axis above 3mm, such as between 3mm and 15mm, such as when the antenna 240 is positioned outside of housing 210.

One or more antennas 240 can comprise a foldable and/or unfoldable antenna, such as is described in applicant's co-pending International PCT application PCT/US2014/043023 (Publication No. WO 2014/205129), titled "Method and Apparatus for Minimally Invasive Implantable Modulators", filed June 18, 2014.

One or more antennas 240 can be positioned inside of housing 210, such as is described herebelow in reference to Figs. 4, 6A-C, and 7A-B. Alternatively or additionally, one or more antennas 240 can be positioned outside of housing 210, such as is described herebelow in reference to Fig. 3.

Implantable system 20, one or more implantable devices 200 and/or one or more antennas 240 can be configured to be positioned at a desired depth beneath the patient's skin, such as at a depth between 0.5cm and 7.0cm, such as a depth of between 1.0cm and 3.0cm.

One or more energy storage assemblies 270 (singly or collectively energy storage assembly 270) can comprise one or more implantable energy storage components, such as one or more batteries (e.g. rechargeable batteries) and/or capacitors. Energy storage assembly 270 can be configured to provide power to one or more of: one or more functional elements 260; controller 250; receiver 230; and combinations of one or more of these. In some embodiments, energy storage assembly 270 further provides power to one or more antennas 240. In some embodiments, energy storage assembly 270 can include digital control for charge/discharge rates, voltage outputs, current outputs, and/or system power distribution and/or management.

Energy storage assembly 270 can comprise one or more capacitors with a single or collective capacitance between 0.01µF and 10F, such as a capacitance between 1µF and 1.0mF, or between 1µF and 10µF. The energy storage assembly 270 can comprise one or more capacitors with capacitance between 1mF and 10F, such as when energy storage assembly 270 comprises super capacitors and/or ultra capacitors. Such large capacitance can be used to store sufficient charge to maintain operation (e.g. maintain delivery of stimulation energy and/or delivery of an agent) without the use (e.g. sufficient proximity) of an associated external device 500. A capacitor or other energy storage element (e.g. a battery) can be chosen to provide sufficient energy to maintain operation for at least 30 seconds, at least 2 minutes, at least 5 minutes, at least 30 minutes, and up to several hours or more (e.g. during the use of shower or during swimming). Energy storage assembly 270 can comprise one or more capacitors with a breakdown voltage above 1.0V, such as a breakdown voltage above 1.5V, 4.0V, 10V, or 15V. In some embodiments, energy storage assembly 270 can comprise capacitors distributed outside of housing 210, such as when one or more capacitors are distributed along lead 265. Energy storage assembly 270 can comprise one or more capacitors with low self-leakage, such as to maintain stored energy for longer periods of time.

One or more controllers 250 (singly or collectively controller 250) can be configured to control one or more functional elements 260, such as a functional element 260 comprising a stimulation-based transducer (e.g. an electrode or other energy delivery element) and/or a sensor (e.g. a physiologic sensor and/or a sensor configured to monitor an implantable device 200 parameter). In some embodiments, controller 250 is configured to transmit a stimulation signal (e.g. transmit stimulation energy) to one or more functional elements 260 (e.g. one or more functional elements 260 comprising an electrode and/or other energy delivery element), independent of the power signal received by one or more antennas 240 (e.g. independent of power transmitted by external system 50), such as by using energy stored in energy storage assembly 270. In these embodiments, the power signal and/or the RF path for the power signal can be adjusted to optimize power efficiency (e.g. by tuning matching network on transmitter 530 and/or receiver 230; configuring antennas 540 and/or 240 in an array; tuning operating frequency; duty cycling the power signal; adjusting antenna 540 and/or 240 position; and the like), and a stimulation signal can be precisely delivered (e.g. by using energy stored on energy storage assembly 270 and generating stimulation signal locally on the implantable device 200) to ensure clinical efficacy. Also, if the power signal transmission (also referred to as "power link") is perturbed unexpectedly, the stimulation signal can be configured so that it is not significantly affected (e.g. unaffected). In some configurations, the stimulation signal being delivered by one or more implantable devices 200 can be insensitive to interference that may be present. In these embodiments, a power transmission signal and stimulation signal can vary in one or more of: amplitude; changes in amplitude; average amplitude; frequency; changes in frequency; average frequency; phase; changes in phase; average phase; waveform shape; pulse shape; duty cycle; polarity; and combinations of one or more of these.

Controller 250 can receive commands from receiver 230, such as one or more commands related to one or more implantable device 200 configuration parameters selected from the group consisting of: stimulation parameter; data rate of receiver; data rate of data transmitted by the first implantable device 200 at least one implantable antenna 240; functional element 260 configuration; state of controller 250; antenna 240 impedance; clock frequency; sensor configuration; electrode configuration; power management parameter; energy storage assembly parameter; agent delivery parameter; sensor configuration parameter; and combinations of one or more of these.

In some embodiments, one or more functional elements 260 comprise a stimulation element configured to deliver energy (e.g. electrical energy) to tissue, and controller 250 is configured to control the energy delivery, such as to control one or more of: amplitude; frequency; pulse width; voltage; current; pulse shape; duty cycle; polarity; drive impedance; energy storage capacity; and combinations of one or more of these.

In some embodiments, one or more functional elements 260 comprise an element configured to deliver electrical energy to tissue (e.g. an electrode), and controller 260 is configured to control charge balance, such as to actively control charge balance. Charge balance can be essential for patient safety in electrical stimulation of nerves or other tissue. Imbalanced stimulation waveforms can cause electrode corrosion and/or dissolution which can lead to deposition of toxic materials in tissue, implant rejection, and nerve damage. The stimulation waveform can be balanced such that net outflow charge approximately equals net inflow charge. With stimulation waveform amplitudes that can vary between 0.1 mA to 10 mA, depending on the treatment, the error in charge balance can be on the order of 0.001% to 0.01%. Controller 250 can comprise AC coupling capacitors that are configured to balance stimulation waveforms passively. The AC coupling capacitance can be fairly large (e.g. greater than 10µF), in order to pass the stimulation waveform with minimal filtering. In some embodiments, apparatus 10 can be configured to perform active charge balancing. In some embodiments, an implantable device 200 can comprise a precise resistor in series with a stimulation electrode-based functional element 260. The precise resistor can be used to measure outflow and inflow currents, such as when controller 250 comprises an analog to digital converter (ADC). Controller 250 can integrate current over time during a first phase in which stimulation energy is delivered, and during a second phase in which a reverse current is applied (e.g. a reverse current used to balance charge). Controller 250 can be configured to balance the total charge in the two phases, to ensure that the net DC current is approximately zero. The integration can be achieved using an analog integrator and/or a digital summer of controller 250, with controller 250 keeping track of the pulse duration. Implantable device 200 can comprise a precise series resistance comprising an on-chip trimmed resistor or an off chip resistor. In some embodiments, implantable device 200 comprises a bank of trimmed resistors that are used to control the net series resistance, such as to adjust resistance based on stimulation amplitude requirements (e.g. to take advantage of the full dynamic range of an ADC of controller 250). In some embodiments, controller 250 comprises a shunt path with an RC-based low pass filter used for both outflow and inflow of current. RC elements of controller 250 can be chosen such that the shunt current is only a fraction of the stimulation current. Since the same RC elements can be used for both outflow and inflow current, the precision required for the RC components can be lower. An ADC can be used to sense the voltage on the capacitor at the end of a stimulation pulse. After the stimulation pulse, the capacitor can be discharged and the polarity of the stimulation current can be reversed and set to any amplitude, until the capacitor is charged to approximately the same voltage (according to the ADC precision) as it was charged during the stimulation pulse. The ADC resolution can be high enough to ensure the residual error is less than what would cause an undesired charge accumulation. ADC resolution requirements can be further reduced by reducing the net capacitance in a shunt RC circuit, to cause accelerated charging of the capacitor. The capacitor can be discharged every time the voltage exceeds a certain predefined threshold, while controller 250 keeps track of the number of times the capacitor has been charged and reset. By resetting the capacitor through a low resistance path, the discharge time can be insignificant compared to the charge time, reducing the error due to discharge period. Since the net charge equivalent to full scale voltage on the ADC can be divided into multiple cycles, the required resolution of the ADC to achieve the same residual error can be divided by the number of cycles.

In some embodiments, controller 250 is configured to produce a stimulation signal comprising a waveform or a waveform pattern (hereinafter stimulation waveform), for one or more functional elements 260 configured as a stimulation element (e.g. such that one or more functional elements 260 deliver stimulation energy comprising or at least resembling that stimulation waveform). Controller 250 can produce a stimulation signal comprising a waveform selected from the group consisting of: square wave; sine wave; triangle wave; ramp; waveform with exponential increase; waveform with exponential decrease; pulse shape which minimizes power consumption; Gaussian pulse shape; pulse train; root-raised cosine; bipolar pulses; and combinations of one or more of these. In some embodiments, controller 250 is configured to produce a stimulation signal comprising a waveform including a combination of two or more waveforms selected from the group consisting of: square wave; sine wave; triangle wave; ramp; waveform with exponential increase; waveform with exponential decrease; pulse shape which minimizes power consumption; Gaussian pulse shape; pulse train; root-raised cosine; bipolar pulses; and combinations of one or more of these. In some embodiments, controller 250 is configured to construct a custom waveform (e.g. an operator customized waveform), such as by adjusting amplitude at specified time steps.

In some embodiments, controller 250 is configured to provide a stimulation signal comprising waveforms and/or pulses repeated at a frequency (e.g. includes a frequency component) between 1.0Hz and 50kHz, such as between 10Hz and 500Hz, between 40Hz and 160Hz and/or between 5kHz and 15kHz. In some embodiments, controller 250 is configured to produce a stimulation signal comprising a frequency between 1Hz and 1000Hz, such as a stimulation signal with a frequency between 10Hz and 500Hz. In some embodiments, controller 250 is configured to produce a stimulation signal comprising a duty cycle between 0.1% and 25%, such as a duty cycle between 1% and 10%. In some embodiments, controller 250 is configured to produce a stimulation signal comprising a frequency modulated stimulation waveform, such as a stimulation waveform comprising a frequency component between 1kHz and 20kHz. In some embodiments, controller 250 is configured to produce a stimulation signal comprising a mix and/or modulation of low frequency and high frequency signals, which can be of any of the waveform shapes described herein. In these embodiments, the stimulation signal can comprise low frequency signals between 1Hz and 1000Hz, and high frequency signals between 1kHz and 50kHz, or between 1kHz and 20kHz. Alternatively or additionally, the stimulation signal can comprise a train of high frequency signals and bursts of low frequency signals, and/or a train of low frequency signals and bursts of high frequency signals. Alternatively or additionally, the stimulation signal can comprise one or more high frequency signals modulated with one or more low frequency signals. Alternatively or additionally, the stimulation signal can cycle among different waveforms shapes at specified time intervals. Alternatively or additionally, the stimulation signal can comprise a pseudo random binary sequence (PRBS) non-return-to-zero or return-to-zero waveform.

Controller 250 can comprise a clamping circuit configured to allow fast charging and/or discharging of the energy storage assembly 270, functional element 260 drivers (e.g. electrode drivers) of controller 250, and/or other components of implantable device 200. The clamping circuit can improve pulse shape by offering additional control and/or configuration of rise and fall times in the shape of the waveform (e.g. to create rapid rise or fall times). In some embodiments, the clamping circuit can be configured to limit the rise and/or fall time to be less than or equal to one-tenth (10%) of the pulse width of an applied stimulation pulse (e.g. less than or equal to 1µs rise and/or fall time for a 10µs stimulation pulse).

In some embodiments, controller 250 comprises a matching network configured to match the impedance of a first antenna 240 with the impedance of the receiver 230. In these embodiments, controller 250's matching network can be adjustable. Alternatively or additionally, controller 250 can comprise an adjustable loading impedance to stabilize the load seen at an antenna 240 under different operating conditions. In some embodiments, the adjustable loading impedance is controlled according to the charge rate of the energy storage assembly 270.

Controller 250 and/or any other component of each implantable device 200 can comprise an integrated circuit comprising one or more components selected from the group consisting of: matching network; rectifier; DC-DC converter; regulator; bandgap reference; overvoltage protection; overcurrent protection; active charge balance circuit; analog to digital converter (ADC); digital to analog converter (DAC); current driver; voltage driver; digital controller; clock generator; data receiver; data demodulator; data modulator; data transmitter; electrode drivers; sensing interface analog front end; power management circuit; energy storage interface; memory register; timing circuit; and combinations of one or more of these.

One or more **receivers 230** (singly or collectively receiver 230) can comprise one or more assemblies, such as demodulator 231, rectifier 232 and/or power converter 233 shown in Fig. 1. In some embodiments, receiver 230 can comprise a DC-DC converter such as a boost converter. Receiver 230 can comprise a data receiver, such as a data receiver including an envelope detector and demodulator and/or an envelope averaging circuit. In some embodiments, one more antennas 240 separately connect to one or more receivers 230. In some embodiments, one or more antennas 240 connect to a single receiver 230, such as via a series connection or a parallel connection.

One or more implantable devices 200 can be configured to transmit a data signal to external system 50. In some embodiments, receiver 230 is configured to drive one or more antennas 240 to transmit data to external system 50 (e.g. to an antenna 540 of an external device 500). Alternatively or additionally, implantable device 200 can be configured to transmit a data signal by having receiver 230 adjust a load impedance to backscatter energy, such as a backscattering of energy which can be detected by external system 50. In some embodiments, data transmission is accomplished by receiver 230 manipulating a signal at a tissue interface, such as to transmit a data signal using body conduction.

In some embodiments, receiver 230 comprises a matching network, such as a matching network configured to detune to prevent oversaturation. For example, implantable system 20 can comprise two or more implantable device 200 each of which includes a receiver 230 comprising a matching network. A first implantable device 200's receiver 230's matching network can be configured to detune based on power received by the second implantable device 200's receiver 230.

Demodulator 231 can comprise circuitry that asynchronously recovers signals modulated on the power signal provided by external system 50, and converts the modulated signals into digital signals. In some embodiments, demodulator 231 asynchronously recovers the modulated signal by comparing a dynamically generated moving average with the envelope, outputting a high voltage when the envelope is greater than the moving average and a low voltage when the envelope is less than the moving average. Data can then be extracted from this resulting digital signal from the width and/or amplitude of the pulses in the signal, according to the encoding method used by external system 50. In some embodiments, demodulator 231 recovers a digital signal that can be used as timing information for an implantable device 200, similar to an on-chip clock. The recovered clock signal can also be used to synchronize an on-chip clock generator of controller 250, such as through the use of a frequency and/or phase locked loop (FLL or PLL).

Rectifier 232 can comprise a power signal rectifier, such as to provide power to the energy storage assembly 270 and/or controller 250. In some embodiments, rectifier 232 comprises one or more self-driven synchronous rectifier (SDSR) stages connected in charge-pump configuration, to boost the voltage from input RF amplitude to the rectifier to a higher voltage. The boosted voltage can directly charge energy storage assembly 270, or be further boosted by a DC-DC converter or boost converter. In some embodiments, rectifier 232 can comprise diode-capacitor ladder stages instead of, or in addition to, SDSR stages. On-chip diodes, such as Schottky diodes, or off-chip diodes can be used in one or more rectifier 232 stages. For maximum efficiency, the rectification elements, such as diodes, can be optimized to minimize forward conduction and/or reverse conduction losses by properly sizing the components and selecting appropriate number of stages based on the input RF voltage and load current.

Power converter 233 can comprise one or more voltage conversion elements such as DC-DC converters that boost or otherwise change the voltage to a desired level. In some embodiments, voltage conversion is achieved with a buck-boost converter, a boost converter, a switched capacitor, and/or charge pumps. One or more power converters 233 can interface with energy storage assembly 270 and charge up associated energy storage components to desired voltages. In some embodiments, power converter 233 receives control signals from controller 250, such as to configure voltages, currents, charge/discharge rates, switching frequencies, and/or other operating parameters of power converter 233.

One or more implantable leads 265 (singly or collectively lead 265) can be attached to one or more housings 210, such as a lead 265 comprising one or more functional elements 260. Lead 265 can comprise one or more functional elements 260 configured as a stimulation element (e.g. an electrode configured to deliver electrical energy in monopolar or bipolar mode or an agent delivery element such as an output port fluidly connected to a reservoir within housing 210). Alternatively or additionally, lead 265 can comprise one or more functional elements 260 configured as a physiologic sensor (e.g. an electrode configured to record electrical activity of tissue or other physiologic sensor as described herebelow). Alternatively or additionally, lead 265 can comprise one or more functional elements 260 configured to transmit signals through tissue to external system 50, such as through body conduction.

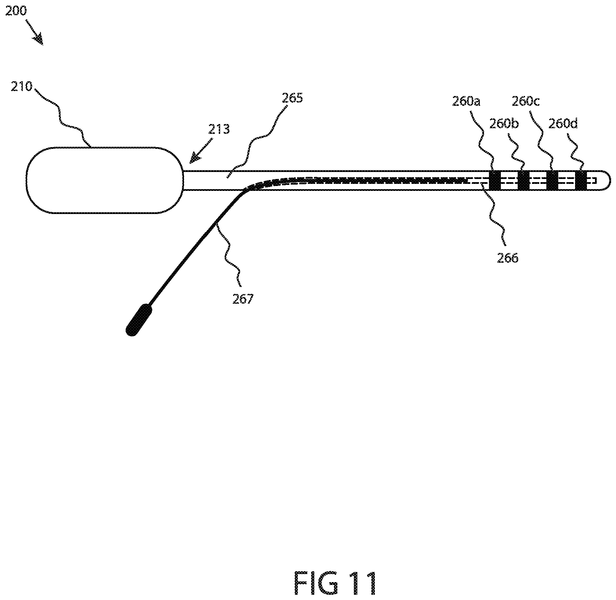

In some embodiments, lead 265 comprises a removable stylet configured to aid in the implantation of lead 265, such as is described herebelow in reference to Fig. 11. In some embodiments, implantable system 20 comprises more than one lead 265, comprising one or more functional elements 260 and attached to one or more housings 210 of one or more implantable devices 200. In some embodiments, one or more leads 265 can be attached to a single housing 210.

In some embodiments, lead 265 comprises a diameter between 1mm and 4mm, such as a diameter between 1mm and 2mm. In some embodiments, lead 265 comprises a length between 3cm and 60cm, such as a length between 6cm and 30cm. One or more leads 265 can include between 2-64 functional elements 260, such as when a lead 265 comprises between 2 and 64 electrodes, such as between 4 and 32 electrodes. In some embodiments, lead 265 can comprise a paddle lead. In some embodiments, lead 265 comprises a single or multi-lumen catheter, such as when an attached implantable device 200 is configured as an agent delivery apparatus as described herein (e.g. a functional element 260 configured as a catheter comprises at least a portion of lead 265).

One or more functional elements 260 (singly or collectively functional element 260) can comprise one or more sensors, transducers and/or other functional elements. In some embodiments, functional elements 260 comprise at least one sensor and/or at least one transducer (e.g. a single functional element 260 or multiple functional elements 260). In some embodiments, functional element 260 comprises a functional element configured to provide a therapy, such as one or more functional elements 260 configured to deliver an agent to tissue (e.g. a needle or catheter), to deliver energy to tissue and/or to otherwise affect tissue. In some embodiments, functional element 260 comprises one or more functional elements 260 configured to record patient information, such as when functional element 260 comprises one or more sensors configured to measure a patient physiologic parameter, as described herebelow. In some embodiments, functional element 260 comprises one or more sensors configured to record an implantable device 200 parameter, also as described herebelow.

One or more functional elements 260 can be positioned on lead 265 as shown in Fig. 1. Alternatively or additionally, one or more functional elements 260 can be positioned on housing 210.

Functional element 260 can comprise one or more functional elements positioned at one or more internal body locations. Functional element 260 can comprise one or more functional elements positioned to interface with (e.g. deliver energy to and/or record a physiologic parameter from) spinal cord tissue, spinal canal tissue, epidural space tissue, and/or nerve tissue. In some embodiments, functional element 260 comprises one or more elements positioned proximate and/or within one or more tissue types and/or locations selected from the group consisting of: one or more nerves; one or more locations along, in and/or proximate to the spinal cord; peripheral nerves of the spinal cord including locations around the back; the tibial nerve (and/or sensory fibers that lead to the tibial nerve); the occipital nerve; the sphenopalatine ganglion; the sacral and/or pudendal nerve; brain tissue, such as the thalamus; baroreceptors in a blood vessel wall, such as in the carotid artery; one or more muscles; the medial nerve; the hypoglossal nerve and/or one or more muscles of the tongue; cardiac tissue; the anal sphincter; the dorsal root ganglion; motor nerves; muscle tissue; the spine; the vagus nerve; the renal nerve; an organ; the heart; the liver; the kidney; an artery; a vein; a bone; and combinations of one or more of these, such as to stimulate and/or record data from the tissue and/or location in which the functional element 260 is positioned proximate to and/or within.

In some embodiments, functional element 260 comprises one or more sensors configured to record data representing a physiologic parameter of the patient. Functional element 260 can comprise one or more sensors selected from the group consisting of: electrode; sensor configured to record electrical activity of tissue; blood glucose sensor; gas sensor; blood gas sensor; ion concentration sensor; oxygen sensor; pressure sensor; blood pressure sensor; heart rate sensor; cardiac output sensor; inflammation sensor; neural activity sensor; neural spike sensor; muscular activity sensor; gastric volume sensor; peristalsis rate sensor; pH sensor; strain gauge; accelerometer; gyroscope; GPS; respiration sensor; respiration rate sensor; flow sensor; viscosity sensor; temperature sensor; magnetic sensor; optical sensor; MEMs sensor; chemical sensor; hormone sensor; impedance sensor; tissue impedance sensor; electrode-tissue interface impedance sensor; body position sensor; body motion sensor; organ motion sensor; physical activity level sensor; perspiration sensor; patient hydration sensor; breath monitoring sensor; sleep monitoring sensor; food intake monitoring sensor; digestion monitoring sensor; urine movement sensor; bowel movement sensor; tremor sensor; pain level sensor; and combinations of one or more of these.

Apparatus 10 and functional element 260 can be configured to record a patient parameter (e.g. patient physiologic and/or patient environment parameter) selected from the group consisting of: blood glucose; blood pressure; EKG; heart rate; cardiac output; oxygen level; pH level; pH of blood; pH of a bodily fluids; tissue temperature; inflammation level; bacteria level; type of bacteria present; gas level; blood gas level; neural activity; neural spikes; neural spike shape; action potential; local field potential (LFP); EEG; muscular activity; gastric volume; peristalsis rate; impedance; tissue impedance; electrode-tissue interface impedance; physical activity level; pain level; body position; body motion; organ motion; respiration rate; respiration level; perspiration rate; sleep level; sleep cycle; digestion state; digestion level; urine production; urine flow; bowel movement; tremor; ion concentration; chemical concentration; hormone level; viscosity of a bodily fluid; patient hydration level; and combinations of one or more of these.

In some embodiments, functional element 260 comprises one or more sensors configured to record data representing a parameter of implantable device 200. In these embodiments, functional element 260 can comprise one or more sensors selected from the group consisting of: an energy sensor; a voltage sensor; a current sensor; a temperature sensor (e.g. a temperature of one or more components of implantable device 200); a contamination detector (e.g. to detect undesired material that has passed through housing 210); an antenna matching and/or mismatching assessment sensor; power transfer sensor; link gain sensor; power use sensor; energy level sensor; energy charge rate sensor; energy discharge rate sensor; impedance sensor; load impedance sensor; instantaneous power usage sensor; average power usage sensor; bit error rate sensor; signal integrity sensor; and combinations of one or more of these. Apparatus 10 can be configured to analyze (e.g. via implantable controller 250, external controller 550 and/or diagnostic assembly 91 described herebelow) the data recorded by functional element 260 to assess one or more of: power transfer; link gain; power use; energy within energy storage assembly 270; performance of energy storage assembly 270; expected life of energy storage assembly 270; discharge rate of energy storage assembly 270; ripple or other variations of energy storage assembly 270; matching of antenna 240 and 540; communication error rate between implantable device 200 and external device 500; integrity of transmission between implantable device 200 and external device 500; and combinations of one or more of these. A functional element 260 can be configured to record temperature, such as when apparatus 10 is configured to deactivate or otherwise modify the performance of an implantable device 500 when the recorded temperature exceeds a threshold.

In some embodiments, one or more functional elements 260 comprise a transducer configured to deliver energy to tissue, such as to treat pain and/or to otherwise stimulate or affect tissue. In some embodiments, functional element 260 comprises a stimulation element, such as one or more transducers selected from the group consisting of: an electrode; an energy delivery element such as an electrical energy delivery element, a light energy delivery element, a laser light energy delivery element, a sound energy delivery element, a subsonic sound energy delivery element and/or an ultrasonic sound delivery element; an electromagnetic field generating element; a magnetic field generating element; a mechanical transducer (e.g. delivering mechanical energy to tissue); a tissue manipulating element; a heat generating element; a cooling (e.g. cryogenic or otherwise heat extracting energy) element; an agent delivery element such as a pharmaceutical drug delivery element; and combinations of one or more of these.

In some embodiments, one or more functional elements 260 comprises a drug or other agent delivery element, such as a needle, port, iontophoretic element, catheter, or other agent delivering element that can be connected to a reservoir of agent positioned within housing 210 (e.g. reservoir 225 described herebelow). In some embodiments, one or more functional elements 260 comprise a drug eluting element configured to improve biocompatibility of implantable system 20.

In some embodiments, one or more functional elements 260 comprise one or more electrodes configured to deliver energy to tissue and/or to sense a patient parameter (e.g. electrical activity of tissue or other patient physiologic parameter). In these embodiments, one or more functional elements 260 can comprise one or more electrodes selected from the group consisting of: microelectrode; cuff electrode; array of electrodes; linear array of electrodes; circular array of electrodes; paddle-shaped array of electrodes; bifurcated electrodes; and combinations of one or more of these.

In some embodiments, apparatus 10 and functional element 260 are configured to both record one or more patient parameters, and also to perform a medical therapy (e.g. stimulation of tissue with energy and/or an agent). In these embodiments, the medical therapy can be performed in a closed-loop fashion, such as when energy and/or agent delivery is modified based on the measured one or more patient physiologic parameters.

In some embodiments, one or more functional elements 260 can comprise one or more electrodes for sensing electrical activity and/or delivering electrical energy. Apparatus 10 can be configured to cause stochastic resonance, and the addition of white noise can enhance the sensitivity of nerves to be stimulated and/or boost weak signals to be recorded by the one or more functional elements 260.

In some embodiments, apparatus 10 and functional element 260 are configured to both record one or more implantable device 200 parameters, and also to perform a medical therapy (e.g. stimulation of tissue with energy and/or an agent). In these embodiments, the medical therapy can be performed in a closed-loop fashion, such as when energy and/or agent delivery is modified based on the measured one or more implantable device 200 parameters.

In some embodiments, one or more functional elements 260 comprise an agent delivery element, such as a fluid delivery element (e.g. a catheter, a porous membrane, an iontophoretic element or a needle) in fluid communication with a reservoir of the agent positioned within housing 210, such as reservoir 225 described herebelow.

In some embodiments, apparatus 10 comprises tool 60. Tool 60 can comprise a data logging and/or analysis tool configured to receive data from external system 50 or implantable system 20, such as data comprising: diagnostic information recorded by external system 50 and/or implantable system 20; therapeutic information recorded by external system 50 and/or implantable system 20; patient information (e.g. patient physiologic information) recorded by implantable system 20; patient environment information recorded by implantable system 20; and combinations of one or more of these. Tool 60 can be configured to receive data from wired or wireless (e.g. Bluetooth) means. Tool 60 can comprise a tool selected from the group consisting of: a data logging and/or storage tool; a data analysis tool; a network such as a LAN or the Internet; a cell phone; and combinations of one or more of these.

In some embodiments, tool 60 comprises a battery charging assembly, such as an assembly configured to recharge one or more power supplies 570 comprising a rechargeable battery or capacitor.

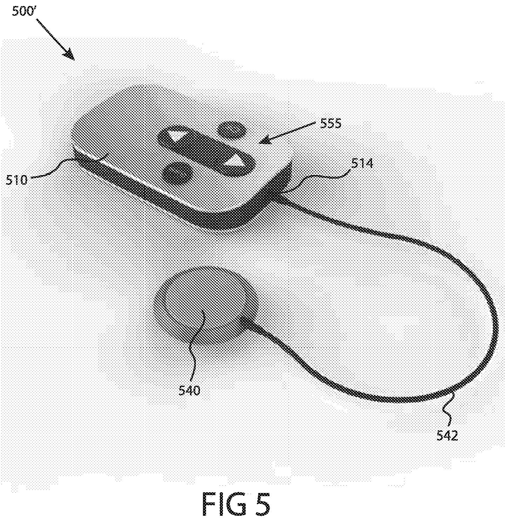

In some embodiments, two or more external system 50 components are connected by a conduit, such as a conduit comprising one or more wires, optical fibers, fluid transport tubes and/or mechanical linkages, such as conduit 542 described herebelow in reference to Fig. 5. Alternatively or additionally, two or more implantable system 20 components are connected by a conduit, such as a conduit comprising one or more wires, optical fibers, fluid transport tube and/or mechanical linkages, such as conduit 242 described herebelow in reference to Fig. 3.

Apparatus 10 can include one or more devices, such as patient attachment device 70 shown in Fig. 1, that is used to attach one or more portions of external system 50 to a location on or proximate the patient. In some embodiments, patient attachment device 70 is constructed and arranged as described in applicant's co-pending United States Provisional Patent Application Serial Number 62/077,181, titled "Method and Apparatus for Implantable Neuromodulation Systems", filed November 8, 2014 (see US 2017/0095667).

Patient attachment device 70 can comprise one or more elements configured to attach one or more external devices 500 at one or more locations on or proximate the patient's skin, that are relatively close to one or more implantable devices 200 that have been implanted in the patient. Patient attachment device 70 can comprise a component selected from the group consisting of: belt; belt with pockets; adhesive; strap; strap with pockets; shoulder strap; shoulder band; shirt; shirt with pockets; clothing; clothing with pockets; epidural electronics packaging; clip; bracelet; wrist band; wrist watch; necklace; and combinations of one or more of these. In some embodiments, patient attachment device 70 comprises a belt configured to surround at least one antenna 540 (e.g. at least one antenna 540 mounted to or otherwise positioned on a printed circuit board such as a flexible printed circuit board). Patient attachment device 70 can include one or more pockets, such as one or more pockets configured to collectively surround one or more of: external device 500; one or more antennas 540; power supply 570; controller 550; and combinations of one or more of these. In some embodiments, patient attachment device 70 comprises multiple pockets, such as to allow repositioning of an external antenna 540, external controller 550, external transmitter 530 and/or external power supply 570 to various different locations, such as to improve transmission of power and/or data to one or more implantable devices 200 and/or improve patient comfort. In some embodiments, one or more antennas 540, power supplies 570, and/or transmitters 530 are connected through flexible cables positioned in patient attachment device 70. In some embodiments, the flexible cables are small coax cables that can accommodate the power levels and frequencies of the carried signals. In some embodiments, the one or more antennas 540 are connected to one or more additional components of external device 500 through a single cable with a local power splitting component and/or active matching element that adjusts signal power to each of the one or more antennas 540.

Apparatus 10 can comprise a device configured to operate (e.g. temporarily operate) one or more implantable devices 200, such as trialing interface 80 shown in Fig. 1. Trialing interface 80 can be configured to deliver power to an implantable device 200, deliver data to an implantable device 200, and/or receive data from an implantable device 200. Trialing interface 80 can be configured to interface with one or more implantable devices 200 during an implantation procedure in which one or more implantable device 200 are implanted in a patient (e.g. a sterile clinical procedure). Trialing interface 80 can be configured to be sterilized one or more times. Trialing interface 80 can comprise one or more antennas, such as an antenna similar to antenna 540 of an external device 500. Trial interface 80 can comprise a transmitter, such as a transmitter similar to transmitter 530 of external device 500, and a power supply, such as a power supply similar to power supply 570 of external device 500. In some embodiments, trialing interface is of similar construction and arrangement to the trialing interface described in applicant's co-pending United States Provisional Patent Application Serial Number 62/077,181, titled "Method and Apparatus for Implantable Neuromodulation Systems", filed November 8, 2014 (see US 2017/0095667). In some embodiments, trialing interface 80 includes a housing to be positioned proximate at least a portion of implantable device 200, such as a housing that surrounds an antenna and a transmitter that is configured to operatively couple to (e.g. transmit power and/or data to) one or more antennas 240 of one or more implantable devices 200.

In some embodiments, one or more implantable devices 200 of implantable system 20 can comprise an implantable transmitter configured to transmit data, such as to transmit data (e.g. stimulation information, patient physiologic information, patient environment information, implantable device 200 performance and/or configuration information, and the like) to one or more external devices 500. In these embodiments, receiver 230 can be configured as both a receiver and a transmitter. One or more implantable devices 200 can be configured to transmit data by sending a signal to (i.e. "driving") one or more antennas 240 or another antenna of implantable device 200. An implantable device 200 can be configured to transmit data using one or more of: load modulation; a signal carrier; and/or body conduction. An implantable device 200 can be configured to adjust the transmission, such as to adjust a data transmission parameter selected from the group consisting of: data rate; pulse width; duration of carrier signal; amplitude of carrier signal; frequency of carrier signal; configurable load; and combinations of one or more of these.

In some embodiments, apparatus 10 comprises a diagnostic assembly, diagnostic assembly 91 shown in Fig. 1. In some embodiments, controller 550 and/or implantable controller 250 comprise all or a portion of diagnostic assembly 91. Diagnostic assembly 91 can be configured to assess, monitor, determine and/or otherwise analyze patient information and/or implantable device 200 information, such as when one or more functional elements 260 and/or 560 are configured as a sensor configured to record patient information (e.g. patient physiologic information and/or patient environment information) and/or apparatus 10 information (e.g. implantable device 200 information) as described hereabove. Diagnostic assembly 91 can be configured to analyze communication and/or the power link between an implantable device 200 and an external device 500. In some embodiments, such a communication link analysis can be performed by measuring bit error rate (BER) of a known data stream during communication signal transmission (also referred to as "communication link") measurement phase (e.g. such as during a calibration procedure). The BER can be tracked by the implant controller 250 or external controller 550, such as to monitor and keep track of any trends in the link. This trend can be used to adjust the link and/or provide feedback to an operator of apparatus 10 (e.g. the patient), in case the link cannot be automatically adjusted to compensate for a negative trend (e.g. such that the operator can perform physical re-adjustment of the external system 50). Alternatively or additionally, a power link analysis can be performed by monitoring charge/discharge rate of the implanted energy storage assembly 270. Similar to the communication link, the power link status and/or trending can be monitored and recorded for link adjustment and/or feedback purposes. Diagnostic assembly 91 can be configured to analyze a result of stimulation energy delivered by implantable device 200, such as when a functional element 260 comprises an electrode to record electrical activity of tissue (e.g. in addition to delivering electrical energy to stimulate tissue). A functional element 260 and/or 560 can comprise a sensor configured to record neural activity and/or muscular activity, and the diagnostic assembly configured to analyze the recorded sensor data. In some embodiments, diagnostic assembly 91 can be configured to analyze impedance, such as when a functional element 260 and/or 560 comprises a sensor configured to record data related to impedance, such as when implantable device 200 performs a frequency sweep, performs an impulse response and/or compares voltage and current of a stimulation waveform.

In some embodiments, apparatus 10 is configured to provide a therapy by delivering stimulation energy to tissue, such as electrical energy delivered to tissue by one or more functional elements 260 comprising one or more electrodes. Alternatively or additionally, apparatus 10 can be configured as an agent-delivery apparatus (e.g. a pharmaceutical or other agent delivery apparatus). In some embodiments, apparatus 10 comprises one or more reservoirs for storing the agent, such as reservoir 525 of external device 500 and/or reservoir 225 of implantable device 200, each shown in Fig. 1. Reservoirs 525 and/or 225 can be fluidly connected to one or more functional elements 560 and/or 260, respectively (e.g. via one or more tubes). Reservoirs 525 and/or 225 can comprise one or more chambers (e.g. independent chambers configured to separately contain incompatible drugs or otherwise prevent undesired multiple drug interactions). Reservoirs 525 and/or 225 can comprise a volume (e.g. a volume to store one or more agents) between 0.1ml and 50ml, such as between 0.1 ml and 3.0ml, or between 0. 1ml and 1.0ml. Reservoirs 525 and/or 225 can comprise pressurized reservoirs or otherwise comprise a fluid pumping mechanism (e.g. a peristaltic mechanism, syringe pump or other fluid pump). Reservoirs 525 and/or 225 and can comprise refillable reservoirs (e.g. when reservoir 225 of an implantable device 200 comprises a valved opening such as a silicone septum or a mechanical valve, either accessible via a needle for refilling). The fluidly attached functional elements 560 and/or 260 can comprise a fluid delivery element selected from the group consisting of: a catheter; a porous membrane; an iontophoretic element; a needle; or combinations of one or more of these. Delivered and/or stored (e.g. in a reservoir) agents can comprise an agent selected from the group consisting of: an analgesic agent such as morphine, fentanyl, lidocaine or other agent delivered to treat pain; a chemotherapeutic agent such as a chemotherapeutic agent delivered systemically and/or to a location in or proximate an organ such as the liver or brain to treat cancer; an antibiotic configured to treat or prevent an infection; a hormone such as a hormone delivered intravenously in hormonal therapy; heart medications such as nitroglycerin, a beta blocker or a blood pressure lowering medication; a carbohydrate such as glucose or dextrose delivered to treat a low blood sugar condition; insulin such as to treat a high blood sugar condition; a diabetic medication; a neurological medication; an epilepsy medication; and combinations of one or more of these. In some embodiments, apparatus 10 comprises the one or more agents stored in reservoir 225 and/or 525. In some embodiments, apparatus 10 is constructed and arranged to deliver the agent (e.g. via a catheter-based functional element 560 and/or 260) to a patient location selected from the group consisting of: a vessel; a blood vessel; a vein; an artery; heart; brain; liver; spine; epidural space; intrathecal space; subcutaneous tissue; bone; and combinations of one or more of these.

In some embodiments, an external device 500 is attached to the patient via a patient attachment device 70 comprising a wrist band, wrist watch or other band configured to position an external device 500 about a limb of the patient (i.e. arm or leg of the patient), such as is described herebelow in reference to Figs. 13, 13A and 13B. In these embodiments, one or more implantable devices 200 are implanted under the skin proximate the intended (limb) location of external device 500 and patient attachment device 70. Apparatus 10 is configured such that external device 500 comprises one or more antennas 540; one or more implantable devices 200 each comprise one or more antennas 240; and each implantable device 200 one or more antennas 240 receive power and/or data from the one or more antennas 540 of the limb-attached external device 500. The limb-attached external device 500 can comprise one or more reservoirs 525 described hereabove and/or one or more functional elements 560 configured as agent delivery elements and/or sensors. The one or more implantable devices 200 can comprise one or more reservoirs 225 described hereabove and/or one or more functional elements 260 configured as agent delivery elements and/or sensors.

In some embodiments, apparatus 10 comprises an agent delivery apparatus and agent is delivered into the patient (e.g. into a blood vessel, muscle or subcutaneous tissue) by an external device 500 functional element 560 (e.g. a needle) based on signals recorded by an implantable device 200 functional element 260 (e.g. a sensor). Alternatively or additionally, agent can be delivered into the patient (e.g. into a blood vessel, muscle or subcutaneous tissue) by an implantable device 500 functional element 260 (e.g. a needle, catheter, porous membrane or iontophoretic delivery element). The amount of agent delivered by functional element 260 can be based on signals recorded by an implantable device 200 functional element 260 (e.g. a sensor) and/or an external device 500 functional element 560 (e.g. a sensor). External device 500 can provide power to one or more implantable devices 200 and/or it can send data (e.g. sensor data from a functional element 560) to implantable device 500, such as to control agent delivery by implantable device 500.

Apparatus 10 can be configured to prevent an electromagnetic field (e.g. an electromagnetic field produced by one or more devices not included in apparatus 10 and/or other present in the patient environment) from adversely affecting and/or otherwise affecting the patient treatment and/or patient information recording (e.g. patient tissue stimulation and/or patient physiologic information gathering) performed by apparatus 10. Electromagnetic fields from one or more apparatus 10 devices and/or otherwise present in the patient environment can potentially interfere with apparatus 10. The architecture of the wireless signal transmissions of apparatus 10 can be configured to include certain unique and/or identifiable patterns in the signals transmitted by apparatus 10 to confirm (upon receipt) that the signal originated from a component of apparatus 10. Alternatively or additionally, the stimulation signal produced by an implantable device 200 can be created independent from a power signal received from an external device 500, so that any electromagnetic interference in the wireless link does not affect generation and delivery of the stimulation signal. In some embodiments, each implantable device 200 and/or external device 500 includes unique identification codes that are required to be transmitted prior to any changes in stimulation or other implantable device 200 configuration, ensuring correct operation in the presence of interference. Alternatively or additionally, the communication link can incorporate handshaking protocols, confirmation protocols, data encryption and/or scrambling, coding and other security measures to ensure that interfering signals do not adversely affect the implantable system 20 performance (e.g. stimulation). In some embodiments, external system 50 and/or implantable system 20 can incorporate electromagnetic absorptive and/or reflective materials to minimize external interference from other sources and/or minimize the probability of apparatus 10 interfering with other systems. Alternatively or additionally, apparatus 10 can incorporate error detection and protocols for entering an alarm state (e.g. and shutting down normal operation) and/or otherwise ensuring safe operation.

**Referring now to** **Fig. 2****,** a schematic anatomical view of an apparatus for treating and/or diagnosing a patient comprising multiple implantable devices is illustrated, consistent with the present inventive concepts. Apparatus 10 comprises implantable system 20 and external system 50. Implantable system 20 can comprise two or more implantable devices, such as implantable devices 200a and 200b, up to 200n (singly or collectively implantable device 200) shown in Fig. 2. Each implantable device 200 is shown implanted beneath the skin of patient P. External system 50 can comprise one or more external devices 500, such as external devices 500a, 500b up to 500n (singly or collectively external device 500) shown in Fig. 2. Apparatus 10 of Fig. 2 can comprise tool 60, patient attachment device 70, trialing interface 80 and/or diagnostic assembly 91, not shown but such as is described hereabove in reference to Fig. 1.

Each external device 500 is configured to transmit power and/or data to one or more implantable devices 200. In some embodiments, one or more external devices 500 are configured to transmit both power and data (e.g. simultaneously and/or sequentially) to one or more implantable devices 200. In some embodiments, one or more external devices 500 are further configured to receive data from one or more implantable devices 200. Each external device 500 can comprise housing 510, power supply 570, transmitter 530 and/or antenna 540, any or all of which can be of similar construction and arrangement to the similar components of external device 500 described hereabove in reference to Fig. 1.

External system 50 can further comprise controller 550, which can comprise a user interface, such as user interface 555 and can be of similar construction and arrangement to controller 550 described hereabove in reference to Fig. 1. Controller 550 is configured to control one or more external devices 500, such as external devices 500a, 500b through 500n shown in Fig. 2. Controller 550 can send commands to an external device 500 via a wireless and/or wired connection (wired connection not shown but such as a connection comprising one or more insulated conductive wires). In some embodiments, one or more external devices 500 comprise controller 550, such as when user interface 555 is integrated into a housing 510 of an external device 500.

In some embodiments, a first external device 500a is positioned proximate a first implantable device 200a, and a second external device 500b is positioned proximate a second implantable device 200b, as shown in Fig. 2. In some embodiments, one or more external devices define a radiation footprint, such as is described herebelow in reference to Figs. 8, 9 or 10. The radiation footprint can be expanded by incorporating an array of antennas 540 into external system 50 and/or an array of antennas 240 into implantable system 20. External system 50 can activate one or more antennas 540 within the array based on power link and/or data link monitoring information that controller 550 receives from one or more implantable devices 200. The acceptable range of depths between external antenna 540 and implantable antenna 240 can vary (e.g. vary between applications and/or patient geometry), such as an acceptable depth range between 0.3cm and 7cm, between 0.5cm and 5cm, or between 1cm and 3cm. Lateral and/or angular misalignment can be compensated for by utilizing controllable polarizations and activation of one or more antennas 540 in an antenna array and/or by using orthogonal implantable antennas 240. Alternatively or additionally, two or more implantable antennas 240 can be oriented in different planes with respect to each other and/or implantable antennas 240 can comprise combinations of dipole and loop antennas. In some embodiments, lateral misalignment tolerance can be between 0.1 cm and 10 cm, such as between 0.1 cm and 5 cm, or between 0.1 cm and 3 cm.

Each implantable device 200 is configured to receive power and/or data from one or more external devices 500. In some embodiments, one or more implantable devices 200 are configured to receive both power and data (e.g. simultaneously and/or sequentially) from one or more external devices 500. In some embodiments, a single external device 500 sends power and/or data to multiple implantable devices 200. Alternatively or additionally, two or more external devices 500 can send power and/or data to a single implantable device 200.

In some embodiments, one or more implantable devices 200 are further configured to transmit data to one or more external devices 500. Each implantable device 200 can comprise housing 210, energy storage assembly 270, receiver 230, demodulator 231, rectifier 232, power converter 233, antenna 240, controller 250 and/or lead 265, any or all of which can be of similar construction and arrangement to the similar components of implantable device 200 described hereabove in reference to Fig. 1.

Each lead 265a, 265b through 265n (singly or collectively lead 265) can each comprise one or more functional elements 260, such as functional element 260a, 260b through 260n, respectively. Each functional element 260 can comprise one or more functional elements, such as one or more functional elements 260 described hereabove in reference to Fig. 1. Each functional element 260 can comprise a sensor, a transducer and/or other functional elements. In some embodiments, one or more functional elements 260 comprise a sensor such as a sensor configured to record data representing a patient parameter or an implantable device 200 parameter. In some embodiments, one or more functional elements 260 comprise an electrode or other element configured to deliver energy to tissue, such as to treat pain and/or to stimulate tissue.

In some embodiments, one or more components of external system 50 (e.g. one or more power supplies 570, antennas 540, and the like) can comprise swappable, replaceable, and/or position-adjustable components. One or more portions of external system 50 can be positioned on, in and/or within an elastic belt with multiple pockets to surround one or more components of the external system 50 (e.g. for patient comfort and/or ease of use). The patient can choose the placement of the supported components depending on physical activity and/or comfort preference (e.g. patients who prefer sleeping on their back can choose to position a power supply 570 in a pocket on a side or front body position of the belt). Power supply 570 (e.g. a rechargeable battery) can be disconnected and replaced with a different power supply 570. The discharged battery can be placed in a recharging dock (e.g. a tool 60 described hereabove in reference to Fig. 1 configured as a power supply 570 recharging assembly). Swappable power supplies 570 can be beneficial for patients, such as by avoiding a recharge protocol which requires spending time by a recharge unit and/or carrying a recharge unit with them.

In some embodiments, a flexible cable, such as a flexible coaxial cable of thin diameter (for comfort of a patient), can be used to connect one or more antennas 540 to external transmitter 530. Alternatively or additionally, one or more flexible or semi-rigid cables can be used for low frequency control or DC connections, such as between a swappable and/or position-adjustable battery 570, external transmitter 530, and/or one or more external controllers 550.

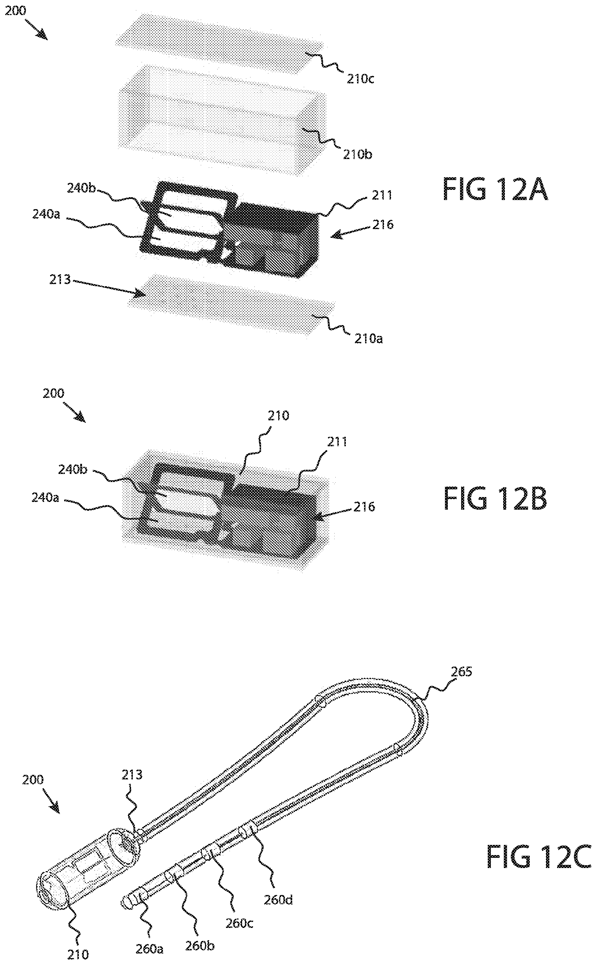

**Referring now to** **Fig. 3****,** a perspective view of an implantable device comprising an antenna extension is illustrated, consistent with the present inventive concepts. Implantable device 200 comprises housing 210, antenna 240 and lead 265. Housing 210, lead 265 and/or one or more other components of implantable device 200 can be of similar construction and arrangement to the similar components of implantable device 200 described hereabove in reference to Figs. 1 or 2. Housing 210 can comprise a rectangular prism-shaped construction, such as is shown in Fig. 3. Housing 210 comprises major axis Aₘₐⱼ, and minor axis Aₘᵢₙ, each as shown. Major axis Aₘₐⱼ can comprise a length less than or equal to 20mm, such as less than or equal to 12mm or 10mm. Minor axis Amin can comprise a length less than or equal to 8mm, such as less than or equal to 6mm. Antenna 240 is positioned outside of housing 210, such as an antenna 240 operably attached to housing 210 via a flexible extension, conduit 242, for example one or more conductors surrounded by insulation and passing through housing 210 at feedthrough 214. Conduit 242 can comprise a length between 0.5cm and 60cm, such as a length between 0.5cm and 10cm, or between 0.5cm and 5cm. In some embodiments, housing 210, conduit 242, antenna 240, and lead 265 can be contained within a single enclosure. In some embodiments, conduit 242, antenna 240, and lead 265 can be connected through feedthroughs 213 and 214 in housing 210. One or more portions of housing 210 (e.g. as described herebelow in reference to Fig. 12A), as well as feedthroughs 213 and/or 14, can be constructed and arranged to provide a sealed environment within housing 210.

Lead 265 passes through housing 210 at feedthrough 213. In some embodiments, a covering such as a silicone cover (e.g. a silicone overmold) covers all or a portion of housing 210 and/or feedthroughs 213 and/or 214. Alternatively or additionally, a sealing agent can comprise an adhesive (e.g. epoxy) placed to seal one or more housing 210 seams or feedthroughs 213 and/or 214 locations.

Lead 265 comprises an implantable lead including multiple functional elements 260, such as the four functional elements shown, functional elements 260a, 260b, 260c and 260d. Functional elements 260 can comprise a sensor, transducer or other functional element, such as functional element 260 described hereabove in reference to Fig. 1. In some embodiments, functional elements 260a-d comprise two or more electrodes configured to provide bipolar electrical stimulation of tissue, such as to stimulate tissue (e.g. nerve tissue) at the implantation site of functional elements 260a-d, such as to treat pain or provide another medical therapy. In some embodiments, one or more portions of housing 210 are configured as an electrode, such as to deliver monopolar electrical stimulation via one or more electrode-based functional elements 260 of lead 265.

**Referring now to** **Fig. 4****,** a perspective view of an implantable device comprising a housing surrounding a printed circuit board is illustrated, consistent with the present inventive concepts. Implantable device 200 comprises housing 210, antenna 240 and lead 265. Housing 210, antenna 240, lead 265 and/or one or more other components of implantable device 200 can be of similar construction and arrangement to the similar components of implantable device 200 described hereabove in reference to Figs. 1 or 2. In the embodiment of Fig. 4, housing 210 has been illustrated as transparent to visualize components internal to housing 210. Housing 210 can comprise a pill-shaped construction, such as is shown in Fig. 4. Housing 210 comprises major axis Aₘₐⱼ, and minor axis Aₘᵢₙ, each as shown. Major axis Aₘₐⱼ can comprise a length less than or equal to 15mm, such as less than or equal to 12mm or 10mm. Minor axis Aₘᵢₙ can comprise a length less than or equal to 8mm, such as less than or equal to 6mm. Antenna 240 is positioned within housing 210, such as an antenna 240 mounted to or otherwise positioned on a substrate 211 (e.g. a printed circuit board or other substrate). Lead 265 passes through housing 210 at feedthrough 213. Feedthrough 213 can be constructed and arranged similar to feedthrough 213 described hereabove in reference to Fig. 3. Lead 265 is operably attached (e.g. electrically attached) to substrate 211 and comprises an implantable lead including multiple functional elements 260a-d, each of which can be of similar construction and arrangement to the similar components described hereabove in reference to Figs. 1, 2 or 3.

Substrate 211 further comprises implantable components 216, which can include electronic or other components configured to provide a function selected from the group consisting of: energy storage, such as is provided by energy storage assembly 270 described hereabove in reference to Figs. 1 or 2; reception of power and/or data, such as is provided by receiver 230 described hereabove in reference to Figs. 1 or 2; demodulation, such as is provided by demodulator 231 described hereabove in reference to Fig. 1; rectification, such as is provided by rectifier 232 described hereabove in reference to Fig. 1; power conversion, such as is provided by power converter 233 described hereabove in reference to Fig. 1; charge balance, such as is described hereabove in reference to Fig. 1; and combinations of one or more of these. In some embodiments, components 216 comprise one or more elements selected from the group consisting of: digital circuitry; a digital component; analog circuitry; an analog component; a programmable gate array; a field programmable gate array; a digital to analog converter; an analog to digital converter; a microcontroller; a microprocessor; a multiplexor; an energy storage element; a capacitor, an inductor; and combinations of one or more of these.

**Referring now to** **Fig. 5****,** an external device comprising a tethered antenna and an integral user interface is illustrated, consistent with the present inventive concepts. External device 500' is configured to transmit power and/or data to one or more implantable devices, such as one or more implantable devices 200 described herein. In some embodiments, external device 500' is configured to transmit both power and data (e.g. simultaneously and/or sequentially) to one or more implantable devices 200. In some embodiments, external device 500' is configured to receive data from one or more implantable devices 200. External device 500' comprises housing 510, user interface 555, and antenna 540, any or all of which can be of similar construction and arrangement to the similar components of external device 500 described hereabove in reference to Figs. 1 or 2.

Antenna 540 is operably attached to a first end of conduit 542, for example a flexible conduit comprising one or more conductors surrounded by insulation and passing through housing 510 at feedthrough 514. A second end of conduit 542 operably connects to one or more components of external device 500' positioned within housing 510 (components not shown but such as those described hereabove in reference to external device 500' described hereabove in reference to Figs. 1 or 2). In some embodiments, conduit 542 comprises a length between 2.0 inches and 60.0 inches, and comprises between 1 and 16 insulation-separated wires. Housing 510 and/or antenna 540 can comprise adhesive on a surface, such as to adhesively attach housing 510 and/or antenna 540, respectively, to the patient's skin. Alternatively, a separate fixation device can be included (e.g. patient attachment device of Fig. 1), such as to strap or otherwise secure housing 510 and/or antenna 540 to the patient. Antenna 540 can comprise an array of antennas, any or all of which can be positioned on a flexible material and/or contained in a flexible enclosure.

External device 500' can comprise a controller, or a portion of a controller, not shown but such as a controller of similar construction and arrangement to controller 550 described hereabove in reference to Figs. 1 or 2. The controller can include a user interface, such as user interface 555 shown positioned on an outer surface of housing 510. User interface 555 can include one or more user input components (e.g. switches) and/or user output components (e.g. a light or a display), as described in detail hereabove. In some embodiments, external device 500' and/or user interface 555 are configured to control one or more separate external devices 500.

**Referring now to** **Figs. 6A, 6B and 6C****,** in Fig. 6A a top view of an implantable substrate in an unfolded state and comprising multiple antennas is illustrated; in Fig. 6B, a perspective view of the implantable substrate of Fig. 6A, in a folded state, is illustrated; and in Fig. 6C, an end view of an implantable device including the implantable substrate of Fig. 6B positioned within a housing is illustrated; all consistent with the present inventive concepts. Substrate 211 and one or more components attached to substrate 211 can be constructed and arranged to be positioned within a housing of an implantable device of the present inventive concepts, such as housing 210 and implantable device 200 describe hereabove in reference to Figs. 1, 2, 3 or 4. An implantable receiver, receiver 230 and an implantable controller, controller 250, have been positioned on substrate 211. Receiver 230, controller 250 and one or more other components positioned on substrate 211, can be of similar construction and arrangement as similar components described hereabove in reference to Fig. 1.

Substrate 211 of Figs. 6A-C comprises portions 211a, 211b and 211c shown. Three antennas, antennas 240a, 240b and 240c (singly or collectively antenna 240), have been positioned on portion 211a. Antennas 240 can be arranged in the triangular geometry shown in Figs. 6B and 6C. In some embodiments, antennas 240 are arranged in an X-shaped pattern herebelow shown in Figs. 12A-C. In some embodiments, substrate 211a containing antennas 240 comprises rotatable portions (e.g. hinged portions), bendable portions, flexible material and/or is otherwise constructed to allow antennas 240 to transition from the single plane arrangement shown in Fig. 6A, to the multi-planar arrangement shown in Figs. 6B and 6C (e.g. to rotate about axes A1 and A2 and reside in three planes oriented 60° relative to each other).

Antennas 240 are operably (e.g. electrically) connected to controller 250 and receiver 230 via implantable wires or other conduits, conduits 215. Conduits 215 can comprise one or more electrical traces of substrate 211, such as when substrate 211 comprises a printed circuit board such as a flexible printed circuit board (e.g. a printed circuit board comprising one or more flexible portions).

The antenna 240 arrangements shown in Figs. 6B and 6C, and 12A and 12B, can allow the antennas 240 to capture different polarizations of electromagnetic energy transmitted by external system 50. In some embodiments, the antennas 240 have a major axis between 4mm and 8mm, and a minor axis between 3mm and 6mm. In some embodiments, antennas 240 comprise one or more dipole antennas, such as one or more antennas 240 separately connected through one or more conduits 215. In some embodiments, the two or more antennas 240 can be positioned on planes oriented between 30° and 90° relative to each other, such as two antennas 240 positioned on orthogonally oriented planes. In some embodiments, the two or more antennas 240 are oriented three different planes.

Substrate 211 can be constructed and arranged such that substrate 211c rotates about axis A3. In Figs. 6B and 6C, substrate 211c has been rotated under substrate 211b, such as to fit within housing 210 as shown in Fig. 6C. Substrate 211 comprises multiple pins, wires, vias, pads, or other connecting elements (e.g. electrical connecting elements), connectors 212 (shown positioned on substrate portion 211c). Connectors 212 are constructed and arranged to mate or otherwise align with a corresponding set of holes of housing 210, feedthroughs 213 as shown in Fig. 6C. In some embodiments, one or more extending wires or other extending conduits of a lead, such as lead 265 described herein, pass through feedthroughs 213 and connect to connectors 212. In some embodiments, feedthroughs 213 are encased in a sealing agent, such as a silicone covering (e.g. a silicone overmold) and/or an adhesive such as an epoxy material is included to form a seal.

Implantable device 200 of Fig. 6C can comprise an atraumatic or other implantable covering, such as covering 218 shown. Covering 218 can comprise an elastomeric material positioned around all or a portion of housing 210. Implantable device 200 (e.g. implantable housing 210) comprises a major axis (e.g. major axis Aₘₐⱼ as described hereabove in reference to Figs. 3 or 4) and minor axis Aₘᵢₙ, as shown. Major axis Aₘₐⱼ can comprise a length less than or equal to 20mm, such as less than or equal to 15mm, 12mm or 10mm. Minor axis Aₘᵢₙ can comprise a length less than or equal to 8mm, such as a length less than or equal to 6mm or 5mm.

In some embodiments, implantable device 200 comprises a lead comprising one or more functional elements 260 (e.g. one or more electrodes or other sensors and/or transducers as described herein), not shown but such as lead 265 described hereabove in reference to Figs. 1, 2, 3, 4 or 10 which passes through housing 210 and operably connects (e.g. electrically, fluidly, optically and/or mechanically) to one or more of components 216.

**Referring now to** Figs. **7A and** 7B, in Fig. 7A an end view of an implantable substrate in an unfolded state and comprising multiple components is illustrated; in Fig. 7B, an end sectional view of an implantable device comprising the implantable substrate of Fig. 7A, in a folded state and positioned within an implantable housing is illustrated; all consistent with the present inventive concepts. Substrate 211 and one or more components attached to substrate 211 can be constructed and arranged to be positioned within a housing of an implantable device of the present inventive concepts, such as housing 210 and implantable device 200 describe hereabove in reference to Figs. 1 or 2. Components 216a-e (singly or collectively components 216) have been positioned on substrate 211 as shown.

Multiple implantable components 216, such as components 216a-e can be positioned at one or more locations upon one or more surfaces of substrate 211 (e.g. either top or bottom surfaces of substrate 211, at any location) such as to minimize the volume of substrate 211 and all its components 216 when substrate 211 is folded, such as to allow substrate 211 and attached components 216 to be positioned within a smaller housing 210 as shown in Fig. 7B. For example, components 216d and 216e have been separated and otherwise positioned on an unfolded substrate 211 (as shown in Fig. 7A) such that when substrate 211 is folded (as shown in Fig. 7 B), components 216d and 216e are in a compact side-by-side arrangement.

As described hereabove in reference to Figs. 6A-C, substrate 211 can comprise a printed circuit board, such as a flexible printed circuit board comprising traces or other electrical conduits (not shown but such as conduits 215 described hereabove in reference to Figs. 6A-C) electrically connecting one or more components 216. In some embodiments, substrate 211 comprises rotatable portions, bendable portions, flexible material and/or is otherwise constructed to allow substrate 211 to transition from the single plane arrangement shown in Fig. 7A, to the multi-planar arrangement shown in Figs. 7B.

Substrate 211 comprises multiple pins, wires, vias, pads, or other connecting elements (e.g. electrical connecting elements), not shown but such as connectors 212 described hereabove in reference to Figs. 6A-C. These connectors can be constructed and arranged to mate or otherwise allow connection with an external conduit of wires, such as lead 265 described herein.

Components 216 can be configured to function as one or more assemblies of implantable device 200, such as receiver 230, controller 250, functional element 260 and/or energy storage assembly 270 as described hereabove in reference to Fig. 1. In some embodiments, implantable device 200 comprises a lead comprising one or more functional elements 260 (e.g. one or more electrodes or other sensors and/or transducers as described herein), not shown but such as lead 265 described hereabove in reference to Figs. 1, 2, 3, 4 or 10 which passes through housing 210 and operably connects (e.g. electrically, fluidly, optically and/or mechanically) to one or more of components 216.

**Referring now to** **Fig. 8****,** an anatomical top view of a medical apparatus comprising an external antenna positioned relative to multiple implantable devices that have been implanted in a patient is illustrated, consistent with the present inventive concepts. Multiple implantable devices 200, such as implantable devices 200a, 200b, 200c and 200d shown, can be implanted in a patient, beneath the surface Sp of the patient's skin. An external antenna, antenna 540 can be positioned proximate the surface of the patient's skin (e.g. adhesively attached to the patient's skin or held in place with a securing device as describe herein). Antenna 540 and implantable devices 200 can be of similar construction and arrangement to antenna 540 and implantable device 200, respectively, described hereabove in reference to Figs. 1 or 2. Antenna 540 can comprise one or more antennas 540, which can be positioned in one or more external devices 500 (e.g. one or more antennas 540 in a single external device 500, or multiple external devices 500 each containing one or more antennas 540). Each antenna 540 can be positioned within a housing 510 of an external device 500.

One or more antennas 540 used to transmit power and/or data to one or more implantable devices 200 define a radiation footprint area F₁, such that a projection from area F₁ into the patient defines a set of acceptable locations for implantation of the one or more implantable devices 200. A projection from area F₁ can comprise a projection whose cross sectional area changes with depth into the patient. For example, with the radiation footprint area F₁ shown, implantable devices 200a, 200b and 200c will reliably receive power and/or data from antenna 540, and implantable device 200d may or may not receive sufficient power transfer and/or reliable data transfer. Each antenna 540 is positioned such that an associated implantable device 200 receives sufficient power to operate. Each antenna 540 provides RF energy that decays with the depth in tissue beneath its physical footprint (i.e. the outer dimensions of the antenna 540). Depending on the design of each antenna 540, the radiation footprint can be different than the physical footprint of the one or more antennas 540 delivering the power. The radiation footprint is specified as the cross-sectional area at a given depth at which the energy density is sufficient to power the implanted implantable devices 200. The projection of acceptable locations for implanted implantable devices 200 comprises a volume enclosed by the radiation footprint and the depth at which the implanted implantable device 200 receives a minimum energy to operate. Electromagnetic simulations can be used to determine precise dimensions of the radiation footprint for one or more specific external antennas 540 design and transmission frequency, and the one or more external antennas 540 can be optimized to increase the size of this radiation footprint. Also, the radiation footprint of an array of external antennas 540 can comprise the superposition of the radiation footprint of each external antenna 540 individually. Therefore, using an array of external antennas 540 expands the projection of acceptable implant locations for each implantable device 200 inside the body, which desensitizes placement of each external antenna 540 on the surface of the skin and simplifies use by the patient or other user of apparatus 10.

In some embodiments, one or more antennas 540 used to transfer power and/or data to one or more implantable devices 200, comprise a periphery beyond and/or area greater than area F₁ as shown. In other embodiments, antenna 540 comprises a periphery within and/or an area less than area F₁. In some embodiments, a known or calculated radiation footprint area F₁ is used by a clinician in selecting implant locations for one or more implantable devices 200 (e.g. the location of one or more antennas 240 of the implantable devices 200). Alternatively or additionally, a known or calculated radiation footprint area F₁ is used by an operator of system 10 (e.g. the patient, a family member, nurse, clinician or healthcare provider) to position one or more antennas 540 relative to one or more already implanted implantable devices 200. In some embodiments, an array of antennas 540 are fixed relative to each other (e.g. in a flexible package), and collectively define a radiation footprint area F₁ as described hereabove.

**Referring now to** **Fig. 9****,** an anatomical view of a medical apparatus comprising an external antenna comprising multiple concentric loops and positioned relative to multiple implantable devices that have been implanted in a patient is illustrated, consistent with the present inventive concepts. Multiple implantable devices 200, such as implantable devices 200a, 200b, 200c and 200d shown, can be implanted in a patient, beneath the surface Sp of the patient's skin. Implantable devices 200 can be of similar construction and arrangement to antenna 540 and implantable device 200, respectively, described hereabove in reference to Figs. 1 or 2. Similar to antenna 540 described hereabove in reference to Fig. 8, an antenna 540 can be positioned proximate the surface of the patient's skin (e.g. adhesively attached to the patient's skin or held in place with a securing device as describe herein). In the embodiment of Fig. 9, antenna 540 comprises a multiple concentric loop antenna, such as an antenna with four concentric loops as shown.

Antenna 540 defines a radiation footprint area F₁, such that a projection from area F₁ into the patient defines a set of acceptable locations for implantation of one or more implantable devices 200. A projection from area F₁ can comprise a cross sectional area that changes with depth into the patient. For example, with the radiation footprint area F₁ shown, implantable devices 200a, 200b and 200c will reliably receive power and/or data from antenna 540.

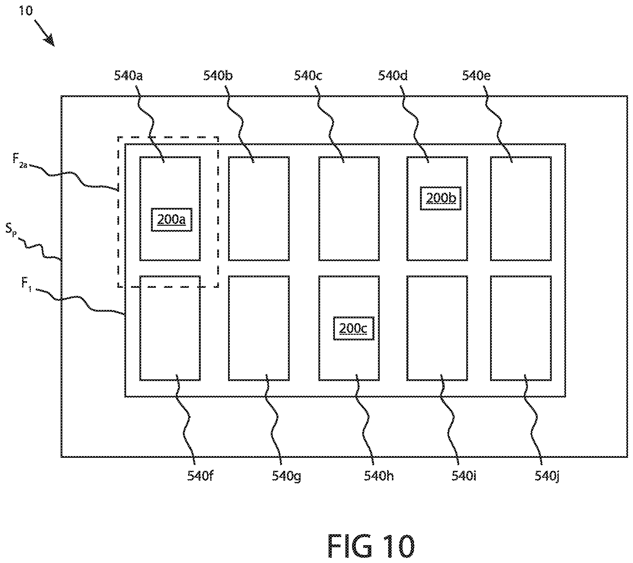

**Referring now to** **Fig. 10****,** an anatomical view of a medical apparatus comprising an external antenna comprising an array of antennas and positioned relative to multiple implantable devices that have been implanted in a patient is illustrated, consistent with the present inventive concepts. Multiple implantable devices 200, such as the three implantable devices 200 shown, can be implanted in a patient, beneath the surface Sp of the patient's skin. An array of antennas 540 (ten shown) can be positioned proximate the surface of the patient's skin (e.g. adhesively attached to the patient's skin or held in place with a securing device as describe herein). Antennas 540 and implantable devices 200 can be of similar construction and arrangement to antenna 540 and implantable device 200, respectively, described hereabove in reference to Figs. 1 or 2.

Each antenna 540 defines a radiation footprint area F₂ (only F₂ₐ, shown in Fig. 10, the radiation footprint for antenna 540a), such that a projection from area F₁ into the patient defines a set of acceptable locations for implantation of one or more implantable devices 200. A projection from area F₁ can comprise a cross sectional area that changes with depth into the patient. In this embodiment, the external system of the present inventive concepts can be configured such that only the antennas 540 positioned above an implantable device 200, are activated (e.g. to transmit power and/or data to the associated implantable device 200 beneath), such as antennas 540a, 540d and 540h shown, while antennas 540b, 540c, 540e, 540f, 540g, 540i, 540j remain inactive during use of the external system (e.g. to optimize use of energy).

**Referring now to** **Fig. 11****,** a side view of an implantable device comprising a lead with a removable stylet is illustrated, consistent with the present inventive concepts. Implantable device 200 comprises housing 210, which can surround one or more components such as is described hereabove in reference to implantable device 200 and housing 210 of Fig. 1. Lead 265 passes through housing 210 at feedthrough 213. Feedthrough 213 can be constructed and arranged similar to feedthrough 213 described hereabove in reference to Figs, 3 or 4. Lead 265 comprises one or more functional elements 260, such as the four functional elements 260a-d shown. Functional elements 260 can comprise one or more sensors and/or transducers, such as are described hereabove in reference to Fig. 1. In some embodiments, one or more functional elements 260 comprise one or more electrodes, such as electrodes configured to record electrical activity of tissue and/or deliver electrical energy to tissue. In some embodiments, one or more functional elements 260 comprise an agent delivery element, such as a fluid delivery element (e.g. a catheter, a porous membrane, an iontophoretic element or a needle) in fluid communication with a reservoir of the agent positioned within housing 210.

Lead 265 comprises a blind lumen, lumen 266 into which an implantation assisting filament, stylet 267 has been positioned. Stylet 267 is constructed and arranged to provide stiffness to lead 265 during insertion of lead 265 in tissue (e.g. tunneling of lead 265 through tissue), and removed from lumen 266 after lead 265 is properly positioned proximate the desired tissue interface location (e.g. one or more functional elements 260 are positioned proximate the desired tissue interface location).

**Referring now to** **Figs. 12A** **andl2B,** an exploded view and an assembled view of an implantable device comprising an implantable housing surrounding multiple antennas and various electrical components is illustrated, consistent with the present inventive concepts. Implantable device 200 comprises a three piece housing 210 comprising housings 210a, 210b and 210c as shown, which are sealed to each other during assembly of each implantable device 200 (e.g. via adhesive or a bonding method such as solvent bonding, welding and the like). Implantable device 200 further comprises lead 265 which passes through housing 210 at feedthrough 213 (e.g. as described hereabove). Lead 265 comprises one or more functional elements 260, such as one or more electrodes, sensors, transducers or other functional elements as described hereabove in reference to Fig. 1.

In some embodiments, housing 210 comprises a two-piece housing (e.g. constructed of two discrete housing portions), such as when housings 210a and 210b comprise a single housing, or housings 210b and 210c comprise a single housing. Housing 210 can comprise one or more materials, such as glass. In some embodiments, housing 210 comprises a major axis less than or equal to 20mm, such as a major axis less than or equal to 15mm, 12mm or 10mm. In some embodiments, housing 210 comprises a minor axis less than or equal to 8mm, such as a minor axis less than or equal to 6mm. Housings 210 can comprise a wall thickness between 0.2mm and 1.0mm, such as a wall thickness between 0.2mm and 0.5mm, such as a wall thickness of approximately 0.3mm. Implantable device 200 further comprises antennas 240, such as the two antennas 240a and 240b shown positioned on relatively orthogonal planes of a portion of substrate 211 (e.g. a foldable portion of substrate 211). Implantable device 200 further comprises components 216, which have been positioned on substrate 211, such as when substrate 211 comprises a printed circuit board (e.g. a flexible printed circuit board) comprising one or more electrical traces electrically connecting one or more components 216 and/or one or more of antennas 240. Components 216 can be configured to function as one or more assemblies of implantable device 200, such as receiver 230, controller 250, functional element 260 and/or energy storage assembly 270 as described hereabove in reference to Fig. 1. In some embodiments, implantable device 200 comprises a lead comprising one or more functional elements 260 (e.g. one or more electrodes or other sensors and/or transducers as described herein), not shown but such as lead 265 described hereabove in reference to Figs. 1, 2, 3, 4 or 10 which passes through housing 210 and operably connects (e.g. electrically, fluidly, optically and/or mechanically) to one or more of components 216.

**In** **Fig. 12C****,** a perspective view of the implantable device 200 of Figs. 12A and 12B is illustrated, further comprising a lead 265, consistent with the present inventive concepts. Lead 265 comprises one or more functional elements 260, such as functional elements 260a-d shown. Functional elements 260a-d can comprise one or more sensors and/or transducers (e.g. electrodes) such as are described hereabove. Lead 265 passes through housing 210 at feedthrough 213, such as via a sealed passageway as described herein, and operably connects to one or more components within housing 210, also as described herein.

**Referring now to** **Fig. 13****,** a perspective view of an apparatus comprising an implantable device and a limb-attached external device positioned proximate the implantable device; **Fig. 13A** is an end sectional view of the apparatus of Fig. 13, also shown positioned about a limb of the patient; and **Fig. 13B** is a side sectional view of the implantable device of Figs. 13 and 13A; all consistent with the present inventive concepts. Apparatus 10 comprises external device 500 and one or more implantable devices 200'. External device 500 has been positioned proximate the patient P's wrist via patient attachment device 70 as shown. Implantable device 200' is implanted under the patient's skin, such as in subcutaneous tissue of the patient. Implantable device 200' can comprise a reservoir 225, such as a reservoir for maintaining one or more agents. Implantable device 200' can comprise fill port 226 (e.g. a septum or mechanical valve in fluid communication with reservoir 225), which can be constructed and arranged to allow reservoir 225 to be refilled by inserting a needle (e.g. a needle attached to a syringe or other agent-containing chamber) through the patient's skin and into fill port 226.

External device 500' can comprise housing 510, user interface 555 positioned on an outer surface of housing 510, transmitter 530, power supply 570, controller 550, antenna 540 (e.g. one or more external antennas in one or more configurations as described herein), functional element 560 and/or one or more other components, each of which can be of similar construction and arrangement to the similar components of external device 500 described hereabove in reference to Fig. 1. In some embodiments, housing 510 comprises a watch-like construction, such as when user interface 555 is configured to provide time, date and/or one or more watch functions. In some embodiments, external device 500 comprises reservoir 525, which can be fluidly connected to one or more functional elements 560 (e.g. via one or more fluid delivery tubes as shown), such as when a functional element 560 comprises a needle, catheter or other agent delivery element configured to deliver one or more agents beneath the patient's skin (e.g. into a blood vessel or subcutaneous tissue). Functional element 560 can comprise one or more functional elements, such as an agent delivery element, a sensor and/or a transducer.

Implantable device 200' comprises housing 210, receiver 230, antennas 240 (e.g. one or more implantable antennas in one or more configurations as described herein), controller 250, energy storage assembly 270, functional element 260 (e.g. functional element 260a and 260b shown) and/or one or more other components, each of which can be of similar construction and arrangement to the similar components of implantable device 200 described hereabove in reference to Fig. 1. In some embodiments, implantable device 200' comprises reservoir 225, which can be fluidly connected to functional element 260a (e.g. via one or more fluid delivery tubes as shown), such as when functional element 260a comprises an agent delivery element such as a catheter. Functional element 260b can comprise a sensor, such as a sensor configured to provide agent delivery control information to implantable device 200' (e.g. to control agent delivered from reservoir 225 to functional element 260a and into the patient) and/or to provide agent delivery control information to external device 500 (e.g. to control agent delivered from reservoir 525 to functional element 560 and into the patient).

The distance between antenna 540 of external device 500 and antenna 240 of implantable device 200 can be adjusted (e.g. via selection of the implantation site of implantable device 200', selection of the positioning of external device 500 via patient attachment device 70, and/or inclusion of a spacer, such as an adjustable spacer or other spacer similar to spacer 511 described hereabove in reference to Fig. 1), such as to optimize the coupling between antenna 540 and antenna 240 (i.e. to improve transmission of signals between external device 500 and implantable device 200').

Apparatus 10 of Figs. 13A-C can be configured to deliver one or more agents and/or to treat one or more diseases, such as is described hereabove in reference to Fig. 1. Alternatively or additionally, apparatus 10 of Figs. 13A-C can be configured to deliver energy and/or to record patient information, such as patient physiologic or patient environment information, also as described hereabove in reference to Fig. 1.

While the preferred embodiments of the devices have been described in reference to the environment in which they were developed, they are merely illustrative of the principles of the present inventive concepts. Modification or combinations of the above-described assemblies, other embodiments, ans configurations for carrying out the invention, and variations of aspects of the invention that are obvious to those of skill in the art are intended to be within the scope of the claims.

## Claims

1. An implantable device comprising:
at least one implantable antenna (240) configured to receive a transmission signal from an external device;
an implantable receiver (230) configured to receive the transmission signal from the at least one implantable antenna;
at least one implantable functional element (260) configured to interface with the patient;
an implantable controller (250) configured to control the at least one implantable functional element (260);
an implantable energy storage assembly (270) configured to provide power to an element selected from the group consisting of: the at least one implantable functional element; the implantable controller; the implantable receiver; and combinations thereof; and
an implantable housing (210) surrounding at least the implantable controller and the implantable receiver, wherein the implantable device further comprises an implantable lead (265), wherein the implantable lead comprises the at least one implantable functional element (260) and wherein the implantable lead comprises a blind lumen (266) and a stylet (267) insertable into the blind lumen, wherein the blind lumen (266) has a lateral port through which the stylet (267) is removable.

2. An implantable device according to claim 1, wherein the lead (265) passes through housing at a feedthrough (213).

3. An implantable device according to claim 1 or 2, wherein a covering covers all or a portion of housing.

4. An implantable device according to claim 3, wherein the covering is a silicone cover.

5. An implantable device according to claim 1 or 2, wherein the lead (265) passes through the housing at a feedthrough and a covering such as a silicone cover covers all or a portion of the housing and/or feedthrough.

6. An implantable device according to any preceding claim, wherein the implantable controller (250) is configured to produce a stimulation signal for the at least one implantable functional element (260).

7. An implantable device according to claim 6, wherein the stimulation signal comprises at least one of a pseudo random binary sequence non-return-to-zero waveform or a pseudo random binary sequence return-to-zero waveform.

8. An implantable device according to any preceding claim, wherein the at least one implantable functional element (260) comprises at least one electrode.

9. implantable device according to any preceding claim, wherein the at least one implantable functional element (260) comprises an agent delivery element.

10. An implantable device according to any preceding claim, wherein the implantable device is configured to stimulate the spinal cord of the patient.

11. An implantable device according to any preceding claim, wherein the implantable energy storage assembly (270) comprises:
at least one capacitor; and/or
at least one battery, for example a rechargeable battery.

12. An implantable device according to any preceding claim, wherein the implantable energy storage assembly (270) is configured to receive power from the transmission signal.

13. A medical apparatus for a patient, comprising:
an implantable system comprising an implantable device (200) according to any preceding claim; and
an external system (50) configured to transmit one or more transmission signals, each transmission signal comprising at least power or data; and
wherein the external system (50) comprises an external device (500) comprising:
at least one external antenna (540) configured to transmit the transmission signal to the implantable system, the transmission signal comprising at least power or data;
an external transmitter (530) configured to drive the at least one external antenna;
an external power supply (570) configured to provide power to at least the external transmitter; and
an external controller (550) configured to control the external transmitter (530).

## Patentansprüche

1. Eine implantierbare Vorrichtung, die Folgendes beinhaltet:
mindestens eine implantierbare Antenne (240), die dazu ausgelegt ist, ein Sendesignal von einer externen Vorrichtung zu empfangen;
einen implantierbaren Empfänger (230), der dazu ausgelegt ist, das Sendesignal von der mindestens einen implantierbaren Antenne zu empfangen;
mindestens ein implantierbares funktionelles Element (260), das dazu ausgelegt ist, eine Schnittstelle mit dem Patienten zu bilden;
eine implantierbare Steuerung (250), die dazu ausgelegt ist, das mindestens eine implantierbare funktionelle Element (260) zu steuern;
eine implantierbare Energiespeicherbaugruppe (270), die dazu ausgelegt ist, ein Element mit Strom zu versorgen, das aus der Gruppe ausgewählt ist, die aus Folgenden besteht: dem mindestens einen implantierbaren funktionellen Element, der implantierbaren Steuerung, dem implantierbaren Empfänger und Kombinationen davon, und
einem implantierbaren Gehäuse (210), das mindestens die implantierbare Steuerung und den implantierbaren Empfänger umgibt, wobei die implantierbare Vorrichtung ferner ein implantierbares Kabel (265) beinhaltet, wobei das implantierbare Kabel das mindestens eine implantierbare funktionelle Element (260) beinhaltet und wobei das implantierbare Kabel ein Blindlumen (266) und einen Mandrin (267), der in das Blindlumen eingeführt werden kann, beinhaltet, wobei das Blindlumen (266) eine laterale Öffnung aufweist, durch die der Mandrin (267) entfernt werden kann.

2. Implantierbare Vorrichtung nach Anspruch 1, wobei das Kabel (265) an einer Durchführung (213) durch das Gehäuse tritt.

3. Implantierbare Vorrichtung nach Anspruch 1 oder 2, wobei eine Abdeckung das Gehäuse ganz oder teilweise abdeckt.

4. Implantierbare Vorrichtung nach Anspruch 3, wobei die Abdeckung eine Silikonabdeckung ist.

5. Implantierbare Vorrichtung nach Anspruch 1 oder 2, wobei das Kabel (265) an einer Durchführung durch das Gehäuse tritt und eine Abdeckung wie etwa eine Silikonabdeckung das Gehäuse und/oder die Durchführung ganz oder teilweise abdeckt.

6. Implantierbare Vorrichtung nach einem der vorhergehenden Ansprüche, wobei die implantierbare Steuerung (250) dazu ausgelegt ist, ein Stimulationssignal für das mindestens eine implantierbare funktionelle Element (260) zu produzieren.

7. Implantierbare Vorrichtung nach Anspruch 6, wobei das Stimulationssignal mindestens eines von einer Pseudozufallsbinärsequenz-Nicht-Rückkehr-zu-null-Wellenform oder einer Pseudozufallsbinärsequenz-Rückkehrzu-null-Wellenform beinhaltet.

8. Implantierbare Vorrichtung nach einem der vorhergehenden Ansprüche, wobei das mindestens eine implantierbare funktionelle Element (260) mindestens eine Elektrode beinhaltet.

9. Implantierbare Vorrichtung nach einem der vorhergehenden Ansprüche, wobei das mindestens eine implantierbare funktionelle Element (260) ein Wirkstoffzuführungselement beinhaltet.

10. Implantierbare Vorrichtung nach einem der vorhergehenden Ansprüche, wobei die implantierbare Vorrichtung dazu ausgelegt ist, das Rückenmark des Patienten zu stimulieren.

11. Implantierbare Vorrichtung nach einem der vorhergehenden Ansprüche, wobei die implantierbare Energiespeicherbaugruppe (270) Folgendes beinhaltet:
mindestens einen Kondensator; und/oder
mindestens eine Batterie, zum Beispiel eine wiederaufladbare Batterie.

12. Implantierbare Vorrichtung nach einem der vorhergehenden Ansprüche, wobei die implantierbare Energiespeicherbaugruppe (270) dazu ausgelegt ist, Strom von dem Sendesignal zu empfangen.

13. Ein medizinisches Gerät für einen Patienten, das Folgendes beinhaltet:
ein implantierbares System, das eine implantierbare Vorrichtung (200) nach einem der vorhergehenden Ansprüche beinhaltet; und
ein externes System (50), das dazu ausgelegt ist, ein oder mehrere Sendesignale zu senden, wobei jedes Sendesignal mindestens Strom oder Daten beinhaltet; und
wobei das externe System (50) eine externe Vorrichtung (500) beinhaltet, die Folgendes beinhaltet:
mindestens eine externe Antenne (540), die dazu ausgelegt ist, das Sendesignal zu dem implantierbaren System zu senden, wobei das Sendesignal mindestens Strom oder Daten beinhaltet;
einen externen Sender (530), der dazu ausgelegt ist, die mindestens eine externe Antenne anzutreiben;
eine externe Stromversorgung (570), die dazu ausgelegt ist, mindestens dem externen Sender Strom bereitzustellen;
und
eine externe Steuerung (550), die dazu ausgelegt ist, den externen Sender (530) zu steuern.

## Revendications

1. Dispositif implantable comprenant :
au moins une antenne implantable (240) configurée pour recevoir un signal d'émission d'un dispositif externe ;
un récepteur implantable (230) configuré pour recevoir le signal d'émission de l'au moins une antenne implantable ;
au moins un élément fonctionnel implantable (260) configuré pour assurer l'interface avec le patient ;
un dispositif de commande implantable (250) configuré pour commander l'au moins un élément fonctionnel implantable (260) ;
un ensemble de stockage d'énergie implantable (270) configuré pour fournir du courant à un élément sélectionné parmi le groupe constitué par : l'au moins un élément fonctionnel implantable ; le dispositif de commande implantable ; le récepteur implantable ; et des combinaisons de ceux-ci ; et
un boîtier implantable (210) entourant au moins le dispositif de commande implantable et le récepteur implantable, dans lequel le dispositif implantable comprend en outre un fil implantable (265), dans lequel le fil implantable comprend l'au moins un élément fonctionnel implantable (260) et dans lequel le fil implantable comprend une lumière borgne (266) et un stylet (267) insérable jusque dans la lumière borgne, dans lequel la lumière borgne (266) a un orifice latéral à travers lequel le stylet (267) peut être retiré.

2. Dispositif implantable selon la revendication 1, dans lequel le fil (265) passe à travers le boîtier au niveau d'une traversée (213).

3. Dispositif implantable selon la revendication 1 ou 2, dans lequel une couverture couvre la totalité ou une partie du boîtier.

4. Dispositif implantable selon la revendication 3, dans lequel la couverture est une couverture de silicone.

5. Dispositif implantable selon la revendication 1 ou 2, dans lequel le fil (265) passe à travers le boîtier au niveau d'une traversée et une couverture telle qu'une couverture de silicone couvre la totalité ou une partie du boîtier et/ou de la traversée.

6. Dispositif implantable selon l'une quelconque des revendications précédentes, dans lequel le dispositif de commande implantable (250) est configuré pour produire un signal de stimulation pour l'au moins un élément fonctionnel implantable (260).

7. Dispositif implantable selon la revendication 6, dans lequel le signal de stimulation comprend au moins l'une d'une forme d'onde de non retour à zéro à séquence binaire pseudo-aléatoire ou d'une forme d'onde de retour à zéro à séquence binaire pseudo-aléatoire.

8. Dispositif implantable selon l'une quelconque des revendications précédentes, dans lequel l'au moins un élément fonctionnel implantable (260) comprend au moins une électrode.

9. Dispositif implantable selon l'une quelconque des revendications précédentes, dans lequel l'au moins un élément fonctionnel implantable (260) comprend un élément de délivrance d'agent.

10. Dispositif implantable selon l'une quelconque des revendications précédentes, dans lequel le dispositif implantable est configuré pour stimuler la moelle épinière du patient.

11. Dispositif implantable selon l'une quelconque des revendications précédentes, dans lequel l'ensemble de stockage d'énergie implantable (270) comprend :
au moins un condensateur ; et/ou
au moins une batterie, par exemple une batterie rechargeable.

12. Dispositif implantable selon l'une quelconque des revendications précédentes, dans lequel l'ensemble de stockage d'énergie implantable (270) est configuré pour recevoir du courant du signal d'émission.

13. Appareil médical pour un patient, comprenant :
un système implantable comprenant un dispositif implantable (200) selon l'une quelconque des revendications précédentes ; et
un système externe (50) configuré pour émettre un ou plusieurs signaux d'émission, chaque signal d'émission comprenant au moins du courant ou des données ; et
dans lequel le système externe (50) comprend un dispositif externe (500) comprenant :
au moins une antenne externe (540) configurée pour émettre le signal d'émission au système implantable, le signal d'émission comprenant au moins du courant ou des données ;
un émetteur externe (530) configuré pour piloter au moins une antenne externe ;
une source de courant externe (570) configurée pour fournir du courant à l'au moins un émetteur externe ; et
un dispositif de commande externe (550) configuré pour commander l'émetteur externe (530).
